(19) **Europäisches Patentamt**
**European Patent Office**
**Office européen des brevets**

(11) **EP 4 620 978 A1**

(12) **EUROPEAN PATENT APPLICATION**
published in accordance with Art. 153(4) EPC

(43) Date of publication:
**24.09.2025 Bulletin 2025/39**

(21) Application number: **23885090.3**

(22) Date of filing: **03.11.2023**

(51) International Patent Classification (IPC):
***C07K 16/30*** (2006.01)     ***C12N 5/10*** (2006.01)

(52) Cooperative Patent Classification (CPC):
**C07K 16/28; C07K 16/30; C12N 5/06; C12N 5/10;**
**C12N 15/62**

(86) International application number:
**PCT/CN2023/129566**

(87) International publication number:
**WO 2024/094165 (10.05.2024 Gazette 2024/19)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **04.11.2022  CN 202211377234**

(71) Applicant: **T-Maximum Pharmaceutical (Suzhou)**
**Co., Ltd.**
**Suzhou, Jiangsu 215000 (CN)**

(72) Inventors:
• **SHANG, Xiaoyun**
**Suzhou, Jiangsu 215000 (CN)**

• **JIANG, Haijuan**
**Suzhou, Jiangsu 215000 (CN)**
• **MA, Shaowen**
**Suzhou, Jiangsu 215000 (CN)**

(74) Representative: **Weickmann & Weickmann**
**PartmbB**
**Postfach 860 820**
**81635 München (DE)**

Remarks:
The complete document including Reference
Table(s) and the Sequence Listing(s) can be
downloaded from the EPO website

(54) **UNIVERSAL CAR-T CELL TARGETING B7H3, AND PREPARATION METHOD THEREFOR AND USE THEREOF**

(57)    A B7H3 binding polypeptide, comprising two different anti-B7H3 single domain antibodies. The present invention further relates to a chimeric antigen receptor comprising B7H3 binding polypeptide, and a universal CAR-T cell comprising the chimeric antigen receptor. An antigen on the surface of a tumor cell is recognized while the TCR and HLA-A genes expressed by the cells are knocked out, so that an immune rejection reaction caused by allogeneic CAR-T treatment is reduced, the cell survival time is prolonged, and the anti-tumor effect is improved.

EP 4 620 978 A1

**Description**

## TECHNICAL FIELD

The present disclosure relates to the fields of gene knockout strategies, molecular biology, and immunotherapy, and particularly to a universal B7H3 UCAR-T cell based on base editing, a preparation method therefor, and use thereof.

## BACKGROUND

Glioblastoma accounts for 15% of all brain tumors, arising either *de novo* from normal brain cells or progressing from low-grade astrocytomas. Generally, the survival post-diagnosis ranges from 12 to 15 months, with a 5-year survival rate of only 3% to 7%. Without treatment, the survival time is generally 3 months. About 3 out of every 100 thousand people are newly diagnosed with glioblastoma every year, which is the most common cancer of brain origin and the second most common brain tumor after meningioma.

With the development of tumor immunity theory and the progress of technology, cellular immunotherapy for tumors attracts more and more attention. CAR-T cell technology is a cell-based therapeutic approach that has produced excellent results in tumor immunotherapy, especially in the treatment of hematologic tumors. CAR-T immunotherapy utilizes genetically engineered T cells capable of antigen-specific recognition and elimination of tumor cells expressing target antigens, independent of MHC restriction. The CAR-T immunotherapy has achieved good effects in the treatment of various B-cell malignant tumors, for example, CD19-targeted CAR-T cells for the treatment of acute lymphocytic leukemia (ALL), chronic lymphocytic leukemia (CLL), and non-Hodgkin's lymphoma (NHL). At the same time, clinical applications of CAR-T cells for multiple myeloma and relapsed/refractory multiple myeloma are underway, demonstrating encouraging outcomes.

B7-H3, also referred to as CD276, belongs to the immunomodulatory protein B7 family and is a type I membrane protein with an extracellular domain sequence similar to those of other B7 family members. The B7-H3 gene is located on human chromosome 15 and consists of ten exons, of which exons 4 to 7 encode extracellular IgV-IgC domains. mRNA of B7-H3 is expressed in various normal tissues and some tumor cell lines, and is not detectable in peripheral blood mononuclear cells (PBMCs). However, the expression of B7-H3 can be induced on dendritic cells and monocytes by inflammatory cytokines (IFNy) and compositions of PMA and ionomycin. Although B7-H3 mRNA is widely expressed in normal tissues, the expression levels of B7-H3 protein are extremely low or absent in normal tissues, indicating that the expression of B7-H3 protein is subjected to strict post-transcriptional regulation. In contrast, the B7-H3 protein is overexpressed in various malignant tumors and is associated with poor prognosis, relatively high tumor grade and tumor metastasis, drug resistance, and low overall survival.

The differential expression of B7-H3 between tumors and healthy tissues makes it very suitable as a therapeutic target, since targeting this antigen results in very limited side effects. The results of preclinical studies have shown that the inhibition or reduction of B7-H3 protein expression in tumor cells can reduce cell proliferation and glycolysis, and increase drug sensitivity of tumor cells.

The CAR-T cell therapy targeting B7-H3 has been studied. A preclinical study demonstrated that B7-H3 CAR-T cells exhibit significant anti-tumor activity *in vivo,* inducing regression of established solid sarcomas in multiple allograft models (including osteosarcoma, medulloblastoma, and Ewing's sarcoma).

However, autologous T cells from patients have limited expansion capacity or reduced functions *in vitro,* thereby leading to an insufficient quantity or poor quality of the manufactured CAR-T cell products. Universal CAR-T cells are T cells isolated from healthy donors, and the prepared CAR-T cells not only have high expansion efficiency and strong activity, but also have an improved infection positive rate. However, the universal CAR-T still faces the problems of graft-versus-host disease (GVHD) and immune rejection. The CRISPR/Cas9 system is the most commonly used gene editing method, and can be used for producing T cells with TCR deficiency and HLA class I molecule deficiency, and for mitigating immune rejection caused by allogeneic cell therapy.

The present disclosure aims to prepare a universal CAR-T cell targeting the dual epitopes of B7H3 protein, which recognizes tumor surface antigens while simultaneously having its TCR and HLA-A genes knocked out, thereby mitigating immune rejection caused by allogeneic CAR-T therapy, extending cell persistence, and improving anti-tumor efficacy.

## SUMMARY

In one aspect, the present application provides an isolated B7H3-binding polypeptide comprising a first heavy chain variable region of a heavy-chain antibody (VHH-1) and a second heavy chain variable region of a heavy-chain antibody (VHH-2), both targeting B7H3, wherein the VHH-1 comprises an HCDR1 set forth in SEQ ID NO: 1, an HCDR2 set forth in SEQ ID NO: 3, and an HCDR3 set forth in SEQ ID NO: 5; and/or the VHH-2 comprises an HCDR1 set forth in SEQ ID NO: 2, an HCDR2 set forth in SEQ ID NO: 4, and an HCDR3 set forth in SEQ ID NO: 6.

In some embodiments, the VHH-1 and/or VHH-2 comprises a human antibody, a humanized antibody, or a chimeric antibody.

In some embodiments, the VHH-1 and VHH-2 are linked directly or via a linker.

In some embodiments, the B7H3-binding polypeptide sequentially comprises, from the N-terminus to the C-terminus, i) VHH-1-linker-VHH-2; or ii) VHH-2-linker-VHH-1.

In some embodiments, the linker comprises the amino acid sequence set forth in SEQ ID NO: 22.

In some embodiments, the VHH-1 comprises:

i) an HFR1 comprising the amino acid sequence set forth in SEQ ID NO: 7, an HFR2 comprising the amino acid sequence set forth in SEQ ID NO: 11, an HFR3 comprising the amino acid sequence set forth in SEQ ID NO: 13, and an HFR4 comprising the amino acid sequence set forth in SEQ ID NO: 16; or

ii) an HFR1 comprising the amino acid sequence set forth in SEQ ID NO: 8, an HFR2 comprising the amino acid sequence set forth in SEQ ID NO: 11, an HFR3 comprising the amino acid sequence set forth in SEQ ID NO: 14, and an HFR4 comprising the amino acid sequence set forth in SEQ ID NO: 17.

In some embodiments, the VHH-1 comprises the amino acid sequence set forth in SEQ ID NO: 18 or SEQ ID NO: 19.

In some embodiments, the VHH-2 comprises:

i) an HFR1 comprising the amino acid sequence set forth in SEQ ID NO: 9, an HFR2 comprising the amino acid sequence set forth in SEQ ID NO: 12, an HFR3 comprising the amino acid sequence set forth in SEQ ID NO: 15, and an HFR4 comprising the amino acid sequence set forth in SEQ ID NO: 16; or

ii) an HFR1 comprising the amino acid sequence set forth in SEQ ID NO: 10, an HFR2 comprising the amino acid sequence set forth in SEQ ID NO: 12, an HFR3 comprising the amino acid sequence set forth in SEQ ID NO: 14, and an HFR4 comprising the amino acid sequence set forth in SEQ ID NO: 17.

In some embodiments, the VHH-2 comprises the amino acid sequence set forth in SEQ ID NO: 20 or SEQ ID NO: 21.

In some embodiments, the B7H3-binding polypeptide comprises the amino acid sequence set forth in any one of SEQ ID NO: 23, SEQ ID NO: 24, SEQ ID NO: 26, and SEQ ID NO: 25.

In another aspect, the present application provides an immunoconjugate comprising the B7H3-binding polypeptide described herein.

In another aspect, the present application provides a chimeric antigen receptor (CAR) comprising an extracellular antigen-binding domain targeting B7H3, a transmembrane domain, an intracellular co-stimulatory signaling domain, and an intracellular signaling domain, wherein the extracellular antigen-binding domain comprises at least two VHHs that bind to distinct epitopes of B7H3.

In some embodiments, the extracellular antigen-binding domain comprises two VHHs that bind to distinct epitopes of B7H3.

In another aspect, the present application provides a chimeric antigen receptor comprising an extracellular antigen-binding domain targeting B7H3, a transmembrane domain, an intracellular co-stimulatory signaling domain, and an intracellular signaling domain, wherein the extracellular antigen-binding domain comprises the B7H3-binding polypeptide described herein.

In some embodiments, the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4 (CD244), FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

In some embodiments, the transmembrane domain comprises a transmembrane domain derived from CD8A.

In some embodiments, the transmembrane domain comprises the amino acid sequence set forth in any one of SEQ ID NO: 62 to SEQ ID NO: 110.

In some embodiments, the transmembrane domain comprises the amino acid sequence set forth in SEQ ID NO: 62.

In some embodiments, the intracellular co-stimulatory signaling domain comprises an intracellular co-stimulatory signaling domain derived from one or more proteins selected from the group consisting of: CD28, CD137, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

In some embodiments, the intracellular co-stimulatory signaling domain is derived from a 4-1BB co-stimulatory signaling domain.

In some embodiments, the intracellular co-stimulatory signaling domain comprises the amino acid sequence set forth in any one of SEQ ID NO: 111 to SEQ ID NO: 143.

In some embodiments, the intracellular co-stimulatory signaling domain comprises the amino acid sequence set forth in SEQ ID NO: 112.

In some embodiments, the intracellular signaling domain comprises an intracellular signaling domain derived from one or more proteins selected from the group consisting of: CD3ζ, CD3δ, CD3y, CD3ε, CD79a, CD79b, FceRIγ, FceRIβ, FcyRIIa, bovine leukemia virus gp30, Epstein-Barr virus (EBV) LMP2A, simian immunodeficiency virus PBj14 Nef, DAP10, DAP-12, and a domain comprising at least one ITAM.

In some embodiments, the intracellular signaling domain comprises a signaling domain derived from CD3ζ.

In some embodiments, the intracellular signaling domain comprises the amino acid sequence set forth in any one of SEQ ID NO: 127, SEQ ID NO: 131, SEQ ID NO: 132, and SEQ ID NO: 144 to SEQ ID NO: 154.

In some embodiments, the intracellular signaling domain comprises the amino acid sequence set forth in SEQ ID NO: 144.

In some embodiments, the chimeric antigen receptor comprises a hinge region between the extracellular antigen-binding domain and the transmembrane domain, wherein the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

In some embodiments, the hinge region comprises a hinge region derived from CD8A.

In some embodiments, the hinge region comprises the amino acid sequence set forth in any one of SEQ ID NO: 155 to SEQ ID NO: 176.

In some embodiments, the hinge region comprises the amino acid sequence set forth in SEQ ID NO: 163.

In some embodiments, a non-targeting portion of the chimeric antigen receptor comprises a CD8A hinge region, a CD8A transmembrane domain, a 4-1BB intracellular co-stimulatory signaling domain, and a CD3ζ intracellular signaling domain.

In some embodiments, the non-targeting portion of the chimeric antigen receptor comprises the amino acid sequence set forth in SEQ ID NO: 27.

In some embodiments, the chimeric antigen receptor further comprises a signal peptide fragment, wherein the C-terminus of the signal peptide fragment is linked to the N-terminus of the extracellular antigen-binding domain.

In some embodiments, the chimeric antigen receptor sequentially comprises, from the N-terminus to the C-terminus, the signal peptide fragment, the extracellular antigen-binding domain, the transmembrane domain, the intracellular co-stimulatory signaling domain, and the intracellular signaling domain.

In some embodiments, the signal peptide fragment comprises a CD8A signal peptide fragment.

In some embodiments, the signal peptide fragment comprises the amino acid sequence set forth in SEQ ID NO: 28.

In some embodiments, the chimeric antigen receptor comprises the amino acid sequence set forth in any one of SEQ ID NO: 33 to SEQ ID NO: 36.

In another aspect, the present application provides one or more isolated polypeptides comprising the chimeric antigen receptor described herein and an enhancer capable of enhancing one or more activities of an immune effector cell.

In some embodiments, the enhancer comprises a cytokine and/or a cytokine receptor.

In some embodiments, the enhancer is a membrane-bound cytokine and/or cytokine receptor.

In some embodiments, the enhancer is selected from the group consisting of: IL-2, IL-3, IL-4, IL-6, IL-7, IL-8, IL-10, IL-11, IL-12, IL-15, IL-17, IL-18, IL-21, IL-23, receptors thereof, functional variants thereof, and combinations thereof.

In some embodiments, the enhancer comprises IL-15 or a functional variant thereof, or a chimeric cytokine receptor comprising an IL-15 receptor (IL-15R).

In some embodiments, the enhancer comprises membrane-bound IL-15 (mIL-15).

In some embodiments, the enhancer comprises the amino acid sequence set forth in SEQ ID NO: 46 or SEQ ID NO: 54.

In some embodiments, the polypeptide comprises i) the amino acid sequence set forth in any one of SEQ ID NO: 33 to SEQ ID NO: 36; and ii) the amino acid sequence set forth in SEQ ID NO: 46 or SEQ ID NO: 54.

In some embodiments, the chimeric antigen receptor and the enhancer are located in separate polypeptides.

In some embodiments, the chimeric antigen receptor and the enhancer are located in the same polypeptide. For example, the chimeric antigen receptor and the enhancer may be linked directly or indirectly.

In some embodiments, the chimeric antigen receptor and the enhancer are linked via a linker. For example, the polypeptide may comprise the following structures, from the N-terminus to the C-terminus, i) the chimeric antigen receptor, the linker, and the enhancer; or ii) the enhancer, the linker, and the chimeric antigen receptor.

In some embodiments, the linker comprises a self-cleaving peptide.

In some embodiments, the self-cleaving peptide comprises P2A, F2A, E2A, or T2A.

In some embodiments, the T2A comprises the amino acid sequence set forth in SEQ ID NO: 44.

In some embodiments, the polypeptide comprises the amino acid sequence set forth in SEQ ID NO: 241 or SEQ ID NO: 243.

In another aspect, the present application provides one or more isolated nucleic acid molecules encoding the B7H3-binding polypeptide described herein, the chimeric antigen receptor described herein, or the polypeptide described herein.

In another aspect, the present application provides one or more isolated nucleic acid molecules comprising a first nucleotide sequence encoding the chimeric antigen receptor described herein, and a second nucleotide sequence encoding an enhancer capable of enhancing one or more activities of an immune effector cell.

In some embodiments, the enhancer comprises a cytokine and/or a cytokine receptor.

In some embodiments, the enhancer is a membrane-bound cytokine and/or cytokine receptor.

In some embodiments, the enhancer is selected from the group consisting of: IL-2, IL-3, IL-4, IL-6, IL-7, IL-8, IL-10, IL-11, IL-12, IL-15, IL-17, IL-18, IL-21, IL-23, receptors thereof, functional variants thereof, and combinations thereof.

In some embodiments, the enhancer comprises IL-15 or a functional variant thereof, or a chimeric cytokine receptor comprising an IL-15 receptor (IL-15R).

In some embodiments, the enhancer comprises membrane-bound IL-15 (mIL-15).

In some embodiments, the enhancer comprises the amino acid sequence set forth in SEQ ID NO: 46 or SEQ ID NO: 54.

In some embodiments, the nucleic acid molecule comprises i) a nucleotide sequence encoding the amino acid sequence set forth in any one of SEQ ID NO: 33 to SEQ ID NO: 36; and ii) the amino acid sequence set forth in SEQ ID NO: 47 or SEQ ID NO: 55.

In some embodiments, the first nucleotide sequence and the second nucleotide sequence are located in separate nucleic acid molecules.

In some embodiments, the first nucleotide sequence and the second nucleotide sequence are located in the same nucleic acid molecule.

In some embodiments, the first nucleotide sequence and the second nucleotide sequence are linked directly.

In some embodiments, the first nucleotide sequence and the second nucleotide sequence are linked indirectly.

In some embodiments, the first nucleotide sequence and the second nucleotide sequence are linked via a spacer sequence.

In some embodiments, the spacer sequence comprises an internal ribosome entry site (IRES) or encodes a self-cleaving polypeptide.

In some embodiments, the spacer sequence encodes a viral self-cleaving polypeptide.

In some embodiments, the spacer sequence encodes a viral self-cleaving 2A polypeptide (2A).

In some embodiments, the viral self-cleaving 2A polypeptide comprises a foot-and-mouth disease virus (FMDV) (F2A) peptide, an equine rhinitis A virus (ERAV) (E2A) peptide, a Thoseaasigna virus (TaV) (T2A) peptide, a porcine teschovirus-1 (PTV-1) (P2A) peptide, a Theilovirus 2A peptide, or an encephalomyocarditis virus 2A peptide.

In some embodiments, the spacer sequence comprises a nucleotide sequence encoding T2A.

In some embodiments, the spacer sequence comprises the nucleotide sequence set forth in SEQ ID NO: 43.

In some embodiments, the IRES is selected from at least one of the group consisting of: poliomyelitis IRES, rhinovirus IRES, encephalomyocarditis virus IRES (EMCV-IRES), picornavirus IRES, foot-and-mouth disease virus IRES (FMDV-IRES), oropharyngeal virus IRES, Kaposi's sarcoma-associated herpesvirus IRES (KSHV-IRES), hepatitis A virus IRES, hepatitis C virus IRES, classical swine fever virus IRES, pestivirus IRES, bovine viral diarrhea virus IRES, Friend murine leukemia virus IRES, Moloney murine leukemia virus IRES (MMLV-IRES), Rous sarcoma virus IRES, human immunodeficiency virus IRES (HIV-IRES), Plautia stali intestine virus IRES, dicistrovirus IRES, cricket paralysis virus IRES, Triatoma virus IRES, Rhopalosiphum padi virus IRES, Marek's disease virus IRES, fibroblast growth factors (FGF-1 IRES and FGF-2 IRES), platelet-derived growth factor B (PDGF/c-sis IRES), vascular endothelial growth factor (VEGF IRES), and insulin-like growth factor 2 (IGF-II IRES).

In some embodiments, the nucleic acid molecule sequentially comprises, from the 5' end to the 3' end, i) the first nucleotide sequence, the spacer sequence, and the second nucleotide sequence; or ii) the second nucleotide sequence, the spacer sequence, and the first nucleotide sequence.

In some embodiments, the nucleic acid molecule sequentially comprises, from the 5' end to the 3' end, a nucleotide sequence encoding the CAR described herein, the spacer sequence, and a nucleotide sequence encoding the enhancer.

In some embodiments, the nucleic acid molecule comprises the nucleotide sequence set forth in SEQ ID NO: 240 or SEQ ID NO: 242.

In another aspect, the present application provides a vector comprising the isolated nucleic acid molecule described herein.

In some embodiments, the vector is an expression vector.

In some embodiments, the vector is selected from the group consisting of: a DNA vector, an RNA vector, a plasmid, a lentiviral vector, an adenoviral vector, an adeno-associated viral vector, and a retroviral vector.

In another aspect, the present application provides a cell comprising the isolated nucleic acid molecule described herein or the vector described herein; and/or expressing the B7H3-binding polypeptide or chimeric antigen receptor described herein.

In another aspect, the present application provides an immune effector cell comprising the isolated nucleic acid molecule described herein or the vector described herein; and/or expressing the chimeric antigen receptor or polypeptide described herein.

In another aspect, the present application provides an immune effector cell comprising a chimeric antigen receptor targeting B7H3 and a membrane-bound IL15 molecule (mIL15).

In some embodiments, an extracellular antigen-binding domain of the chimeric antigen receptor comprises at least two VHHs that bind to distinct epitopes of B7H3.

In some embodiments, the extracellular antigen-binding domain comprises two VHHs that bind to distinct epitopes of B7H3.

In some embodiments, the mIL15 comprises the amino acid sequence set forth in SEQ ID NO: 46 or SEQ ID NO: 54.

In some embodiments, the immune effector cell comprises the chimeric antigen receptor described herein.

In some embodiments, the chimeric antigen receptor comprises the amino acid sequence set forth in any one of SEQ ID NO: 29 to SEQ ID NO: 36.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In some embodiments, the immune effector cell comprises an autologous or non-autologous immune effector cell.

In some embodiments, the immune effector cell comprises a modified immune effector cell.

In some embodiments, the modified immune effector cell comprises a cell that reduces immune rejection caused by allogeneic cell therapy.

In some embodiments, the functions of T cell antigen receptor (TCR) and major histocompatibility complex (MHCI, MHCII) in the modified immune effector cell are suppressed in a T cell.

In some embodiments, the modification comprises down-regulating the expression and/or activity of one or more of immune rejection-associated genes.

In some embodiments, the immune rejection-associated gene is selected from one or more of the following genes: TRAC, TRBC, HLA-A, HLA-B, B2M, and CIITA.

In some embodiments, the expression and/or activity of the TRAC gene and the HLA-A gene in the modified immune effector cell is down-regulated as compared to a corresponding unmodified cell.

In some embodiments, the expression and/or activity of the CIITA gene in the modified immune effector cell is not down-regulated as compared to the corresponding unmodified cell.

In some embodiments, the expression and/or activity of the B2M gene in the modified immune effector cell is not down-regulated as compared to the corresponding unmodified cell.

In some embodiments, the expression and/or activity of the TRAC gene and the HLA-A gene in the modified immune effector cell is down-regulated as compared to a corresponding wild-type cell.

In some embodiments, the expression and/or activity of the TRAC gene, the HLA-A gene, and the HLA-B gene in the modified immune effector cell is down-regulated as compared to the corresponding unmodified cell.

In some embodiments, the expression and/or activity of the B2M gene in the modified immune effector cell is not down-regulated as compared to the corresponding wild-type cell.

In some embodiments, the expression and/or activity of the CIITA gene in the modified immune effector cell is not down-regulated as compared to the corresponding wild-type cell.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene knockout, gene mutation, and/or gene silencing.

In some embodiments, the modification comprises knocking out either of two TRAC alleles and knocking out either of two HLA-A alleles in the immune effector cell.

In some embodiments, the modification comprises knocking out both TRAC alleles and knocking out either of both HLA-A alleles in the immune cell.

In some embodiments, the modification comprises knocking out an exon of the TRAC gene and knocking out an exon of the HLA-A gene in the immune cell.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification further comprises administering to the immune effector cell a single guide RNA (sgRNA) targeting an exonic region of the TRAC gene.

In some embodiments, the sgRNA targeting the exonic region of the TRAC gene comprises the nucleotide sequence set forth in any one of SEQ ID NO: 177 to SEQ ID NO: 191.

In some embodiments, the modification comprises administering to the immune effector cell sgRNA targeting an exonic region of the HLA-A gene.

In some embodiments, the sgRNA targeting the exonic region of the HLA-A gene comprises the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises the nucleotide sequence set forth in any one of SEQ ID NO: 237 to SEQ ID NO: 239.

In some embodiments, the modification further comprises administering to the cell a Cas enzyme.

In some embodiments, the Cas enzyme comprises a Cas9 protein.

In some embodiments, the antisense RNA comprises the nucleotide sequence set forth in any one of SEQ ID NO: 233 to SEQ ID NO: 236.

In some embodiments, the immune effector cell is an HLA-B homozygous cell.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the immune effector cell is an HLA-A homozygous or heterozygous cell.

In some embodiments, the HLA-A homozygote or heterozygote comprises HLA-A*02 homozygote, HLA-A* 11 homozygote, HLA-A*02/A*11 heterozygote, or HLA-A*24 homozygote.

In another aspect, the present application provides a method for preparing an immune effector cell, which comprises introducing the nucleic acid molecule described herein or the vector described herein into an immune effector cell.

In some embodiments, the method further comprises: modifying an immune effector cell before/after introducing the nucleic acid molecule described herein or the vector described herein into the immune effector cell, wherein the modification comprises down-regulating the expression and/or activity of one or more of immune rejection-associated genes.

In some embodiments, the immune rejection-associated gene is selected from one or more of the following genes: TRAC, TRBC, HLA-A, HLA-B, B2M, and CIITA.

In some embodiments, the expression and/or activity of the TRAC gene and the HLA-A gene in the immune effector cell is down-regulated as compared to the expression and/or activity of the corresponding gene in a corresponding unmodified cell.

In some embodiments, the expression and/or activity of the CIITA gene is not down-regulated as compared to the expression and/or activity of the corresponding gene in the corresponding unmodified cell.

In some embodiments, the expression and/or activity of the B2M gene is not down-regulated as compared to the expression and/or activity of the corresponding gene in the corresponding unmodified cell.

In some embodiments, the expression and/or activity of the TRAC gene and the HLA-A gene in the immune effector cell is down-regulated as compared to a corresponding wild-type cell.

In some embodiments, the expression and/or activity of the TRAC gene, the HLA-A gene, and the HLA-B gene in the immune effector cell is down-regulated as compared to the corresponding wild-type cell.

In some embodiments, the expression and/or activity of the CIITA gene is not down-regulated as compared to the corresponding wild-type cell.

In some embodiments, the expression and/or activity of the B2M gene is not down-regulated as compared to the corresponding wild-type cell.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene knockout, gene mutation, and/or gene silencing.

In some embodiments, the modification comprises knocking out either of two TRAC alleles and knocking out either of two HLA-A alleles in the immune effector cell.

In some embodiments, the modification comprises knocking out both TRAC alleles and knocking out either of both HLA-A alleles in the immune cell.

In some embodiments, the modification comprises knocking out an exon of the TRAC gene and knocking out an exon of the HLA-A gene in the immune cell.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification comprises administering to the immune effector cell sgRNA targeting an exonic region of the TRAC gene.

In some embodiments, the sgRNA targeting the exonic region of the TRAC gene comprises the nucleotide sequence set forth in any one of SEQ ID NO: 177 to SEQ ID NO: 191.

In some embodiments, the modification comprises administering to the immune effector cell sgRNA targeting an exonic region of the HLA-A gene.

In some embodiments, the sgRNA targeting the exonic region of the HLA-A gene comprises the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises the nucleotide sequence set forth in any one of SEQ ID NO: 237 to SEQ ID NO: 239.

In some embodiments, the modification further comprises administering to the cell a Cas enzyme.

In some embodiments, the Cas enzyme comprises a Cas9 protein.

In some embodiments, the antisense RNA comprises the nucleotide sequence set forth in any one of SEQ ID NO: 233 to SEQ ID NO: 236.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In some embodiments, the immune effector cell comprises an autologous or non-autologous immune effector cell.

In some embodiments, the cell is an HLA-B homozygous or heterozygous cell.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the cell is an HLA-A homozygous or heterozygous cell.

In some embodiments, the HLA-A homozygote or heterozygote comprises HLA-A*02 homozygote, HLA-A* 11 homozygote, HLA-A*02/A*11 heterozygote, or HLA-A*24 homozygote.

In another aspect, the present application provides use of the isolated nucleic acid molecule described herein, the vector described herein, the cell described herein, or the immune effector cell described herein in the preparation of a CAR-T cell.

In another aspect, the present application provides a pharmaceutical composition comprising the B7H3-binding polypeptide described herein, the isolated nucleic acid molecule described herein, the vector described herein, the cell described herein, and/or the immune effector cell described herein, and optionally a pharmaceutically acceptable carrier.

In another aspect, the present application provides use of the B7H3-binding polypeptide described herein, the isolated nucleic acid molecule described herein, the vector described herein, the cell described herein, the immune effector cell described herein, and/or the pharmaceutical composition described herein in the treatment of a disease or disorder associated with the expression of B7H3.

In some embodiments, the disease or disorder associated with the expression of B7H3 comprises a disease or disorder associated with up-regulation of the expression of B7H3.

In some embodiments, the disease or disorder associated with the expression of B7H3 comprises cancer.

In some embodiments, the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

In another aspect, the present application provides a method for preventing or treating a disease or disorder associated with the expression of B7H3, which comprises administering to a subject in need thereof an effective amount of the B7H3-binding polypeptide described herein, the isolated nucleic acid molecule described herein, the vector described herein, the cell described herein, the immune effector cell described herein, and/or the pharmaceutical composition described herein.

In some embodiments, the disease or disorder associated with the expression of B7H3 comprises a disease or disorder associated with up-regulation of the expression of B7H3.

In some embodiments, the disease or disorder associated with the expression of B7H3 comprises cancer.

In some embodiments, the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

Other aspects and advantages of the present application will be readily apparent to those skilled in the art from the following detailed description. Only exemplary embodiments of the present application are shown and described in the following detailed description. As will be recognized by those skilled in the art, the content of the present application enables those skilled in the art to make changes to the specific embodiments disclosed without departing from the spirit and scope of the invention to which the present application pertains. Accordingly, descriptions in the drawings and specification of the present application are only illustrative rather than restrictive.

## BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. A brief description of the drawings is as below.

FIG. 1 shows a lentiviral expression vector encoding the bi-epitopic B7H3 chimeric antigen receptor (CAR) gene of the present application.

FIG. 2 shows the cytotoxic efficacy of the B7H3 universal CAR-T (UCAR-T) cells described herein against target cells.

FIG. 3 shows cytokine secretion assay of the B7H3 UCAR-T cells described herein co-cultured with the target cells.

FIG. 4 shows the *in vivo* anti-tumor efficacy of the B7H3-targeted CAR-T cells described herein.

FIG. 5 shows the *in vitro* safety of the B7H3-targeted UCAR-T cells described herein.

FIG. 6 shows *in vivo* half-life assay results of the B7H3-targeted UCAR cells described herein.

FIGs. 7 to 12 show the results of graft-versus-host disease (GVHD) and *in vivo* rejection response of T cells with double knockout of TRAC and HLA-A.

FIG. 13 shows off-target analysis of the T cells with double knockout of TRAC and HLA-A described herein.

FIG. 14 shows chromosome translocation analysis of the T cells with double knockout of TRAC and HLA-A described herein.

FIG. 15 shows karyotype analysis of the T cells with double knockout of TRAC and HLA-A described herein.

FIG. 16 shows residual Cas9 analysis of the T cells with double knockout of TRAC and HLA-A described herein.

## DETAILED DESCRIPTION

The embodiments of the present application are described below with reference to specific examples, and other advantages and effects of the present application will be readily apparent to those skilled in the art from the disclosure of the present specification.

**Definitions of Terms**

In the present application, the term "chimeric antigen receptor" or "CAR" generally refers to a group of polypeptides, generally two types in the simplest embodiment, which, when in an immune effector cell, provide the cell with specificity for a target cell (generally a cancer cell) and produce an intracellular signal. In some embodiments, the CAR comprises at least one extracellular antigen-binding domain (such as a VHH, scFv, or a portion thereof), a transmembrane domain, and a cytoplasmic signaling domain (also referred to herein as an "intracellular signaling domain") that comprises a functional signaling domain derived from a stimulatory molecule and/or a co-stimulatory molecule as defined below. In some embodiments, the group of polypeptides is in the same polypeptide chain (e.g., comprise a chimeric fusion protein). In some embodiments, the group of polypeptides is not contiguous with each other, e.g., in different polypeptide chains. In some aspects, the group of polypeptides comprises a dimerization switch that can couple the polypeptides to each other in the presence of a dimerization molecule, e.g., can couple an antigen-binding domain to an intracellular signaling domain. In one aspect, the stimulatory molecule of the CAR is a ζ chain associated with a T cell receptor complex. In one aspect, the cytoplasmic signaling domain comprises a primary signaling domain (e.g., a CD3-ζ primary signaling domain). In one aspect, the cytoplasmic signaling domain further comprises one or more functional signaling domains derived from at least one co-stimulatory molecule as defined below. In one aspect, the co-stimulatory molecule may be selected from the group consisting of: 4-1BB (i.e., CD137), CD27, ICOS, and/or CD28. In one aspect, the CAR comprises a chimeric fusion protein, which may comprise an extracellular antigen recognition domain, a transmembrane domain, and an intracellular signaling domain comprising a functional signaling domain derived from a stimulatory molecule. In one aspect, the CAR comprises a chimeric fusion protein, which may comprise an extracellular antigen recognition domain, a transmembrane domain, and an intracellular signaling domain comprising a functional signaling domain derived from a co-stimulatory molecule and a functional signaling domain derived from a stimulatory molecule. In one aspect, the CAR comprises a chimeric fusion protein, which may comprise an extracellular antigen recognition domain, a transmembrane domain, and an intracellular signaling domain comprising a functional signaling domain derived from one or more co-stimulatory molecules and a functional signaling domain derived from a stimulatory molecule. In one aspect, the CAR comprises a chimeric fusion protein, which may comprise an extracellular antigen recognition domain, a transmembrane domain, and an intracellular signaling domain comprising at least two functional signaling domains derived from one or more co-stimulatory molecules and a functional signaling domain derived from a stimulatory molecule. In one aspect, the CAR comprises an optional leader sequence at the amino-terminus (N-ter) of the CAR fusion protein. In one aspect, the CAR further comprises a leader sequence at the N-terminus of the extracellular antigen recognition domain, wherein the leader sequence is optionally cleaved from the antigen recognition domain (e.g., VHH) during cell processing and localizes the CAR to the cell membrane.

In the present application, the term "antibody" generally refers to being used in the broadest sense and specifically encompasses monoclonal antibodies, polyclonal antibodies, dimers, polymers, multispecific antibodies (e.g., bispecific antibodies), and antibody fragments so long as they exhibit the desired biological activity (Miller et al., (2003) Jour. of Immunology 170: 4854-4861). The antibody may be a murine antibody, a human antibody, a humanized antibody, or a chimeric antibody, or derived from other species.

A full-length antibody typically refers to an antibody that consists of two "full-length antibody heavy chains" and two "full-length antibody light chains". The "Full-length antibody heavy chain" generally refers to a polypeptide consisting of, from the N-terminus to the C-terminus, an antibody heavy chain variable domain (VH), an antibody heavy chain constant domain 1 (CH1), an antibody hinge region (HR), an antibody heavy chain constant domain 2 (CH2), and an antibody heavy chain constant domain 3 (CH3), abbreviated as VH-CH1-HR-CH2-CH3; and in the case of antibodies of the IgE subclass, it optionally further comprises an antibody heavy chain constant domain 4 (CH4). In some embodiments, the "full-length antibody heavy chain" is a polypeptide consisting of, from the N-terminus to the C-terminus, VH, CH1, HR, CH2, and CH3. The "full-length antibody light chain" is generally a polypeptide consisting of, from the N-terminus to the C-terminus, an antibody light chain variable domain (VL) and an antibody light chain constant domain (CL), abbreviated as VL-CL. The antibody light chain constant domain (CL) may be κ (kappa) or λ (lambda). The two full-length antibody chains are linked together by inter-polypeptide disulfide bonds between the CL domain and the CH1 domain and between the hinge regions of the full-length antibody heavy chains. Examples of typical full-length antibodies are natural antibodies such as IgG (e.g., IgG1 and IgG2), IgM, IgA, IgD, and IgE.

In the present application, the term "antigen-binding fragment" (also referred to herein as a "targeting portion" or "antigen-binding portion") generally refers to a portion of an antibody molecule, which comprises amino acids responsible for specific binding between an antibody and an antigen. The portion of the antigen specifically recognized and bound by the antibody is referred to as an "epitope" described above. The antigen-binding domain may typically comprise an antibody light chain variable region (VL) and an antibody heavy chain variable region (VH); however, it does not necessarily comprise both. An Fd fragment, for example, has two VH regions and generally retains some of the antigen-binding functions of the intact antigen-binding domain. Examples of antigen-binding fragments of antibodies include: (1) a Fab fragment, a monovalent fragment having a VL, a VH, a light chain constant region (CL), and a CH1 domain; (2) a F(ab')$_2$ fragment, a bivalent fragment having two Fab fragments linked by a disulfide bridge in the hinge region; (3) an Fd fragment, having two VH and CH1 domains; (4) an Fv fragment, having VL and VH domains of a single arm of an antibody; (5) a dAb fragment (Ward et al., "Binding Activities of a Repertoire of Single Immunoglobulin Variable Domains Secreted From Escherichia coli," Nature 341:544-546(1989), which is incorporated herein by reference in its entirety), having a VH domain; (6) an isolated complementarity-determining region (CDR); (7) a single-chain Fv (scFv), e.g., derived from a scFV library, although the two domains VL and VH of the Fv fragment are encoded by independent genes, they may be joined using a recombinant method via a synthetic linker, and the synthetic linker enables the VL and VH regions to be prepared as a single protein chain (called single-chain Fv (scFv)), in which the VL and VH regions pair to form a monovalent molecule (see, for example, Huston et al., "Protein Engineering of Antibody Binding Sites: Recovery of Specific Activity in an Anti-Digoxin Single-Chain Fv Analogue Produced in Escherichia coli," Proc. Natl. Acad. Sci. USA 85:5879-5883(1988)); and (8) VHH, relating to a variable antigen-binding domain of a heavy-chain antibody derived from Camelidae (camel, dromedary, llama, alpaca, etc.) (see Nguyen V.K. et al., 2000, The EMBO Journal, 19, 921-930; Muyldermans S., 2001, J Biotechnol., 74, 277-302, and review Vanlandschoot P. et al., 2011, Antiviral Research 92, 389-407). VHH may also be referred to as nanobody (Nb) and/or single-domain antibody. These antibody fragments are obtained using conventional techniques known to those skilled in the art, and assessed for the function in the same manner as for intact antibodies.

In the present application, the term "single-domain antibody" or "VHH" generally refers to a class of antibodies that lack an antibody light chain and have only a heavy chain variable region. In certain cases, the single-domain antibody may be derived from Bactrian camels, dromedaries, alpacas, llamas, nurse sharks, smooth dogfishes or rays (see, e.g., Kang Xiaozhen et al., Chinese Journal of Biotechnology, 2018, 34(12): 1974-1984). For example, the single-domain antibody may be derived from alpacas. The single-domain antibody may consist of a heavy chain variable region (VH). The term "heavy chain variable region" generally refers to the amino-terminal domain of the heavy chain of an antigen-binding fragment. The heavy chain variable region may be further divided into hypervariable regions termed complementarity-determining regions (CDRs), which are scattered over more conserved regions termed framework regions (FRs). Each heavy chain variable region may consist of three CDRs and four FRs arranged from the amino-terminus to the carboxyl-terminus in the following order: FR1, CDR1, FR2, CDR2, FR3, CDR3, and FR4. The heavy chain variable region comprises a binding domain that interacts with an antigen.

In the present application, the term "complementarity-determining region" (CDR) generally refers to a complementarity-determining region within a variable region of an antigen-binding fragment. In the present application, there are 3 CDRs present in the heavy chain variable region, and the CDRs are designated HCDR1, HCDR2 and HCDR3 for each variable region. The exact boundaries of those CDRs have been defined differently according to different systems. The system described by Kabat (Kabat et al., Sequences of Proteins of Immunological Interest, National Institutes of Health, Bethesda, Md. (1987) and (1991)) provides not only a clear residue numbering system applicable to any variable region of an antigen-binding fragment, but also precise residue boundaries defining 3 CDRs. Those CDRs may be referred to as Kabat CDRs. Chothia and colleagues (Chothia and Lesk, J. Mol. Biol., 196: 901-917 (1987) and Chothia et al., Nature 342: 877-883(1989)) found that although there is large diversity at the amino acid sequence level, certain sub-portions within Kabat CDRs take almost identical peptide backbone conformations. Those sub-portions were designated L1, L2 and L3 or H1, H2 and H3, wherein "L" and "H" refer to the light and heavy chain regions, respectively. Those regions may be referred to as Chothia CDRs, which have boundaries that overlap with Kabat CDRs. Other boundaries defining CDRs that overlap with Kabat CDRs have been described by Padlan (FASEB J. 9: 133-139 (1995)) and MacCallum (J Mol Biol 262 (5): 732-45 (1996)). In addition, other CDR boundary definitions may not strictly follow one of the above systems, but will nevertheless overlap with Kabat CDRs, although they may be shortened or lengthened according to predictions or experimental findings that a particular residue or a particular group of residues, or even the entire CDR, does not significantly affect the antigen binding. In the present application, the IMGT numbering scheme is used.

In the present application, the term "FR" generally refers to the more highly conserved portions of antibody variable domains, which are referred to as framework regions. For example, the variable domains of natural heavy and light chains may each comprise four FR regions, namely four in VH (H-FR1, H-FR2, H-FR3, and H-FR4), and four in VL (L-FR1, L-FR2, L-FR3, and L-FR4). A "framework region" generally refers to a portion of the antibody variable region recognized in the art that is present between the more divergent (i.e., hypervariable) CDRs. Such framework regions are typically referred to as frameworks 1 to 4 (FR1, FR2, FR3, and FR4) and provide a backbone for presenting six CDRs (three from the heavy chain and three from the light chain) in the three-dimensional space to form an antigen-binding surface.

In the present application, the term "homology" may generally be equivalent to sequence "identity". A homologous sequence may include an amino acid sequence that may be at least 80%, 85%, 90%, 99.1%, 99.2%, 99.3%, 99.4%, 99.5%, 99.6%, 99.7%, 99.8%, or 99.9% identical to a subject sequence. Generally, the homolog will comprise the same active site, etc. as the subject amino acid sequence. Homology may be considered in terms of similarity (i.e., amino acid residues having similar chemical properties/functions), or may be expressed in terms of sequence identity. In the present application, reference to a sequence having a percent identity of any one of the SEQ ID NOs of an amino acid sequence or a nucleotide sequence refers to a sequence having the percent identity over the entire length of the referenced SEQ ID NO.

To determine sequence identity, sequence alignments can be performed by various means known to those skilled in the art, e.g., using BLAST, BLAST-2, ALIGN, NEEDLE, or Megalign (DNASTAR) software, etc. Those skilled in the art can determine appropriate parameters for alignment, including any algorithms required to achieve optimal alignment over the full length of the sequences being compared.

In the present application, the term "functional variant" generally refers to a variant comprising substantially the same function as the amino acid sequence (e.g., may possess the properties of the chimeric antigen receptor) and comprising an amino acid sequence having at least 85% (e.g., at least 85%, at least 90%, at least 91%, at least 92%, at least 93%, at least 94%, at least 95%, at least 96%, at least 97%, at least 98%, at least 99%, or at least 100%) sequence identity to the amino acid sequence. In some embodiments, the variant of the amino acid sequence is an amino acid sequence having substantially the same function as the amino acid sequence (e.g., may possess the properties of the chimeric antigen receptor) and comprising, on its basis, substitution, deletion, or addition of one or more (e.g., 1-2, 1-3, 1-4, 1-5, 1-6, 1-7, 1-8, 1-9, 1-10, or more) amino acids.

In the present application, the term "specific binding" when referring to the interaction of a binding molecule (e.g., an antibody) with its binding partner (e.g., an antigen) generally means that the interaction is dependent on the presence of a specific structure (e.g., an antigenic determinant or epitope) on the binding partner. In other words, the antibody will preferentially bind to or recognize a binding partner even when the binding partner is present in a mixture of other molecules or organisms. The binding may be mediated by covalent or non-covalent interaction or a combination of both. In other words, the term "specific binding" generally refers to the immunospecific binding to an antigenic determinant or epitope without immunospecific binding to other antigenic determinants or epitopes. A binding molecule with immunospecific binding to an antigen may bind to other peptides or polypeptides with relatively low affinity as determined by, for example, radioimmunoassay (RIA), enzyme-linked immunosorbent assay (ELISA), BIACORE, or other assays known in the art. The binding molecule or the fragment thereof with immunospecific binding to an antigen may cross-react with a related antigen with the same epitope. In certain cases, the binding molecule or the fragment thereof with immunospecific binding to an antigen does not cross-react with other antigens.

In the present application, the term "$KD$" is used interchangeably with "KD" and generally refers to a dissociation equilibrium constant, in M (mol/L), of a particular antibody-antigen interaction. $KD$ may be calculated from the concentration of substance AB and the concentration of substance A and substance B resulting from its dissociation: $KD = c(A) \times c(B) / c(AB)$. It can be seen from this equation that a larger $KD$ indicates more dissociation and weaker affinity between substances A and B; conversely, a smaller $KD$ indicates less dissociation and stronger affinity between substances A and B.

As will be understood by those skilled in the art, the sequences of various IL-15 molecules are known in the art. In one aspect, the IL-15 is wild-type IL-15. In some aspects, the IL-15 is mammalian IL-15 (e.g., homo sapiens interleukin 15 (IL15), transcript variant 3, mRNA, NCBI reference sequence: NM_000585.4; canis lupus familiaris interleukin 15, mRNA, NCBI reference sequence: NM_001197188.1; felis catus interleukin 15 (IL15), mRNA, NCBI reference sequence: NM_001009207.1). Examples of "mammalia" or "mammal" include primates (e.g., humans), canines, felines, rodents, swine, ruminants, and the like. Specific examples include humans, dogs, cats, horses, cattle, sheep, goats, rabbits, guinea pigs, rats, and mice. In one specific aspect, the mammalian IL-15 is human IL-15.

In the present application, the term "isolated" generally refers to a molecule (e.g., an antibody, a nucleic acid, etc.) that is at least partially isolated from other molecules to which it is generally bonded in its natural state. The "isolated polypeptide" is substantially free of other biomolecules, such as nucleic acids, proteins, lipids, carbohydrates, cell debris, and growth media. The "isolated nucleic acid" is at least partially isolated from a nucleic acid that generally flanks a polynucleotide in its natural state.

In the present application, the term "isolated polypeptide" generally refers to any polypeptide comprising more than one different polypeptide chain. In some embodiments, the isolated polypeptide comprises two polypeptide chains, which comprise a chimeric antigen receptor and an enhancer, respectively. In some embodiments, the polypeptide chain comprising a chimeric antigen receptor and the polypeptide chain comprising an enhancer may be linked together via one or more intermolecular bonds (including, but not limited to, disulfide bonds) to form a macromolecular complex of two or more polypeptides. In some embodiments, the isolated polypeptide may comprise a single polypeptide of two different polypeptide chains (e.g., a chimeric antigen receptor and an enhancer) linked via a polypeptide linker. In this case, the polypeptides may physically represent a single chain, but two or more portions of the single chain can function as if they are two separate polypeptide chains. In some other embodiments, the linker is a cleavable peptide linker (e.g., a 2A peptide), and the chimeric antigen receptor and the enhancer on the same peptide chain can be cleaved into two polypeptide chains.

In the present application, the term "isolated nucleic acid molecule" generally refers to an isolated form of nucleotides, deoxyribonucleotides or ribonucleotides or analogs thereof of any length, isolated from their natural environment, or artificially synthesized.

The "internal ribosome entry site" or "IRES" as used herein refers to an element that facilitates direct entry of internal ribosomes to the start codon (e.g., ATG) of the cistron (protein coding region), resulting in translation of the cap-independent gene, see, e.g., Jackson et al., 1990, Trends Biochem Sci 15(12): 477-83, and Jackson and Kaminski., 1995, RNA 1(10): 985-1000. In specific embodiments, the vector contemplated by the present disclosure comprises one or more target polynucleotides encoding one or more polypeptides. In specific embodiments, to achieve efficient translation of various polypeptides, the polynucleotide sequences may be separated by one or more IRES sequences or polynucleotide sequences encoding self-cleaving polypeptides.

In the present application, the term "self-cleaving peptide" or "sequence encoding a self-cleaving peptide" generally refers to a linker sequence used in a vector to incorporate a site that facilitates ribosome skipping and thereby produce two polypeptides from a single promoter, such self-cleaving peptides including, but not limited to, T2A and P2A peptides or sequences encoding self-cleaving peptides.

In the present application, the term "vector" generally refers to a nucleic acid molecule capable of self-replicating in a suitable host, which transfers an inserted nucleic acid molecule into a host cell and/or between host cells. The vector may include vectors primarily for the insertion of DNA or RNA into a cell, vectors primarily for the replication of DNA or RNA, and vectors primarily for the expression of transcription and/or translation of DNA or RNA. The vector further includes vectors having a variety of the above-described functions. The vector may be a polynucleotide capable of being transcribed and translated into a polypeptide when introduced into a suitable host cell. Generally, the vector can produce the desired expression product by culturing an appropriate host cell comprising the vector.

In the present application, the term "viral vector" is used broadly to refer to a nucleic acid molecule (e.g., transfer plasmid) or viral particle that mediates the transfer of nucleic acids. The nucleic acid molecule includes virus-derived nucleic acid elements that generally facilitate the transfer or integration of the nucleic acid molecules into the genome of a cell. The viral particle generally includes various viral components and sometimes further includes host cell components in addition to nucleic acids. The viral vector may refer to a virus or viral particle capable of transferring nucleic acids into a cell, or the transferred nucleic acid itself.

In the present application, the term "lentivirus" generally refers to a group (or genus) of complex retroviruses. Exemplary lentiviruses include, but are not limited to: human immunodeficiency virus (HIV; including HIV type 1 and HIV type 2); visna-maedivirus (VMV); caprine arthritis-encephalitis virus (CAEV); equine infectious anemia virus (EIAV); feline immunodeficiency virus (FIV); bovine immunodeficiency virus (BIV); and simian immunodeficiency virus (SIV). In one embodiment, an HIV-based vector backbone (i.e., HIV cis-acting sequence element) is preferred. In particular embodiments, the lentivirus is used to deliver a polynucleotide comprising the CAR to a cell.

In the present application, the term "host cell" or "cell" generally refers to an individual cell, cell line, or cell culture that may contain or has contained a vector comprising the isolated nucleic acid molecule described herein, or that is capable of expressing the isolated antigen-binding fragment described herein. The host cell may comprise progeny of a single host cell. Due to natural, accidental or deliberate mutations, progeny cells may not necessarily be identical in morphology or in genome to the original parent cell, but are capable of expressing the isolated antigen-binding fragment described herein. The host cell may be obtained by transfecting cells with the vector described herein *in vitro.* The host cell may be a prokaryotic cell (e.g., *E. coli*) or a eukaryotic cell (e.g., a yeast cell, a COS cell, a Chinese hamster ovary (CHO) cell, a HeLa cell, an HEK293 cell, a COS-1 cell, an NS0 cell, or a myeloma cell). For example, the host cell may be an *E. coli* cell. For example, the host cell may be a yeast cell. For example, the host cell may be a mammalian cell. For example, the mammalian cell may be a CHO-K1 cell.

In the present application, the term "T cell" or "T lymphocyte" may be any T cell, such as a cultured T cell, e.g., a primary T cell, or a T cell from the cultured T cell line, or a T cell obtained from a mammal (preferably a primate, species including monkey, dog, or human). If obtained from a mammal, the T cells may be obtained from a number of sources including, but not limited to, blood, bone marrow, lymph nodes, thymus, or other tissues or fluids. The T cell may also be enriched or purified. The T cell may be obtained by maturing a hematopoietic stem cell into a T cell *in vitro* or *in vivo.* In exemplary aspects, the T cell is a human T cell. In exemplary aspects, the T cell is a T cell isolated from a human. The T cell may be any type of T cell, including NKT cells, and may have any developmental stage, including but not limited to, CD4+/CD8+ double positive T cells; CDA+ helper T cells; e.g., Th1 and Th2 cells, CD8+ T cells (e.g., cytotoxic T cells); peripheral blood mononuclear cells (PBMCs); peripheral blood leukocytes (PBLs); tumor infiltrating cells (TILs); memory T cells; untreated T cells, and the like. Preferably, the T cell is a CD8+ T cell or a CD4+ T cell. In some alternatives, the T cell is allogeneic (from different donors of the same species) to the recipient subject that receives the cell or cell to be received (e.g., the cells are in the form of a therapeutic composition); in some alternatives, the T cell is autologous (the donor and recipient are the same); in some alternatives, the T cell is syngeneic (the donor and recipient are different, but are homozygotic twins).

In the present application, the term "natural killer cell" ("NK cell") generally refers to a cytotoxic lymphocyte in the immune system. NK cells provide a rapid response to virus-infected cells and respond to transformed cells. Generally, immune cells detect peptides from pathogens that are presented by major histocompatibility complex (MHC) molecules on the surface of infected cells, thereby triggering cytokine release, causing lysis or apoptosis. However, NK cells are unique, as they have the ability to recognize stressed cells regardless of whether peptides from pathogens are present on MHC molecules. They are named "natural killer cells" because of the initial notion that they do not require pre-activation to kill targets. NK cells are large granular lymphocytes (LGLs) and are known to differentiate and mature in the bone marrow (where they then enter the circulation). In some embodiments, the NK cell is a mammalian NK cell. Examples of "mammalia" or "mammal" include primates (e.g., humans), canines, felines, rodents, swine, ruminants, and the like. Specific examples include humans, dogs, cats, horses, cattle, sheep, goats, rabbits, guinea pigs, rats, and mice. In one embodiment, the mammalian NK cell is a human NK cell.

In the present application, the term "immune effector cell" generally refers to an immune cell involved in an immune response and performing an effector function. For example, performing the effector function may include clearing foreign antigens, promoting an immune effector response, or the like. The immune effector cells may include plasma cells, T cells, B cells, natural killer (NK) cells, natural killer T (NKT) cells, mast cells, and myeloid-derived phagocytes.

The immune effector cell of the present application may be autologous/autogeneic ("self") or non-autologous ("non-self", e.g., allogeneic, syngeneic, or xenogeneic).

In the present application, the term "autologous" generally refers to cells from the same subject. "Allogeneic" generally means that cells are of the same species as but genetically different from the cells to which they are compared. "Syngeneic" generally means that cells are from different subjects but genetically identical to the cells to which they are compared. "Xenogeneic" generally means that cells are of different species from the cell to which they are compared. In some embodiments, the cells of the present application are autologous or allogeneic.

In the present application, the term "modification" generally refers to changing the state or structure of the cell and/or a change in the state or structure of the cell. The change is generally compared to the state or structure of the corresponding unmodified cell. The change may include a change in endogenous gene expression level or function, for example, a down-regulation, up-regulation, or non-expression of the endogenous gene expression level of the cell by genetic engineering means, which may include homologous recombination, CRISPR/Cas9 system gene editing, or the like; the change may also include a change in cellular protein expression, structure, or function, for example, a change in the expression, structure, or function of the corresponding protein achieved by the change in the expression level or function of the endogenous gene, such as a change in the expression, structure or function of a protein achieved by the regulation of protein translation and post-translational modification; the change may also include the introduction of foreign genes, the expression of foreign proteins, and the like.

In the present application, the term "TRAC" generally refers to a T cell receptor alpha constant. A T cell receptor (TCR) generally refers to a specific receptor located on the surface of the T cell, which is capable of recognizing antigens that bind to major histocompatibility complex (MHC) molecules. TCRs are generally composed of two different protein chains (i.e., heterodimers). In humans, TCRs in most T cells are composed of one $\alpha$ chain and one $\beta$ chain (encoded by TRA and TRB, respectively), and this class of T cells is referred to as $\alpha\beta$ T cells; and in a few T cells, TCRs are composed of $\gamma$ chain and $\delta$ chain (encoded by TRG and TRD, respectively), and this class of T cells is referred to as $\gamma\delta$ T cells. Generally, $\alpha\beta$ T cells account for about 95% of the total T cells, $\gamma\delta$ T cells account for about 5% of the total T cells, and the ratios vary during ontogenesis and in diseased states (e.g., leukemia), and also differ among species. Each chain constituting TCRs comprises a variable region and a constant region. In humans, the gene encoding $\alpha$ chain (TRA, e.g., information as shown by HGNC: 12027) is located on chromosome 14 and consists of multiple gene fragments, including a variable fragment (V), a joining fragment (J), and a constant fragment (C). The TRAC gene generally refers to a gene sequence encoding the T cell receptor $\alpha$ chain constant region (C) (e.g., information as shown by HGNC:12029) and is located on chromosome 14 (14q11.2; 14:22,547,505-22,552,131). Generally, one of the genes of the variable fragments (V) encoding the N-fragment of the antigen recognition domain is rearranged with one of the joining fragments (J) to produce a functional V-region exon, which is transcribed and linked to the constant region (C) by splicing, thereby forming a coding sequence of the T cell receptor $\alpha$ chain.

In the present application, the term "major histocompatibility complex antigen" ("MHC", also referred to as "human leukocyte antigen" ("HLA") in humans) generally refers to a protein expressed on the surface of a cell that confers a unique antigenic identity to the cell. MHC/HLA antigens are target molecules that are recognized by T cells and NK cells as being derived from the same source of hematopoietic stem cells as immune effector cells ("self") or as being derived from another source of hematopoietic repopulating cells ("non-self"). Two major classes of HLA antigens are recognized: HLA class I and HLA class II. HLA class I antigens (A, B, and C in humans) allow each cell to be recognized as "self", while HLA class II antigens (DR, DP, and DQ in humans) are involved in reactions between lymphocytes and antigen-presenting cells. Both have been implicated in the rejection of transplanted organs. An important aspect of the HLA gene system is its polymorphism. Each gene for MHC class I (A, B, and C) and MHC class II (DP, DQ, and DR) exists in different alleles. HLA alleles are designated by numbers and subscripts. For example, two unrelated individuals may carry class I HLA-B genes B5 and Bw41, respectively. Allelic products differ in one or more amino acids of the $\alpha$ and/or $\beta$ domains. A number of specific antibodies or nucleic acid reagents are used to type HLA haplotypes of individuals using leukocytes that express class I and class II molecules. Genes commonly used for HLA typing are six MHC class I and II proteins, i.e., two alleles for each of HLA-A, HLA-B, and HLA-DR. The HLA genes are clustered in a "super locus" present on chromosome position 6p21, wherein the "super locus" encodes 6 classical transplantation HLA genes and at least 132 protein-coding genes that play important roles in the regulation of the immune system as well as some other fundamental molecular and cellular processes. The complete locus measures roughly 3.6 Mb with at least 224 loci. One effect of such clustering is that a "haplotype", i.e., a group of alleles present on a single chromosome, is inherited from one parent and tends to be inherited as a group. The group of alleles inherited from each parent forms a haplotype, in which some alleles tend to be associated together. Identifying haplotypes of a patient may help predict the probability of finding a matching donor and help formulate a search strategy, because some alleles and haplotypes are more common than others and they are distributed at different frequencies in different racial and ethnic groups.

In the present application, "HLA-A" generally refers to a class of human leukocyte antigen polypeptide chains encoded by an HLA-A gene located on human chromosome 6p21.3 (e.g., information as shown by HGNC:4931). HLA-A is one of the three major polypeptide types that constitute MHC class I molecules on the surface of human cells, and others further include HLA-B and HLA-C. A heterodimer composed of an α chain encoded by the HLA-A gene and a β chain encoded by a B2M gene (β2-microglobulin) is an HLA-A class MHC I molecule. The α chain encoded by the HLA-A gene may comprise an α1 domain, an α2 domain, an α3 domain, a transmembrane region, and a cytoplasmic region, wherein the α1 domain and the α2 domain may bind to a peptide fragment so as to present the peptide fragment to an immune cell by the MHC I molecule (e.g., HLA-A class). In humans, similar to most mammals, the α chain of the MHC I molecule is polymorphic, and there are many variations in the primary structure thereof. As of December 2013, there are 2432 known HLA-A alleles in total, which encode 1740 active proteins and 117 inactive proteins. In the present application, HLA-A alleles may include sequence information on different HLA-A alleles recorded in the IMGT/HLA database version 3.38.0 (https://www.ebi.ac.uk/ipd/imgt/hla/) and designated by the WHO HLA Factor Nomenclature Committee.

In the present application, the term "HLA-B" generally refers to a part of the gene family of human leukocyte antigen (HLA) complexes. HLA is a human version of the major histocompatibility complex (MHC), and MHC is a gene family present in many species. The complex genes are divided into three basic groups: class I, class II, and class III. In humans, the HLA-B gene and the two related genes HLA-A and HLA-C are the major genes of MHC class I. The HLA-B gene is located in the cell band 21.3 of the short (p) arm of chromosome 6 from base pairs 31,353,871 to 31,357,211. HLA-B is one of the three major HLAs that should be matched between the donor and recipient. They are HLA-A, HLA-B (both are MHC class I), and HLA-DR (MHC class II). If two tissues have the same genes encoding the three HLAs, the possibility and severity of rejection are minimized. Hundreds of versions (alleles) of HLA-B are known, each version having a specific number (e.g., HLA-B27). Closely related alleles are grouped together, for example, at least 28 very similar alleles are subtypes of HLA-B27. These subtypes are designated as HLA-B*2701 to HLA-B*2728.

In the present application, the term "HLA-matched" refers to a donor-recipient pair in which none of the HLA antigens are mismatched between the donor and recipient, such as a donor providing a hematopoietic stem cell graft to a recipient in need of hematopoietic stem cell transplantation therapy. HLA-matched (i.e., in which all 6 alleles are matched) donor-recipient pairs have a reduced risk of graft rejection, because endogenous T cells and NK cells are less likely to recognize the incoming graft as foreign, and are thus less likely to generate an immune response against the graft.

In the present application, the term "HLA-mismatched" refers to a donor-recipient pair in which at least one HLA antigen (particularly with respect to HLA-A, HLA-B, and HLA-DR) is mismatched between the donor and recipient, such as a donor providing a hematopoietic stem cell graft to a recipient in need of hematopoietic stem cell transplantation therapy. In some embodiments, one haplotype is matched, and the other is mismatched. HLA-mismatched donor-recipient pairs may have an increased risk of graft rejection relative to HLA-matched donor-recipient pairs, because endogenous T cells and NK cells are more likely to recognize the incoming graft as foreign in the case of HLA-mismatched donor-recipient pairs, and such T cells and NK cells are thus more likely to generate an immune response against the graft.

In the present application, the term "B2M" generally refers to β2-microglobulin, which is one of the components of MHC class I molecules. β2 microglobulin (also referred to as β chain) may form an MHC class I molecule with an α chain encoded by HLA. B2M is generally expressed in all nucleated cells. In humans, β2 microglobulin is encoded by the B2M gene located at 15q21.1 (e.g., information as shown by HGNC:914).

In the present application, the term "CIITA" generally refers to a transactivator of a class II major histocompatibility complex (MHC II). The transactivator may be a protein having an acidic transcriptional activation domain, 4 LRRs (leucine-rich repeats), and a GTP binding domain. The protein may be located in the cell nucleus, act as a positive regulator of the gene transcription of the class II major histocompatibility complex (MHC II), and be referred to as a "master control factor" for the expression of these genes. The protein may also bind to GTP and utilize the binding to GTP to transport itself into the cell nucleus, where it generally functions by acetyltransferase (AT) activity in a coactivator-like manner. In humans, the protein is encoded by a gene located at 16p13.13 (e.g., information as shown by HGNC:7067), and several transcript variants encoding different isoforms can be produced.

In the present application, the term "wild-type cell" generally refers to a cell that naturally occurs or is of natural origin.

In the present application, the term "nucleic acid" or "polynucleotide" or "nucleic acid molecule" generally refers to deoxyribonucleic acid (DNA) or ribonucleic acid (RNA) and polymers thereof in either single-stranded or double-stranded form. Unless specifically limited, the term may include nucleic acids comprising analogs of natural nucleotides that have similar binding properties as the reference nucleic acid (e.g., with sequence information shown) and are metabolized in a manner similar to naturally occurring nucleotides. Unless otherwise indicated, the sequence of a nucleic acid may include conservatively modified variants thereof (e.g., degenerate codon substitutions), alleles, orthologs, SNPs, and complementary sequences, as well as the sequences explicitly indicated.

In the present application, the term "expression" generally refers to the transcription and/or translation of a particular nucleotide sequence.

In the present application, the term "gene mutation" generally refers to a change in the composition or arrangement order of base pairs occurring in the structure of genes, such as a point mutation caused by a single base change, or deletion, duplication, insertion, and the like of a plurality of bases.

In the present application, the term "gene silencing" generally refers to the prevention of the expression of certain genes by regulatory mechanisms. The gene silencing may primarily include two types: one is transcriptional gene silencing (TGS) caused by factors such as DNA methylation, heterochromatization, and position effect at the transcriptional level, and the other is post-transcriptional gene silencing (PTGS), which is the effect on gene expression at the post-transcriptional level by specific intervention in the target RNA. Generally, when a gene is silenced, the expression of the corresponding gene is down-regulated/reduced. When a gene is knocked out, it is generally not expressed. For example, the expression of a specific gene in a cell disappears when all alleles of the specific gene are knocked out. Gene silencing is generally considered to be a gene knockdown mechanism, and methods commonly used to silence genes may be, for example, RNAi and the like.

In the present application, the term "endogenous" refers to any substance derived from or produced within an organism, a cell, a tissue, or a system.

In the present application, the term "exogenous" refers to any substance introduced from or produced outside of an organism, a cell, a tissue, or a system.

In the present application, the term "antisense RNA" generally refers to a single-stranded RNA complementary to a transcript mRNA (messenger RNA). The antisense RNA may inhibit the expression of genes by binding to mRNA. For example, the binding of the antisense RNA to the target mRNA results in an increased sensitivity of the double-stranded RNA molecule to RNA enzyme III, and causes the degradation of the double-stranded RNA molecule. For example, the antisense RNA binds to an upstream non-coding region of mRNA, thereby directly inhibiting the translation of the target mRNA.

In the present application, the term "siRNA" generally refers to the abbreviation of small interfering RNA or short interfering RNA. siRNA is a class of double-stranded, non-coding RNA molecules that are about 18-28 base pairs in length and may cause the degradation of mRNA by the complementary binding to mRNA, thereby interfering with the expression of a specific gene. In some embodiments, siRNA may be a product obtained by treating a long double-stranded RNA or shRNA with Dicer enzyme. In some embodiments, siRNA enters a cell to form an RNA-induced silencing complex (RISC) with other proteins, the sense strand is degraded, and the antisense strand may bind to a complementary targeting sequence, thereby achieving gene silencing.

In the present application, the term "shRNA" generally refers to the abbreviation of short hairpin RNA, i.e., "short hairpin RNA". shRNA generally comprises two short inverted repeats separated by a stem-loop sequence to form a hairpin structure. Generally, shRNA may further comprise 5-6 T bases as transcription terminators for RNA polymerase III. In some embodiments, shRNA may enter a cell via a viral vector or plasmid, and be transcribed under the action of polymerase II or polymerase III. The transcripts are exported from the cell nucleus (generally via Exportin 5), and then transported to RISC after Dicer treatment. The sense strand is degraded, and the antisense strand may bind to a complementary targeting sequence, thereby achieving gene silencing.

In the present application, the term "CRISPR/Cas system" generally refers to a group of molecules comprising an RNA-guided nuclease or other effector molecules and a gRNA molecule, and the molecules are capable of directing and realizing modification of a nucleic acid by the RNA-guided nuclease or other effector molecules at a target sequence, e.g., causing degradation of the target sequence. In some embodiments, the CRISPR system comprises gRNA and a Cas protein, e.g., Cas9 protein. A system comprising Cas9 or a functional mutant thereof is referred to herein as "Cas9 system" or "CRISPR/Cas9 system". In some embodiments, the gRNA molecule and Cas molecule may be complexed to form a ribonucleoprotein (RNP) complex.

In the present application, the terms "gRNA molecule", "guide RNA", "instruction RNA", "direct RNA", "guide RNA molecule", and "gRNA" can be used interchangeably and generally refer to a nucleic acid molecule capable of facilitating the specific guidance of the RNA-guided nuclease or other effector molecules (generally complexed with a gRNA molecule) onto the target sequence. In some embodiments, the guidance is achieved by the hybridization of a portion of gRNA with DNA (e.g., via a gRNA guide domain) and by the binding of a portion of the gRNA molecule to the RNA-guided nuclease or other effector molecule (e.g., at least via gRNAtracr). In some embodiments, the gRNA molecule consists of a single, contiguous polynucleotide molecule, referred to herein as a "single guide RNA", "sgRNA", or the like. In other embodiments, the gRNA molecule consists of multiple (e.g., two) polynucleotide molecules that are themselves capable of association (typically by hybridization), referred to herein as a "dual guide RNA", "dgRNA", or the like.

In the present application, the term "Cas protein" generally refers to an enzyme responsible for cleaving DNA in the CRISPR/Cas system. The enzyme may include enzymes from types I, II, and III CRISPR/Cas systems, e.g., Cas3, Cas9, and Cas10.

In the present application, the term "Cas9 protein" generally refers to an enzyme responsible for cleaving DNA, which is from the bacterial type II CRISPR/Cas system. Cas9 may include wild-type proteins and functional mutants thereof.

In the present application, "allele" generally refers to a form of a gene sequence at a locus that may have different variations. The locus is also referred to as a gene site or site and refers to a fixed position on a chromosome, e.g., where a gene is located. The arrangement of a locus in the genome is referred to as a genetic map.

In the present application, the term "homozygote" generally refers to a genotype individual in which two alleles of homologous chromosomes are identical at the same locus. A pair of opposing genes may have individuals with two genotypes, AA and aa.

In the present application, the term "heterozygote" generally refers to a genotype individual in which two alleles at the same site on homologous chromosomes in a diploid are not identical, such as Aa. Heterozygous genotypes are generally more adaptive than homozygous dominant or homozygous recessive genotypes, and such a phenomenon is referred to as heterozygote advantage.

In the present application, the terms "tumor" and "cancer" are used interchangeably and generally refer to a disease characterized by rapid and uncontrolled growth of abnormal cells. Cancer cells can spread to other parts of the body locally or through the bloodstream and lymphatic system. Examples of various cancers are described herein and include, but are not limited to, breast cancer, prostate cancer, ovarian cancer, cervical cancer, skin cancer, pancreatic cancer, colorectal cancer, kidney cancer, liver cancer, brain cancer, lymphoma, leukemia, lung cancer, and the like. The term "cancer" or "tumor" includes premalignant and malignant cancers and tumors, and also encompasses solid tumors and non-solid tumors.

In the present application, the term "pharmaceutically acceptable" generally refers to those compounds, materials, compositions, and/or dosage forms that are, commensurate with a reasonable benefit/risk ratio, and suitable, within the scope of sound medical judgment, for use in contact with the tissues of human beings and animals without excessive toxicity, irritation, allergic response, or other problems or complications.

In the present application, the term "pharmaceutically acceptable carrier" generally refers to any of those carriers conventionally used and is limited only by physical or chemical factors (such as solubility and lack of reactivity with active binding agents) and by the route of administration. The pharmaceutically acceptable carrier, such as a vehicle, an adjuvant, an excipient, and a diluent, described herein is well known to those skilled in the art and readily available to the public. In one aspect, the pharmaceutically acceptable carrier is one that is chemically inert to an active ingredient of a pharmaceutical composition and one that does not have adverse side effects or toxicity under the conditions of use. In some embodiments, the carrier does not produce an adverse, allergic, or other untoward reaction when administered to an animal or human. In some aspects, the pharmaceutical composition does not comprise pyrogens and other impurities that may be harmful to humans or animals. The pharmaceutically acceptable carrier includes any and all solvents, dispersion media, coatings, antibacterial and antifungal agents, isotonic and absorption delaying agents, and the like; the use of the pharmaceutically acceptable carrier is well known in the art.

The acceptable carriers, excipients, or stabilizers are non-toxic to recipients and are preferably inert at the doses and concentrations employed, and include buffers, such as phosphate, citrate, or other organic acids; antioxidants, such as ascorbic acid; low molecular weight polypeptides; proteins, such as serum albumin, gelatin, or immunoglobulin; hydrophilic polymers, such as polyvinylpyrrolidone; amino acids, such as glycine, glutamine, asparagine, arginine, or lysine; monosaccharides, disaccharides, and other carbohydrates including glucose, mannose, or dextrin; chelating agents, such as EDTA; sugar alcohols, such as mannitol or sorbitol; salt-forming counter-ions, such as sodium; and/or non-ionic surfactants, such as Tween, Pluronics, or polyethylene glycol (PEG).

In the present application, the term "effective amount" or "effective dose" generally refers to an amount sufficient to achieve, or at least partially achieve, a desired effect. "Therapeutically effective amount" or "therapeutically effective dose" of a drug or therapeutic agent is generally any amount of the drug that promotes the regression of a disease (as evidenced by a decrease in the severity of disease symptoms, an increase in the frequency and duration of the asymptomatic phase of the disease, or the prevention of damage or disability due to the development of the disease) when being used alone or in combination with another therapeutic agent.

"Therapeutically effective amount" or "effective amount" of an anti-B7H3 CAR-T cell is also an amount or dose that has a therapeutically beneficial effect over any toxic or deleterious effects, such as CRS, of the anti-B7H3 CAR-T cell. The term "therapeutically effective amount" includes an amount effective to "treat" a subject (e.g., a patient). In one embodiment, the therapeutically effective dose is the minimum effective dose (MED) of the anti-B7H3 CAR-T cell for treating multiple myeloma in the subject. In one embodiment, the therapeutically effective dose is the maximum tolerated dose (MTD) of the anti-B7H3 CAR-T cell that does not cause the subject to have unresolved CRS.

In the present application, the term "comprise" or "comprising" generally means including, summarizing, containing or encompassing. In certain cases, the term also means "being" or "consisting of ...".

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

In the present application, the term "subject" generally refers to a human or non-human animal, including but not limited to cats, dogs, horses, pigs, cows, sheep, rabbits, mice, rats, monkeys, and the like.

**Detailed Description of Invention**

<u>B7H3-binding polypeptide</u>

In one aspect, the present application provides an isolated B7H3-binding polypeptide comprising a first heavy chain variable region of a heavy-chain antibody (VHH-1) and a second heavy chain variable region of a heavy-chain antibody (VHH-2), both targeting B7H3, wherein the VHH-1 comprises an HCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 1, an HCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 3, and an HCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 5;

the VHH-2 comprises an HCDR1 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 2, an HCDR2 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 4, and an HCDR3 comprising an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 6.

For example, the B7H3-binding polypeptide comprises a first heavy chain variable region of a heavy-chain antibody (VHH-1) and a second heavy chain variable region of a heavy-chain antibody (VHH-2), both targeting B7H3, wherein the VHH-1 may comprise an HCDR1 set forth in SEQ ID NO: 1, an HCDR2 set forth in SEQ ID NO: 3, and an HCDR3 set forth in SEQ ID NO: 5; the VHH-2 may comprise an HCDR1 set forth in SEQ ID NO: 2, an HCDR2 set forth in SEQ ID NO: 4, and an HCDR3 set forth in SEQ ID NO: 6.

In some embodiments, the VHH-1 comprises a heavy chain framework region 1 (HFR1), a heavy chain framework region 2 (HFR2), a heavy chain framework region 3 (HFR3), and a heavy chain framework region 4 (HFR4), wherein the HFR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 7 or SEQ ID NO: 8. The HFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 11. The HFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 13 or SEQ ID NO: 14. The HFR4 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 16.

In some embodiments, the VHH-2 comprises a heavy chain framework region 1 (HFR1), a heavy chain framework region 2 (HFR2), a heavy chain framework region 3 (HFR3), and a heavy chain framework region 4 (HFR4), wherein the HFR1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 9 or SEQ ID NO: 10. The HFR2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 12. The HFR3 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 14 or SEQ ID NO: 15. The HFR4 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 17.

For example, the VHH-1 comprises an HFR1, an HFR2, an HFR3, and an HFR4, and the HFR1, the HFR2, the HFR3, and the HFR4 may be selected from the group consisting of:

i) an HFR1 comprising the amino acid sequence set forth in SEQ ID NO: 7, an HFR2 comprising the amino acid sequence set forth in SEQ ID NO: 11, an HFR3 comprising the amino acid sequence set forth in SEQ ID NO: 13, and an HFR4 comprising the amino acid sequence set forth in SEQ ID NO: 16; and

ii) an HFR1 comprising the amino acid sequence set forth in SEQ ID NO: 8, an HFR2 comprising the amino acid sequence set forth in SEQ ID NO: 11, an HFR3 comprising the amino acid sequence set forth in SEQ ID NO: 14, and an HFR4 comprising the amino acid sequence set forth in SEQ ID NO: 17.

For example, the VHH-2 comprises an HFR1, an HFR2, an HFR3, and an HFR4, and the HFR1, the HFR2, the HFR3, and the HFR4 may be selected from the group consisting of:

i) an HFR1 comprising the amino acid sequence set forth in SEQ ID NO: 9, an HFR2 comprising the amino acid sequence set forth in SEQ ID NO: 12, an HFR3 comprising the amino acid sequence set forth in SEQ ID NO: 15, and an HFR4 comprising the amino acid sequence set forth in SEQ ID NO: 16; and

ii) an HFR1 comprising the amino acid sequence set forth in SEQ ID NO: 10, an HFR2 comprising the amino acid sequence set forth in SEQ ID NO: 12, an HFR3 comprising the amino acid sequence set forth in SEQ ID NO: 14, and an HFR4 comprising the amino acid sequence set forth in SEQ ID NO: 17.

In some embodiments, the VHH-1 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 18 or SEQ ID NO: 19. For example, the VHH-1 may comprise the amino acid sequence set forth in SEQ ID NO: 18 or SEQ ID NO: 19.

In some embodiments, the VHH-2 comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 20 or SEQ ID NO: 21. For example, the VHH-2 may comprise the amino acid sequence set forth in SEQ ID NO: 20 or SEQ ID NO: 21.

For example, the B7H3-binding polypeptide comprises a first heavy chain variable region of a heavy-chain antibody (VHH-1) and a second heavy chain variable region of a heavy-chain antibody (VHH-2), both targeting B7H3, wherein the VHH-1 comprises the amino acid sequence set forth in SEQ ID NO: 18 or SEQ ID NO: 19, and/or the VHH-2 comprises the amino acid sequence set forth in SEQ ID NO: 20 or SEQ ID NO: 21.

In some embodiments, the VHH-1 and/or VHH-2 comprises a human antibody, a humanized antibody, or a chimeric antibody.

In some embodiments, the VHH-1 and VHH-2 are linked directly or via a linker.

In some embodiments, the B7H3-binding polypeptide sequentially comprises, from the N-terminus to the C-terminus, i) VHH-1-linker-VHH-2; or ii) VHH-2-linker-VHH-1.

The term "peptide linker" or "linker" according to the present disclosure defines such an amino acid sequence by which the amino acid sequences of the VHH-1 and the VHH-2 of the B7H3-binding polypeptide of the present disclosure are linked to each other. An essential technical feature of such a peptide linker is that the peptide linker does not comprise any polymerization activity. Preferred amino acid residues for the peptide linker include Gly, Ser, and Thr, characterized by a length of 5 to 25 amino acid residues. Suitable peptide linkers include those described in U.S. Pat. Nos. 4,751,180 and 4,935,233 or WO88/09344. Preferred embodiments of the peptide linker are characterized by the amino acid sequence Gly-Gly-Gly-Gly-Ser, i.e., Gly$_4$Ser, or a polymer thereof, i.e., (Gly$_4$Ser)x, wherein x is the integer 1 or greater. The features of the peptide linker, including lack of promoting secondary structure, are known in the art and are described, for example, in Dall'Acqua et al. (Biochem. (1998) 37, 9266-9273), Cheadle et al. (MolImmunol (1992) 29, 21-30), and Raag and Whitlow (FASEB (1995) 9(1), 73-80). Peptide linkers that do not promote any secondary structure are preferred. As described in the examples, the linking of the domains to each other is provided, e.g., by genetic engineering. Methods for preparing, fusing, and operably linking bispecific single-chain constructs and expressing them in mammalian cells or bacteria are well known in the art (e.g., WO99/54440 or Sambrook et al., Molecular Cloning: A Laboratory Manual, Cold Spring Harbor Laboratory Press, Cold Spring Harbor, New York, 2001).

In some embodiments, the linker comprises the amino acid sequence set forth in SEQ ID NO: 22.

In some embodiments, the B7H3-binding polypeptide comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 23, SEQ ID NO: 24, SEQ ID NO: 26, and SEQ ID NO: 25.

For example, the B7H3-binding polypeptide comprises the amino acid sequence set forth in any one of SEQ ID NO: 23, SEQ ID NO: 24, SEQ ID NO: 26, and SEQ ID NO: 25.

Chimeric antigen receptor

In one aspect, the present application provides a chimeric antigen receptor (CAR) targeting B7H3, which comprises an extracellular antigen-binding domain (targeting portion), wherein the extracellular antigen-binding domain comprises at least two VHHs that bind to distinct epitopes of B7H3. For example, the extracellular antigen-binding domain may comprise two VHHs that bind to distinct epitopes of B7H3.

In some embodiments, the extracellular antigen-binding domain comprises the aforementioned B7H3-binding polypeptide.

For example, the extracellular antigen-binding domain comprises a first heavy chain variable region of a heavy-chain antibody (VHH-1) and a second heavy chain variable region of a heavy-chain antibody (VHH-2), wherein the VHH-1 comprises an HCDR1 set forth in SEQ ID NO: 1, an HCDR2 set forth in SEQ ID NO: 3, and an HCDR3 set forth in SEQ ID NO: 5; and/or the VHH-2 comprises an HCDR1 set forth in SEQ ID NO: 2, an HCDR2 set forth in SEQ ID NO: 4, and an HCDR3 set forth in SEQ ID NO: 6.

As another example, the extracellular antigen-binding domain comprises a VHH-1 and a VHH-2, wherein the VHH-1 may comprise the amino acid sequence set forth in SEQ ID NO: 18 or SEQ ID NO: 19, and/or the VHH-2 may comprise the amino acid sequence set forth in SEQ ID NO: 20 or SEQ ID NO: 21.

As another example, the extracellular antigen-binding domain may comprise the amino acid sequence set forth in any one of SEQ ID NO: 23, SEQ ID NO: 24, SEQ ID NO: 26, and SEQ ID NO: 25.

In some embodiments, the chimeric antigen receptor further comprises a transmembrane domain, an intracellular co-stimulatory signaling domain, and an intracellular signaling domain.

In some embodiments, the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4 (CD244), FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

In some embodiments, the transmembrane domain comprises a transmembrane domain derived from CD8A.

In some embodiments, the transmembrane domain comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 62 to SEQ ID NO: 110.

In some embodiments, the transmembrane domain comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 62.

For example, the transmembrane domain may comprise the amino acid sequence set forth in SEQ ID NO: 62.

In some embodiments, the intracellular co-stimulatory signaling domain comprises an intracellular co-stimulatory signaling domain derived from one or more proteins selected from the group consisting of: CD28, CD137, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

In some embodiments, the intracellular co-stimulatory signaling domain is derived from a 4-1BB co-stimulatory signaling domain.

In some embodiments, the intracellular co-stimulatory signaling domain comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 111 to SEQ ID NO: 143.

In some embodiments, the intracellular co-stimulatory signaling domain comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 112.

For example, the intracellular co-stimulatory signaling domain may comprise the amino acid sequence set forth in SEQ ID NO: 112.

In some embodiments, the intracellular signaling domain comprises an intracellular signaling domain derived from one or more proteins selected from the group consisting of: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, DAP10, DAP-12, and a domain comprising at least one ITAM.

In some embodiments, the intracellular signaling domain comprises a signaling domain derived from CD3ζ.

In some embodiments, the intracellular signaling domain comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 127, SEQ ID NO: 131, SEQ ID NO: 132, and SEQ ID NO: 144 to SEQ ID NO: 154.

In some embodiments, the intracellular signaling domain comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 144.

For example, the intracellular signaling domain may comprise the amino acid sequence set forth in SEQ ID NO: 144.

In some embodiments, the chimeric antigen receptor comprises a hinge region between the targeting portion and the transmembrane domain, wherein the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

In some embodiments, the hinge region comprises a hinge region derived from CD8A.

In some embodiments, the hinge region comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 155 to SEQ ID NO: 176.

In some embodiments, the hinge region comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 155.

For example, the hinge region may comprise the amino acid sequence set forth in SEQ ID NO: 163.

In some embodiments, a non-targeting portion of the chimeric antigen receptor comprises a hinge region, a transmembrane domain, an intracellular co-stimulatory signaling domain, and an intracellular signaling domain. For example, the non-targeting portion of the chimeric antigen receptor may comprise a CD8A transmembrane domain, a CD8A hinge region, a 4-1BB intracellular co-stimulatory signaling domain, and a CD3ζ intracellular signaling domain.

For example, the chimeric antigen receptor uses the B7H3-binding polypeptide of the present application as an extracellular antigen-binding domain linked to an intracellular signaling domain via a hinge region and a transmembrane domain of the CD8A molecule, the intracellular signaling domain consisting of a 4-1BB intracellular co-stimulatory signaling domain and a CD3ζ intracellular signaling domain.

In some embodiments, the non-targeting portion of the chimeric antigen receptor comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 27. For example, the non-targeting portion of the chimeric antigen receptor may comprise the amino acid sequence set forth in SEQ ID NO: 27.

In some embodiments, the chimeric antigen receptor further comprises a signal peptide fragment, wherein the C-terminus of the signal peptide fragment is linked to the N-terminus of the targeting portion. For example, the chimeric antigen receptor may include a CAR comprising a signal peptide, an anti-B7H3 VHH, a CD8A hinge domain, a CD8A transmembrane domain, a 4-1BB co-stimulatory domain, and a CD3ζ primary signaling domain.

In some embodiments, the signal peptide fragment comprises a CD8A signal peptide fragment.

In some embodiments, the signal peptide fragment of the chimeric antigen receptor comprises an amino acid sequence having at least about 90%, about 91%, about 92%, about 93%, about 94%, about 95%, about 96%, about 97%, about 98%, about 99%, or about 99.5% identity to the amino acid sequence set forth in SEQ ID NO: 28. For example, the signal peptide fragment may comprise the amino acid sequence set forth in SEQ ID NO: 28.

In some embodiments, the chimeric antigen receptor sequentially comprises a signal peptide, an extracellular antigen-binding domain, a hinge region, a transmembrane domain, an intracellular co-stimulatory signaling domain, and an intracellular signaling domain, wherein the extracellular antigen-binding domain is the B7H3-binding polypeptide described herein.

For example, the chimeric antigen receptor may comprise a CD8A signal peptide, the B7H3-binding polypeptide of the present application, a CD8A transmembrane domain, a CD8A hinge region, a 4-1BB intracellular co-stimulatory signaling domain, and a CD3ζ intracellular signaling domain.

In some embodiments, the chimeric antigen receptor comprises an amino acid sequence having at least about 90%, about 91%, about 92%, about 93%, about 94%, about 95%, about 96%, about 97%, about 98%, about 99%, or about 99.5% identity to the amino acid sequence set forth in any one of SEQ ID NO: 33 to SEQ ID NO: 36.

For example, the chimeric antigen receptor may comprise the amino acid sequence set forth in any one of SEQ ID NO: 33 to SEQ ID NO: 36.

Isolated polypeptide

In another aspect, the present application provides one or more isolated polypeptides comprising the chimeric antigen receptor described herein and an enhancer capable of enhancing one or more activities of an immune effector cell. In some embodiments, the enhancer comprises a cytokine and/or a cytokine receptor.

In some embodiments, the enhancer is a membrane-bound cytokine and/or cytokine receptor.

In some embodiments, the enhancer is selected from the group consisting of: IL-2, IL-3, IL-4, IL-6, IL-7, IL-8, IL-10, IL-11, IL-12, IL-15, IL-17, IL-18, IL-21, IL-23, receptors thereof, functional variants thereof, and combinations thereof.

In some embodiments, the enhancer comprises IL-15 or a functional variant thereof, or a chimeric cytokine receptor comprising an IL-15 receptor (IL-15R).

In some embodiments, the enhancer comprises membrane-bound IL-15 (mIL-15).

For example, the polypeptide may comprise the chimeric antigen receptor, wherein the chimeric antigen receptor comprises an antigen-binding domain and a non-antigen-binding domain, wherein the non-antigen-binding domain comprises an extracellular domain, a transmembrane domain, and a CD28 intracellular co-stimulatory domain or a functional variant thereof, and a CD3ζ signaling domain or a functional variant thereof, as well as membrane-bound IL-15.

For example, the polypeptide may comprise the chimeric antigen receptor, wherein the chimeric antigen receptor comprises a B7H3-binding domain and a non-antigen-binding domain, wherein the non-antigen-binding domain comprises an extracellular domain, a transmembrane domain, and a CD28 intracellular co-stimulatory domain or a functional variant thereof, and a CD3ζ signaling domain or a functional variant thereof, as well as membrane-bound IL-15.

In some embodiments, the enhancer comprises the amino acid sequence set forth in SEQ ID NO: 46 or an amino acid sequence having at least about 50%, about 60%, about 70%, about 80%, about 90%, about 91%, about 92%, about 93%, about 94%, about 95%, about 96%, about 97%, about 98%, about 99%, or about 99.5% identity to the amino acid sequence set forth in SEQ ID NO: 46.

For example, the polypeptide may comprise the chimeric antigen receptor, wherein the chimeric antigen receptor may comprise the amino acid sequence set forth in SEQ ID NO: 54; and an enhancer, wherein the enhancer may comprise the amino acid sequence set forth in SEQ ID NO: 54.

In some embodiments, the chimeric antigen receptor and the enhancer are located in separate polypeptides.

In some embodiments, the chimeric antigen receptor and the enhancer are located in the same polypeptide. For example, the chimeric antigen receptor and the enhancer may be linked directly or indirectly.

In some embodiments, the chimeric antigen receptor and the enhancer are linked via a linker. For example, the polypeptide may comprise the following structures, from the N-terminus to the C-terminus, i) the chimeric antigen receptor, the linker, and the enhancer; or ii) the enhancer, the linker, and the chimeric antigen receptor.

In some embodiments, the linker comprises a cleavable linker.

In some embodiments, the linker comprises a self-cleaving peptide.

In some embodiments, the self-cleaving peptide comprises a self-cleaving amino acid sequence of a 2A peptide, the 2A peptide being derived from porcine teschovirus-1 (P2A), equine rhinitis A virus (E2A), Thosea asigna virus (T2A), foot-and-mouth disease virus (F2A), or any combination thereof (see, e.g., Kim et al., PLOS One 6: e18556, 2011, the 2A nucleic acids and amino acid sequences being incorporated herein by reference in their entirety).

For example, the polypeptide may comprise the chimeric antigen receptor, wherein the chimeric antigen receptor comprises an antigen-binding domain and a non-antigen-binding domain, wherein the non-antigen-binding domain comprises a CD8A hinge region, a CD8A transmembrane domain, a 4-1BB intracellular co-stimulatory signaling domain, and a CD3ζ intracellular signaling domain, as well as IL-15 or a functional variant thereof; the chimeric antigen receptor and the mIL-15 may be linked via T2A.

In some embodiments, the linker comprises the amino acid sequence set forth in SEQ ID NO: 44 or an amino acid sequence having at least about 50%, about 60%, about 70%, about 80%, about 90%, about 91%, about 92%, about 93%, about 94%, about 95%, about 96%, about 97%, about 98%, about 99%, or about 99.5% identity to the amino acid sequence set forth in SEQ ID NO: 44.

For example, the polypeptide may comprise the chimeric antigen receptor, wherein the chimeric antigen receptor may comprise the amino acid sequence set forth in any one of SEQ ID NOs: 33-36; and an enhancer, wherein the enhancer may comprise the amino acid sequence set forth in SEQ ID NO: 46 or 54; the chimeric antigen receptor and the enhancer may be linked via a self-cleaving peptide, and the linker may comprise the amino acid sequence set forth in SEQ ID NO: 44.

As another example, the polypeptide comprises the amino acid sequence set forth in SEQ ID NO: 241 or 243 or an amino acid sequence having at least about 50%, about 60%, about 70%, about 80%, about 90%, about 91%, about 92%, about 93%, about 94%, about 95%, about 96%, about 97%, about 98%, about 99%, or about 99.5% identity to the amino acid sequence set forth in SEQ ID NO: 241 or 243.

## Nucleic acid molecule, vector, and cell

In another aspect, the present application provides one or more isolated nucleic acid molecules encoding the aforementioned B7H3-binding polypeptide, the aforementioned chimeric antigen receptor, or the aforementioned polypeptide.

In some embodiments, the isolated nucleic acid molecule comprises an amino acid sequence having at least about 90%, about 91%, about 92%, about 93%, about 94%, about 95%, about 96%, about 97%, about 98%, about 99%, or about 99.5% identity to the nucleotide sequence set forth in SEQ ID NO: 240 or SEQ ID NO: 242.

In another aspect, the present application provides a vector comprising the aforementioned isolated nucleic acid molecule.

In some embodiments, the vector is an expression vector.

In some embodiments, the vector is selected from the group consisting of: a DNA vector, an RNA vector, a plasmid, a lentiviral vector, an adenoviral vector, an adeno-associated viral vector, and a retroviral vector. For example, the vector may be a lentiviral vector.

In another aspect, the present application provides a cell i) comprising the aforementioned isolated nucleic acid molecule or the aforementioned vector; and/or ii) expressing the aforementioned B7H3-binding polypeptide or chimeric antigen receptor.

## Immune effector cell

In another aspect, the present application provides an immune effector cell comprising the aforementioned nucleic acid molecule or the aforementioned vector, and/or expressing the aforementioned CAR.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell. For example, the immune effector cell may be a T cell. As another example, the immune effector cell may be a human T cell.

In some embodiments, the immune effector cell comprises an autologous or non-autologous immune effector cell.

In some embodiments, the immune effector cell comprises a modified immune effector cell.

In some embodiments, the modified immune effector cell comprises a cell that reduces immune rejection caused by allogeneic cell therapy.

In some embodiments, the functions of T cell antigen receptor (TCR) and major histocompatibility complex (MHCI, MHCII) in the modified immune effector cell are suppressed in a T cell.

In some embodiments, the modification comprises down-regulating the expression and/or activity of one or more of immune rejection-associated genes.

In some embodiments, the immune rejection-associated gene is selected from one or more of the following genes: TRAC, TRBC, HLA-A, HLA-B, B2M, and CIITA.

In some embodiments, the immune rejection-associated gene is selected from one or more of the following genes: TRAC, TRBC, HLA-A, and HLA-B.

In some embodiments, the immune rejection-associated gene is selected from one or more of the following genes: TRAC, TRBC, and HLA-A.

In some embodiments, the immune rejection-associated gene is selected from one or more of the following genes: TRAC and HLA-A.

In some embodiments, the expression and/or activity of the TRAC gene and the HLA-A gene in the modified immune effector cell is down-regulated as compared to a corresponding unmodified cell.

In some embodiments, the expression and/or activity of the TRAC gene, the HLA-A gene, and the HLA-B gene in the modified immune effector cell is down-regulated as compared to the corresponding unmodified cell.

In some embodiments, the expression and/or activity of the CIITA gene in the modified immune effector cell is not down-regulated as compared to the corresponding unmodified cell.

In some embodiments, the expression and/or activity of the B2M gene in the modified immune effector cell is not down-regulated as compared to the corresponding unmodified cell.

In some embodiments, the expression and/or activity of the TRAC gene and the HLA-A gene in the modified immune effector cell is down-regulated as compared to a corresponding wild-type cell.

In some embodiments, the expression and/or activity of the TRAC gene, the HLA-A gene, and the HLA-B gene in the modified immune effector cell is down-regulated as compared to the corresponding wild-type cell.

In some embodiments, the expression and/or activity of the B2M gene in the modified immune effector cell is not down-regulated as compared to the corresponding wild-type cell.

In some embodiments, the expression and/or activity of the CIITA gene in the modified immune effector cell is not down-regulated as compared to the corresponding wild-type cell.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene knockout, gene mutation, and/or gene silencing.

In some embodiments, the modification comprises knocking out either of two TRAC alleles and knocking out either of two HLA-A alleles in the immune effector cell.

In some embodiments, the modification comprises knocking out both TRAC alleles and knocking out either of both HLA-A alleles in the immune cell.

In some embodiments, the modification comprises knocking out an exon of the TRAC gene and knocking out an exon of the HLA-A gene in the immune cell.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification further comprises administering to the immune effector cell sgRNA targeting an exonic region of the TRAC gene.

In some embodiments, the sgRNA targeting the exonic region of the TRAC gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 177 to SEQ ID NO: 191.

In some embodiments, the modification comprises administering to the immune effector cell sgRNA targeting an exonic region of the HLA-A gene.

In some embodiments, the sgRNA targeting the exonic region of the HLA-A gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 237 to SEQ ID NO: 239.

In some embodiments, the modification further comprises administering to the cell a Cas enzyme.

In some embodiments, the Cas enzyme comprises a Cas9 protein.

In some embodiments, the antisense RNA comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 233 to SEQ ID NO: 236.

In some embodiments, the immune effector cell is an HLA-B homozygous or heterozygous cell.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the immune effector cell is an HLA-A homozygous or heterozygous cell.

In some embodiments, the HLA-A homozygote or heterozygote comprises HLA-A*02 homozygote, HLA-A*11 homozygote, HLA-A*02/A*11 heterozygote, or HLA-A*24 homozygote.

In another aspect, the present application provides a method for preparing an immune effector cell, which comprises introducing the aforementioned nucleic acid molecule or the aforementioned vector into an immune effector cell.

In some embodiments, the method further comprises: modifying an immune effector cell before/after introducing the aforementioned nucleic acid molecule or the aforementioned vector into the immune effector cell, wherein the modification comprises down-regulating the expression and/or activity of one or more of immune rejection-associated genes.

In some embodiments, the method comprises: modifying an immune effector cell after introducing the aforementioned nucleic acid molecule or the aforementioned vector into the immune effector cell, wherein the modification comprises down-regulating the expression and/or activity of one or more of immune rejection-associated genes.

For example, the method for preparing an immune effector cell may comprise:

(1) introducing the aforementioned nucleic acid molecule or the aforementioned vector into an immune effector cell; and

(2) modifying the immune effector cell, wherein the modification comprises down-regulating the expression and/or activity of one or more of immune rejection-associated genes.

In some embodiments, the immune rejection-associated gene is selected from one or more of the following genes: TRAC, TRBC, HLA-A, HLA-B, B2M, and CIITA.

In some embodiments, the expression and/or activity of the TRAC gene and the HLA-A gene in the immune effector cell is down-regulated as compared to the expression and/or activity of the corresponding gene in a corresponding unmodified cell.

In some embodiments, the expression and/or activity of the CIITA gene is not down-regulated as compared to the expression and/or activity of the corresponding gene in the corresponding unmodified cell.

In some embodiments, the expression and/or activity of the B2M gene is not down-regulated as compared to the expression and/or activity of the corresponding gene in the corresponding unmodified cell.

In some embodiments, the expression and/or activity of the TRAC gene and the HLA-A gene in the immune effector cell is down-regulated as compared to a corresponding wild-type cell.

In some embodiments, the expression and/or activity of the CIITA gene is not down-regulated as compared to the corresponding wild-type cell.

In some embodiments, the expression and/or activity of the B2M gene is not down-regulated as compared to the corresponding wild-type cell.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene knockout, gene mutation, and/or gene silencing.

In some embodiments, the modification comprises knocking out either of two TRAC alleles and knocking out either of two HLA-A alleles in the immune effector cell.

In some embodiments, the modification comprises knocking out both TRAC alleles and knocking out either of both HLA-A alleles in the immune cell.

In some embodiments, the modification comprises knocking out an exon of the TRAC gene and knocking out an exon of the HLA-A gene in the immune cell.

In some embodiments, the modification comprises knocking out an exon of the TRAC gene, knocking out an exon of the HLA-A gene, and knocking out an exon of the HLA-B gene in the immune cell.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification comprises administering to the immune effector cell sgRNA targeting an exonic region of the TRAC gene.

In some embodiments, the sgRNA targeting the exonic region of the TRAC gene comprises the nucleotide sequence set forth in any one of SEQ ID NO: 177 to SEQ ID NO: 191.

In some embodiments, the modification comprises administering to the immune effector cell sgRNA targeting an exonic region of the HLA-A gene.

In some embodiments, the sgRNA targeting the exonic region of the HLA-A gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 237 to SEQ ID NO: 239.

In some embodiments, the modification further comprises administering to the cell a Cas enzyme.

In some embodiments, the Cas enzyme comprises a Cas9 protein.

In some embodiments, the antisense RNA comprises an amino acid sequence having at least about 90%, about 91%, about 92%, about 93%, about 94%, about 95%, about 96%, about 97%, about 98%, about 99%, or about 99.5% identity to the nucleotide sequence set forth in any one of SEQ ID NO: 233 to SEQ ID NO: 236.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell. For example, the immune effector cell may be a T cell.

In some embodiments, the immune effector cell comprises an autologous or non-autologous immune effector cell.

In some embodiments, the cell is an HLA-B homozygous or heterozygous cell.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the cell is an HLA-A homozygous or heterozygous cell.

In some embodiments, the HLA-A homozygote or heterozygote comprises HLA-A*02 homozygote, HLA-A* 11 homozygote, HLA-A*02/A*11 heterozygote, or HLA-A*24 homozygote.

For example, the method for preparing an immune effector cell may comprise:

(1) collecting peripheral blood of healthy people, performing an HLA typing assay, selecting typing meeting our requirements, isolating PBMCs, adding CD3 magnetic beads according to a proportion for incubation, and sorting CD3+ T cells; uniformly mixing CD3/CD28 antibody-coupled magnetic beads, taking an appropriate amount of magnetic bead suspension according to the calculated amount, adding the magnetic bead suspension to a T cell culture system, activating T cells, and performing overnight culture;

(2) infecting the T cells according to the titer of B7H3 CAR virus;

(3) simultaneously knocking out TRAC and HLA-A genes; and

(4) sorting CD3-negative T cells: adding CD3 magnetic beads according to a proportion, and collecting CD3-T cells (cells not bound to the magnetic beads).

## Use, pharmaceutical composition, and treatment method

In another aspect, the present application provides use of the aforementioned chimeric antigen receptor, the aforementioned isolated nucleic acid molecule, the aforementioned vector, the aforementioned cell, or the aforementioned immune effector cell in the preparation of a CAR-T cell.

In another aspect, the present application provides a pharmaceutical composition comprising the aforementioned B7H3-binding polypeptide, the aforementioned chimeric antigen receptor, the aforementioned isolated nucleic acid molecule, the aforementioned vector, the aforementioned cell, and/or the aforementioned immune effector cell, and optionally a pharmaceutically acceptable carrier.

For example, the pharmaceutical composition may include: buffers, such as neutral buffered saline, phosphate buffered saline, and the like; sugars, such as glucose, mannose, sucrose, dextran, or mannitol; proteins; polypeptides or amino acids such as glycine; antioxidants; chelating agents, such as EDTA or glutathione; adjuvants, such as aluminum hydroxide; and preservatives.

For example, the pharmaceutical composition comprises the aforementioned immune effector cell and optionally a pharmaceutically acceptable carrier.

In another aspect, the present application provides use of the aforementioned B7H3-binding polypeptide, the aforementioned antigen chimeric receptor, the aforementioned isolated nucleic acid molecule, the aforementioned vector, the aforementioned cell, the aforementioned immune effector cell, and/or the aforementioned pharmaceutical composition in the treatment of a disease or disorder associated with the expression of B7H3.

In some embodiments, the disease or disorder associated with the expression of B7H3 comprises a disease or disorder associated with up-regulation of the expression of B7H3.

In some embodiments, the disease or disorder associated with the expression of B7H3 comprises cancer.

In some embodiments, the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

In another aspect, the present application provides use of the aforementioned B7H3-binding polypeptide, the aforementioned antigen chimeric receptor, the aforementioned isolated nucleic acid molecule, the aforementioned vector, the aforementioned cell, the aforementioned immune effector cell, and/or the aforementioned pharmaceutical composition in the preparation of a medicament for treating a disease or disorder associated with the expression of B7H3.

In some embodiments, the disease or disorder associated with the expression of B7H3 comprises a disease or disorder associated with up-regulation of the expression of B7H3.

In some embodiments, the disease or disorder associated with the expression of B7H3 comprises cancer.

In some embodiments, the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

In another aspect, the present application provides a method for preventing or treating a disease or disorder associated with the expression of B7H3, which comprises administering to a subject in need thereof an effective amount of the aforementioned B7H3-binding polypeptide, the aforementioned antigen chimeric receptor, the aforementioned isolated nucleic acid molecule, the aforementioned vector, the aforementioned cell, the aforementioned immune effector cell, and/or the aforementioned pharmaceutical composition.

In some embodiments, the disease or disorder associated with the expression of B7H3 comprises a disease or disorder associated with up-regulation of the expression of B7H3.

In some embodiments, the disease or disorder associated with the expression of B7H3 comprises cancer.

In some embodiments, the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

**Modified immune effector cell**

In another aspect, the present application provides a modified immune effector cell, wherein the expression and/or activity of TRAC gene and HLA-A gene is down-regulated, the expression and/or activity of B2M gene is not down-regulated, and the expression and/or activity of CIITA gene is not down-regulated as compared to the expression and/or activity of corresponding genes in a corresponding unmodified cell; and the HLA-B typing of the modified immune effector cell is matched with the HLA-B typing of a subject.

In some embodiments, the modified immune effector cell is HLA-B heterozygote and is consistent with two alleles of HLA-B of the subject, or the modified immune effector cell is HLA-B homozygote and is consistent with one of the alleles of HLA-B of the subject.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the modification enables the expression and/or activity of two genes to be down-regulated, wherein the two genes consist of TRAC gene and HLA-A gene.

In some embodiments, the expression and/or activity of the TRAC gene and the HLA-A gene is down-regulated, the expression and/or activity of the B2M gene is not down-regulated, and the expression and/or activity of the CIITA gene is not down-regulated as compared to a corresponding wild-type cell.

In some embodiments, the expression and/or activity of two genes is down-regulated as compared to the corresponding wild-type cell, wherein the two genes consist of TRAC gene and HLA-A gene.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene mutation and/or gene silencing.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification comprises administering to the immune effector cell sgRNA targeting an exonic region of the HLA-A gene.

In some embodiments, the sgRNA targeting the exonic region of the HLA-A gene comprises the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises the nucleotide sequence set forth in any one of SEQ ID NO: 237 to SEQ ID NO: 239.

In some embodiments, the modification further comprises administering to the immune effector cell sgRNA targeting an exonic region of the TRAC gene.

In some embodiments, the sgRNA targeting the exonic region of the TRAC gene comprises the nucleotide sequence set forth in any one of SEQ ID NO: 177 to SEQ ID NO: 191.

In some embodiments, the modification further comprises administering to the cell a Cas enzyme.

In some embodiments, the Cas enzyme comprises a Cas9 protein.

In some embodiments, the antisense RNA comprises the nucleotide sequence set forth in any one of SEQ ID NO: 233 to SEQ ID NO: 236.

In some embodiments, the modified immune effector cell expresses a CAR.

In some embodiments, the CAR comprises an antigen-binding domain, a hinge region, a transmembrane domain, an intracellular co-stimulatory signaling domain, and an intracellular signaling domain.

In some embodiments, the antigen-binding domain specifically binds to a tumor antigen.

In some embodiments, the tumor antigen is selected from the group consisting of: CD19, CD20, CD22, CD33, BCMA, IL13Ra2, EGFR, Her2, GD2, and B7H3.

In some embodiments, the antigen-binding domain is selected from the group consisting of: a monoclonal antibody, a polyclonal antibody, a dimer, a polymer, a multispecific antibody, an intact antibody, an antibody fragment, a human antibody, a humanized antibody, a chimeric antibody, an Fv fragment, F(ab')2, a single-chain Fv(scFv), and a single-domain antibody (VHH).

In some embodiments, the transmembrane domain comprises a transmembrane domain derived from one or more proteins selected from the group consisting of: CD8A, CD8B, CD28, CD3$\epsilon$ (CD3e), 4-1BB, CD4, CD27, CD7, PD-1, TRAC, TRBC, CD3$\zeta$, CTLA-4, LAG-3, CD5, ICOS, OX40, NKG2D, 2B4 (CD244), Fc$\epsilon$RI$\gamma$, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

In some embodiments, the intracellular co-stimulatory signaling domain comprises an intracellular co-stimulatory signaling domain derived from one or more proteins selected from the group consisting of: CD28, CD137, CD27, CD2, CD7, CD8A, CD8B, OX40, CD226, DR3, SLAM, CDS, ICAM-1, NKG2D, NKG2C, B7-H3, 2B4, Fc$\epsilon$RI$\gamma$, BTLA, GITR, HVEM, DAP10, DAP12, CD30, CD40, CD40L, TIM1, PD-1, LFA-1, LIGHT, JAML, CD244, CD100, ICOS, CD40, and MyD88.

In some embodiments, the intracellular signaling domain comprises an intracellular signaling domain derived from one or more proteins selected from the group consisting of: CD3$\zeta$, CD3$\delta$, CD3$\gamma$, CD3$\epsilon$, CD79a, CD79b, FceRI$\gamma$, FceRI$\beta$, Fc$\gamma$RIIa, bovine leukemia virus gp30, Epstein-Barr virus (EBV) LMP2A, simian immunodeficiency virus PBj14 Nef, DAP10, DAP-12, and a domain comprising at least one ITAM.

In some embodiments, the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, Fc$\epsilon$RI$\gamma$, BTLA, GITR, DAP10, TIM1, SLAM, CD30, and LIGHT.

In some embodiments, the CAR further comprises a signal peptide fragment, wherein the C-terminus of the signal peptide fragment is linked to the N-terminus of the targeting portion.

In some embodiments, the signal peptide fragment comprises a CD8A signal peptide fragment.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In some embodiments, the immune effector cell comprises a non-autologous immune effector cell.

In another aspect, the present application provides a method for preparing the aforementioned modified immune effector cell, comprising the following steps:

1) selecting an immune effector cell that matches HLA-B typing of a subject; and

2) down-regulating the expression and/or activity of TRAC gene and HLA-A gene in the immune effector cell, not down-regulating the expression and/or activity of B2M gene, and not down-regulating the expression and/or activity of CIITA gene as compared to the expression and/or activity of corresponding genes in a corresponding unmodified cell.

In some embodiments, the modified immune effector cell is HLA-B heterozygote and is consistent with two alleles of HLA-B of the subject, or the modified immune effector cell is HLA-B homozygote and is consistent with one of the alleles of HLA-B of the subject.

In some embodiments, the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

In some embodiments, the modification enables the expression and/or activity of two genes to be down-regulated, wherein the two genes consist of TRAC gene and HLA-A gene.

In some embodiments, the modification enables the expression and/or activity of three genes to be down-regulated, wherein the two genes consist of TRAC gene, HLA-A gene, and HLA-B gene.

In some embodiments, the expression and/or activity of the TRAC gene and the HLA-A gene is down-regulated, the expression and/or activity of the B2M gene is not down-regulated, and the expression and/or activity of the CIITA gene is not down-regulated as compared to a corresponding wild-type cell.

In some embodiments, the expression and/or activity of two genes is down-regulated as compared to the corresponding wild-type cell, wherein the two genes consist of TRAC gene and HLA-A gene.

In some embodiments, down-regulating the expression level and/or activity of the gene comprises down-regulating the expression and/or activity of a nucleic acid molecule encoding the gene; and/or down-regulating the expression and/or activity of a protein product encoded by the gene.

In some embodiments, the modification comprises: gene mutation and/or gene silencing.

In some embodiments, the modification comprises administering to the immune effector cell one or more substances selected from the group consisting of: antisense RNA, siRNA, shRNA, and a CRISPR/Cas9 system.

In some embodiments, the modification comprises administering to the immune effector cell the CRISPR/Cas9 system.

In some embodiments, the modification comprises administering to the immune effector cell sgRNA targeting an exonic region of the HLA-A gene.

In some embodiments, the sgRNA targeting the exonic region of the HLA-A gene comprises the nucleotide sequence set forth in any one of SEQ ID NO: 192 to SEQ ID NO: 232.

In some embodiments, the modification comprises administering to the immune effector cell sgRNA targeting an exonic region of the HLA-B gene.

In some embodiments, the sgRNA targeting the exonic region of the HLA-B gene comprises the nucleotide sequence set forth in any one of SEQ ID NO: 237 to SEQ ID NO: 239.

In some embodiments, the modification comprises administering to the immune effector cell sgRNA targeting an exonic region of the TRAC gene.

In some embodiments, the sgRNA targeting the exonic region of the TRAC gene comprises the nucleotide sequence set forth in any one of SEQ ID NO: 177 to SEQ ID NO: 191.

In some embodiments, the modification further comprises administering to the cell a Cas enzyme.

In some embodiments, the Cas enzyme comprises a Cas9 protein.

In some embodiments, the antisense RNA comprises the nucleotide sequence set forth in any one of SEQ ID NO: 233 to SEQ ID NO: 236.

In some embodiments, the immune effector cell comprises a human cell.

In some embodiments, the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

In some embodiments, the immune effector cell comprises a non-autologous immune effector cell.

In another aspect, the present application provides a composition comprising the aforementioned modified immune effector cell and a pharmaceutically acceptable carrier.

In another aspect, the present application provides use of the aforementioned modified immune effector cell in the preparation of a CAR-T cell.

In another aspect, the present application provides use of the aforementioned modified immune effector cell in the preparation of a medicament for allogeneic therapy.

In another aspect, the present application provides use of the aforementioned modified immune effector cell in the preparation of a medicament for treating a tumor.

In some embodiments, the tumor comprises a solid tumor and a non-solid tumor.

In some embodiments, the tumor is selected from the group consisting of: liver cancer, gastric cancer, lung cancer, breast cancer, non-small cell lung cancer, B-lymphomas, Hodgkin's lymphoma, gliomas, chronic myelogenous leukemia, and acute myeloid leukemia.

Without being bound by any theory, the following examples are intended only to illustrate the B7H3-binding polypeptide, preparation method, use, etc., of the present application, and are not intended to limit the scope of the present application.

Examples

Example 1. Design of Anti-B7H3 Chimeric Antigen Receptor (CAR)

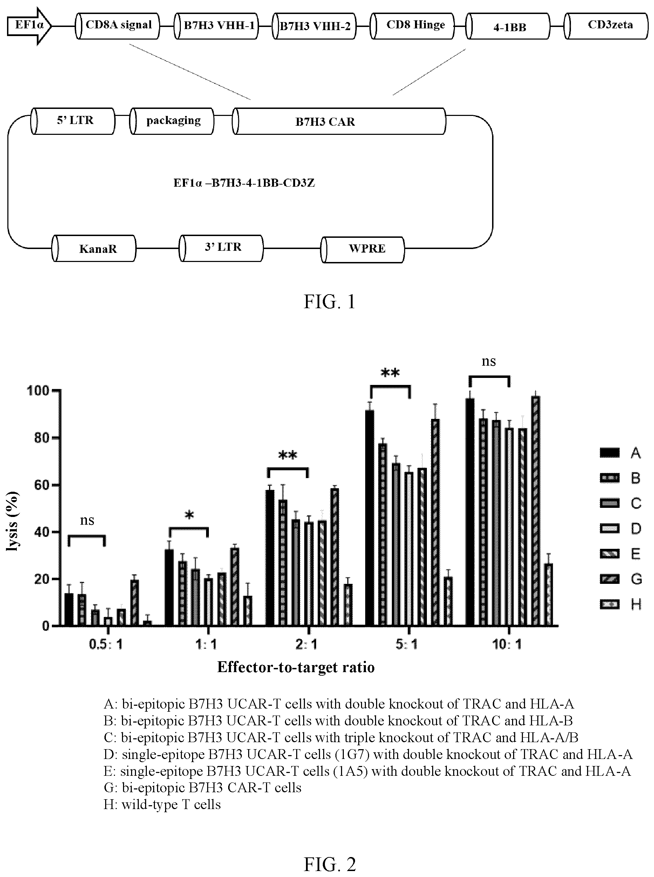

As shown in FIG. 1, the anti-B7H3 CAR structure comprises two B7H3 antigen-binding regions (1G7 and 1A5), a CD8A extracellular hinge region, a CD8A transmembrane region, a 4-1BB intracellular co-stimulatory domain, and a CD3ζ activation signal domain.

The sequence of B7H3 CAR is as follows:

Variable region sequences of B7H3 antibodies

1G7:

GCTGTCCAGCTTGTGGAGTCTGGGGGGGGCCTCGTCCAGACAGGCGG

TTCTCTCCGCCTCTCCTGCTGATTCTGGCAGAATCTTTTCAAACTAC

GCAATGGGCTGGTTCAGGCAGGCACCAGGCAAGGAGCGGGAGTTCGT

GGCTGCTATCATTGGATCTACTGGCACCGTTAACTACGCAGACAGCATG

AAAGGCAGATTTACCATCTCAAGAGATAACGCCAAAAATACTGTAGTC

CTCCAGATGAACTCTCTGAAGCCTGAGGATACCGCTATATATTATTGTGC

GGCGTCATTCAGGTACAGCGGCATCTATGGCCGCGAAGCGGATTTTGTT

AGTTGGGGACAAGGGACCCAAGTGACAGTGTCTTCA

hu1G7:

GCTGTTCAGCTGGTGGAGTCTGGTGGCGGGCTCGTTCAGCCGGGCGGC

AGCCTCAGACTGAGCTGTGCAGCCTCCGGAAGGATCTTTTCAAATTAC

GCAATGGGGTGGTTTAGGCAGGCACCAGGAAAGGAGCGAGAGTTTGT

GGCAGCAATAATCGGTTCTACCGGCACAGTGTATTACGCTGATAGCGTG

AAAGGCAGGTTCACCATTTCAAGGGATAACAGTAAAAACACACTATAC

TTACAGATGAACTCCTTACGAGCCGAAGACACGGCGGTGTACTACTGT

GCCGCATCCTTTAGATACTCTGGAATTTACGGACGCGAGGCCGACTTTG

TCAGCTGGGGTCAGGGGACTCTTGTTACCGTATCCTCT

1A5:

CAGGCTCAACTGGTGGAGTCGGGAGGTGGCTTGGTGCAGACAGGAGG

GAGCCTGCGACTCTCCTGTGCGGCTTCCGGAAGGATTTTCTCTAATTAT

TCTGTCGGATGGTTCCGGCAGGCCCCAGGCAAAGAGCGTGAATTCGTT

GCGGCGATAATCTGGTCGACCGGAACAACGAATCTGGCCGATCCCATG

AAGGGGAGATTCACCATTAGTAGAGACAACGCAAAAAACACAGTAGT

CCTGCAGATGAACAGCCTCAAACCCGAGGATACAGCTATCTATTACTGC

GCAGCTTCCTTTAAATATTCTGGCATATATGGCAGAGAAGCAGATTTTG

GGAGTTGGGGGCAGGGCACCCAGGTGACAGTCAGTTCT

hu1A5:

CAGGTCCAATTAGTGGAAAGTGGTGGGGGATTAGTGCAACCGGGCGG
CTCCCTCCGCCTTTCCTGCGCCGCTTCTGGCAGAATCTTCTCCAACTAC
TCCGTTGGATGGTTTCGTCAGGCCCCAGGAAAAGAGAGAGAATTCGTG
GCTGCTATCATATGGAGCACGGGGACCACCTACTATGCCGATAGCGTCA
AAGGCAGGTTCACTATCAGCAGGGATAATAGCAAGAATACACTATACTT
GCAAATGAACTCGCTGCGAGCCGAAGACACAGCCGTCTACTACTGTGC
GGCGTCATTTAAATACAGCGGCATTTACGGCCGAGAGGCAGATTTCGG
AAGTTGGGGGCAGGGAACCTTGGTGACAGTCTCCTCG

Non-antigen-binding sequences (DNA and amino acid sequence) of B7H3 CAR

DNA sequence:

TTCGTCCCCGTGTTCCTGCCTGCCAAGCCAACAACTACCCCTGCTCCA

CGACCACCTACTCCAGCACCTACCATCGCAAGTCAGCCCCTGTCACTG

CGACCTGAGGCTTGCCGGCCAGCAGCTGGAGGAGCAGTGCACACCCG

AGGCCTGGACTTCGCATGCGATATCTACATTTGGGCACCACTGGCTGGA

ACCTGTGGGGTCCTGCTGCTGAGCCTGGTCATCACCCTGTATTGTAACC

ACAGAAATAAAAGGGGGCGCAAGAAACTGCTGTACATCTTCAAGCAG

CCTTTTATGCGCCCAGTGCAGACAACTCAGGAGGAAGACGGATGCTCT

TGTCGGTTCCCAGAGGAGGAGGAAGGAGGCTGCGAGCTGAGAGTGAA

GTTCAGCCGGAGCGCCGATGCACCAGCATATCAGCAGGGACAGAATCA

GCTGTACAACGAGCTGAATCTGGGCAGGCGCGAGGAATATGACGTGCT

GGATAAGCGACGAGGACGGGACCCCGAAATGGGAGGAAAACCCAGA

AGGAAGAACCCTCAGGAGGGGCTGTATAATGAACTGCAGAAAGACAA

GATGGCTGAGGCATACAGCGAAATTGGAATGAAAGGAGAGCGCCGAC

GGGGGAAGGGACACGATGGGCTGTACCAGGGACTGTCAACCGCCACT

AAAGATACCTACGACGCACTGCACATGCAGGCTCTGCCCCCAAGA

Amino acid sequence:

FVPVFLPAKPTTTPAPRPPTPAPTIASQPLSLRPEACRPAAGGAVHTRGLDF
ACDIYIWAPLAGTCGVLLLSLVITLYCNHRNKRGRKKLLYIFKQPFMRPVQ
TTQEEDGCSCRFPEEEEGGCELRVKFSRSADAPAYQQGQNQLYNELNLGR
REEYDVLDKRRGRDPEMGGKPRRKNPQEGLYNELQKDKMAEAYSEIGM
KGERRRGKGHDGLYQGLSTATKDTYDALHMQALPPR

**Example 2. Construction of Anti-B7H3 CAR Lentiviral Vector**

According to the sequence information on B7H3 and the structure of the CAR vector, a B7H3 CAR lentiviral expression vector was constructed, with the vector schematic shown in FIG. 1. The optimization was performed as follows: a commercial lentiviral expression vector pCDH-CMV-MCS-EF1-copGFP was selected as a backbone, and element modification was performed on the basis of the vector. First, an ampicillin resistance gene β-lactamase on the vector was replaced with aminoglycoside phosphotransferase derived from Tn5 to enable the vector to have kanamycin resistance. Secondly, we deleted the CMV promoter and its adjacent downstream multiple cloning site, which were potentially threatening *in vivo* applications. Finally, the copGFP gene that was started to express by the EF1 promoter in the original vector was deleted, a SalI enzyme digestion site was retained, and a SmaI enzyme digestion site was added to the 5' end of SalI for vector construction to form a final target vector. The added SmaI enzyme digestion site was a single enzyme digestion site of the final target vector, and other sequence portions of the vector did not have the enzyme digestion site. After the optimization, the chimeric antigen receptor lentiviral expression vector was constructed, and lentiviral packaging was performed after confirming that the sequence was correct by Sanger sequencing.

**Example 3. Preparation of B7H3-Targeted UCAR-T Cells**

The present application provides a B7H3 UCAR-T cell with higher safety, which was mainly prepared on the basis of the following two strategies:
The first one is a donor source screening strategy. First, the donor source was based on HLA-A and/or HLA-B homozygotes in the population, and one of the alleles of HLA-A or HLA-B in the patient was consistent with the HLA-A or HLA-B homozygotes in the donor, so that cells from these donors could cover a high number of patient populations, and the rejection response caused by inconsistencies in HLA-A and/or HLA-B subtypes was reduced. For HLA-A, A*02 homozygote, A* 11 homozygote, A*02/A11 heterozygote, and A*24 homozygote, which had relatively high frequency in the population, were selected. For HLA-B, B*40 homozygote, B*15 homozygote, B*46 homozygote, B*13 homozygote, B*51 homozygote, B*58 homozygote, B*07 homozygote, B*35 homozygote, B*44 homozygote, B*52 homozygote, B*57 homozygote, B*54 homozygote, and B*55 homozygote, which had relatively high frequency in the population, were mainly selected.

The second one is a knockout strategy. The TCR molecules, as well as HLA-A and/or HLA-B molecules highly related to rejection, were selected for knockout, and other HLA-I molecules were retained, so that the rejection of allogeneic cells was reduced, and the elimination of HLA molecules by NK cells due to complete knockout were avoided, thereby greatly prolonging the half-life of allogeneic CAR-T cells *in vivo*. Meanwhile, the knockout of TCR could reduce the GVHD response caused by allogeneic cell therapy. For the TCR genes, the TRAC gene encoding the TCRα chain was selected; for HLA-A, A*02 homozygote, A*11 homozygote, and A*02/A11 heterozygote, which had relatively high frequency in the population, were selected; and for HLA-B, B*40 homozygote and B*46 homozygote, which had relatively high frequency in the population, were mainly selected.

**Example 4. Preparation Method for B7H3-Targeted CAR-T Cells**

4-1. Preparation of B7H3-specific UCAR-T cells with double knockout of TRAC and HLA-A

Peripheral blood of healthy people was collected, an HLA typing assay was performed, and typing meeting the requirements was selected.

PBMC isolation: blood in a blood collection tube or a blood collection bag was transferred into a 50 mL centrifuge tube and centrifuged; the upper plasma was discarded, and an equal volume of PBS was added to the lower blood cell layer and mixed uniformly; the mixed solution was pipetted and slowly added to a centrifuge tube containing a lymphocyte isolation solution, with a ratio of DPBS:blood:lymphocyte isolation solution = 1:1:1, and the mixture was centrifuged; and a buffy coat was collected, washed by centrifugation with PBS, diluted, and counted, followed by transferring $1 \times 10^6$ cells

into an EP tube for flow cytometry.

PBMC cryopreservation: whether the cells were cryopreserved or not was determined according to actual conditions. CD3+ T cell sorting: the cells were resuspended in a certain amount of buffer and mixed uniformly; CD3 magnetic beads were added according to 20 $\mu$L of CD3 magnetic beads per 107 cells, and the mixture was mixed uniformly and incubated in a refrigerator at 4 °C; the cells were washed with the buffer and centrifuged, and then the cells were resuspended for magnetic bead isolation; a column was first put on a magnetic pole, and a centrifuge tube was correspondingly put below the magnetic pole; the column (LS) was infiltrated in the buffer, and the cells were added onto the column without generating bubbles; the column was washed 3 times with the buffer, and the column was detached and put into a clean 15 mL centrifuge tube to collect CD3+ T cells; and the buffer was pipetted and added to the column, the CD3-labeled cells were eluted using a plunger, and a part of the cells were subjected to cell counting.

CD3/CD28 magnetic bead activation: the cells were resuspended at a density of $1 \times 10^6$ cells/mL based on the cell counting results; an activating reagent was prepared; a complete medium (containing cytokines IL2, IL7, and IL15) was added; and the cells were resuspended thoroughly and incubated at 37 °C with 5% $CO_2$.

Virus transfection: 24 h after the magnetic bead activation, the virus (i.e., the lentiviral vector prepared in Example 2, where B7H3 VHH1 was 1G7, and B7H3 VHH2 was 1A5) was added according to the MOI of the B7H3 virus; the mixture was centrifuged at room temperature; and after the centrifugation, the cells were incubated at 37 °C with 5% $CO_2$ for 24 h.

Gene knockout: after sampling and counting, the cells were collected and centrifuged, and the cell pellet was resuspended in PBS; an electroporation buffer was prepared according to a ratio of nucleofector solution: supplement = 82:18; the RNP complexes (Cas9:sgRNA = 2:1) were distributed according to each electroporation system using $1 \times 107$ cells; the electroporation was performed; 48 h after the electroporation, the cells were collected; and the editing efficiency was assayed by Sanger sequencing, and the knockout efficiency of the collected cells was assayed by FACS.

The sgRNA sequences are as follows: TRAC sgRNA: AGAGTCTCTCAGCTGGTACA; A02 sgRNA: CTGACCATGAAGCCACCCTG; A11 sgRNA: GGCCCCTCCTGCTCTATCCA. CD3-negative T cell sorting: CD3-negative T cells were sorted; the cells were counted and centrifuged, and the supernatant was discarded; the cells were resuspended in a buffer and mixed uniformly; CD3 magnetic beads were added according to 20 $\mu$L of CD3 magnetic beads per 107 cells, and the mixture was mixed uniformly and incubated in a refrigerator at 4 °C; the cells were washed with the buffer and centrifuged, and then the magnetic bead isolation was performed; a column was first put on a magnetic pole, and a centrifuge tube was correspondingly put below the magnetic pole; the column (LD) was infiltrated in the buffer, and the cells were added onto the column without generating bubbles; and the column was washed 2 times with the buffer, the washed liquid (CD3-T) was collected in a 15 mL centrifuge tube, and a part of the cells was subjected to cell counting. Cell culture: the cell state was observed under a microscope; the cells were diluted, counted, and supplemented with a complete medium to maintain the cell density at $3 \times 10^5$ to $1 \times 10^6$ cells/mL; the liquid was supplemented/changed in the middle time; and the cells were cultured at 37 °C with 5% $CO_2$.

Cell harvesting: the cell suspension was collected in a cell centrifuge tube and centrifuged, and the supernatant was discarded; the cells were washed with normal saline again and centrifuged; a cryopreservation solution was prepared, and the centrifuged cells were resuspended in the cryopreservation solution; the cell suspension was pipetted to a cell cryopreservation bag for a final product by using a syringe; and the cell cryopreservation bag was labeled for the later cryopreservation.

4-2. Preparation of B7H3-specific UCAR-T cells with double knockout of TRAC and HLA-B

The preparation procedure was similar to that of Example 4-1, except that the used sgRNA with knockout of the HLA-A gene was replaced by HLA-B sgRNA, specifically HLA-B40 Sg6: 5-CATGTCCCGGCCCGGCCGCG-3, HLA-B40 Sg19: 5-CCGGACGGGCGCCTCCTCCG-3, and HLA-B46 Sg14: 5-GTGAGCCTGCGGAACCTGCG-3.

4-3. Preparation of B7H3-specific UCAR-T cells with triple knockout of TRAC and HLA-A/B

The preparation procedure was similar to that of Example 4-1, except that the used sgRNA with knockout of the HLA-A gene was replaced by an equal amount of a mixture of HLA-A sgRNA and HLA-B sgRNA.

4-4. Preparation of B7H3-specific UCAR-T cells with triple knockout of TRAC and HLA-A/B and expressing membrane-bound IL15

The preparation procedure was similar to that of Example 4-3, except that the lentivirus used for T cell transfection was replaced by a B7H3-specific CAR lentiviral vector expressing membrane-bound IL15.

4-5. Preparation of autologous B7H3-specific CAR-T cells

The preparation procedure was similar to that of Example 4-3, except that the gene knockout and CD3-negative T cell sorting steps were removed.

4-6. Preparation of single-epitope B7H3 UCAR-T cells with double knockout of TRAC and HLA-A (1G7)

The preparation procedure was similar to that of Example 4-1, except that the structure of the CAR was changed from two B7H3 antigen-binding regions to one B7H3 antigen-binding region 1G7.

4-7. Preparation of single-epitope B7H3 UCAR-T cells with double knockout of TRAC and HLA-A (1A5)

The preparation procedure was similar to that of Example 4-1, except that the structure of the CAR was changed from two B7H3 antigen-binding regions to one B7H3 antigen-binding region 1A5.

**Example 5.** *In Vitro* **Cytotoxicity Analysis of B7H3-Targeted CAR-T Cells**

1. Different types of T cells were prepared as follows:

    A: bi-epitopic B7H3 UCAR-T cells with double knockout of TRAC and HLA-A, as described in Example 4-1;

    B: bi-epitopic B7H3 UCAR-T cells with double knockout of TRAC and HLA-B, as described in Example 4-2;

    C: bi-epitopic B7H3 UCAR-T cells with triple knockout of TRAC and HLA-A/B, as described in Example 4-3;

    D: single-epitope B7H3 UCAR-T cells (1G7) with double knockout of TRAC and HLA-A, as described in Example 4-6;

    E: single-epitope B7H3 UCAR-T cells (1A5) with double knockout of TRAC and HLA-A, as described in Example 4-7;

    G: bi-epitopic B7H3-targeted CAR-T cells (autologous); and

    H: wild-type T cells.

2. Killing of B7H3 CAR-T cells against target cells:
1) B7H3 target cells: a U251-LG cell line with high expression of B7-H3 was selected (the U251 cell line was modified to express luciferase and GFP); and the state of the target cells was adjusted to the log phase, and the cells were continuously passaged 2 times before experiments.
2) The B7H3 CAR-T cells in groups A-G and control T cells were prepared; the knockout efficiency, transfection efficiency, CD3-T sorting efficiency, and the proportion of memory T cells were assayed by flow cytometry, and the expansion fold was counted.

| Experimental group | Knockout efficiency (%) | Transfection efficiency (%) |
|---|---|---|
| A | 97 | 54 |
| B | 98 | 50 |
| C | 98 | 54 |
| D | 98 | 82 |
| E | 96 | 73 |
| G | - | 60 |

3) Several groups of prepared cells were collected by centrifugation, each group of $6 \times 10^6$ cells.
4) The effector cells and target cells were separately resuspended in 1640 + 10% FBS, and the densities were adjusted to $2 \times 10^6$ cells/mL. The target cells were seeded into a 24-well plate at $2 \times 10^5$ cells/well, followed by the addition of effector cells at different E/T ratios (effector-to-target ratios, effector cells:target cells); and the 1640 + 10% FBS medium was added, and the culture wells with a total volume less than 1 mL were supplemented to 1 mL. The well plate was incubated in an incubator with 5% $CO_2$ at 37 °C for 24 h. The cells were seeded at the following E/T ratios: 0.5:1, 1:1, 2:1, 5:1, and 10:1, and repeated three times.

5) After 24 h of culture, the well plate was taken out of the incubator, and 200 μL of supernatant was collected. The lysis capacity of the recombinant CAR-T cells on the target cells was then reflected by the assay for luciferase activity (i.e., Luc activity).

6) The calculation formula for the lysis percentage of the target cells is as follows:

$$Lysis\% = \left(1 - \frac{Luc\text{Activity}_{\text{Mixed sample}}}{Luc\text{Activity}_{\text{Control sample}}}\right) \times 100\%$$

Analysis of results: bi-epitopic B7H3-targeted UCAR-T with double knockout of TRAC and HLA-A and bi-epitopic B7H3 CAR-T cells had significant cytotoxic efficacy against U251 cells (see FIG. 2). From the *in vitro* killing results, it can be seen that the bi-epitopic UCAR-T and CAR-T cells had stronger killing ability against target cells.

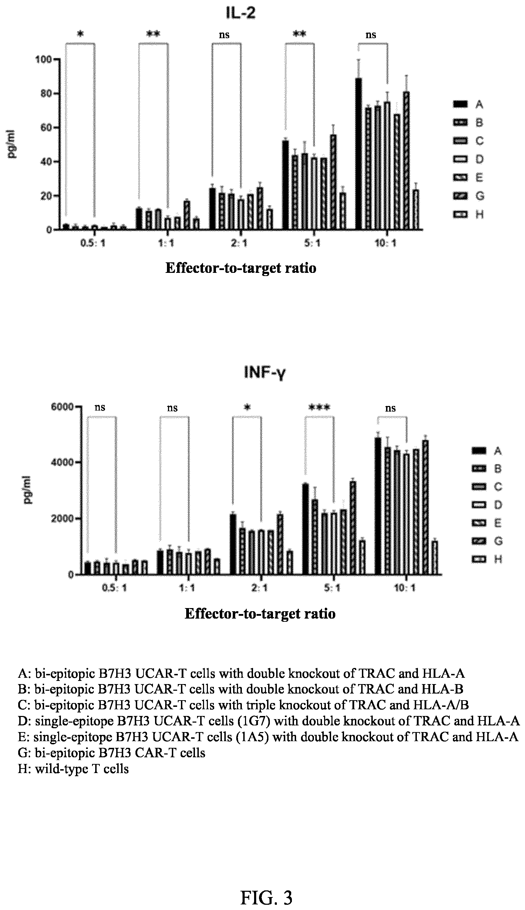

3. Cytokine secretion assay of B7H3-targeted CAR-T cells (autologous and allogeneic) co-cultured with target cells (see FIG. 3).

The supernatants of the co-culture systems described above were collected, and the cytokine secretion levels were assayed. The experimental results are shown in FIG. 3.

Analysis of results: B7H3 UCAR-T and B7H3 CAR-T cells were significantly activated, and the IL-2 and IFN-γ cytokines were extensively secreted. At the same effector-to-target ratio, the secretion levels of IL-2 and INF-γ in the bi-epitopic UCAR-T and CAR-T cells were higher than those of the test cells in other groups, indicating that the bi-epitopic UCAR-T cells are more easily activated and have better cell activity after the activation as compared to the single-epitope UCAR-T cells.

## Example *6. In Vivo* Anti-Tumor Efficacy of B7H3-Targeted CAR-T Cells

Tumor cells U251-LG were intravenously injected into NSG mice aged 8-10 weeks at $2 \times 10^6$ cells/mouse, and the mice were divided into A-G groups of 5 mice each. On day 7 after the tumor was successfully established, the cells in groups A-G were intratumorally injected into the mice at $5 \times 10^6$ cells/mouse, and tumor regression in mice was monitored by luciferase.

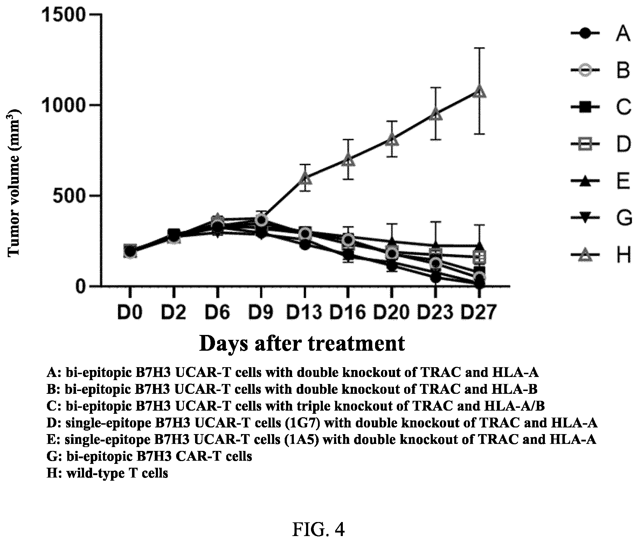

Analysis of results (FIG. 4): the tumor-bearing mice to which the bi-epitopic B7H3 UCAR-T cells with double knockout of TRAC and HLA-A or bi-epitopic B7H3 CAR-T cells were reinfused exhibited a significantly slower growth rate of the tumor. The *in vivo* tumor inhibitory effects of the bi-epitopic cells were more significant, and the recurrence did not occur in the later period of the experiment.

## Example 7. Safety Analysis of B7-H3-Targeted CAR-T

The affinity of the B7H3 antibody screened by the inventors is weaker than that of the B7-H3 antibody (Hbrca84d-2) in the patent CN106279416B, and thus, the safety of the two antibodies was analyzed.

    1. Different types of T cells were prepared as follows:

        A: bi-epitopic B7H3 UCAR-T cells with double knockout of TRAC and HLA-A;

        B: single-epitope B7H3 UCAR-T cells (1G7) with double knockout of TRAC and HLA-A;

        C: single-epitope B7H3 UCAR-T cells (1A5) with double knockout of TRAC and HLA-A;

        D: bi-epitopic B7H3 CAR-T cells;

        E: B7H3 UCAR-T cells with double knockout of TRAC and HLA-A (Hbrca84d-2);

        F: B7H3 CAR-T cells (Hbrca84d-2); and

        G: wild-type T cells.

The preparation procedure of E was similar to that of Example 4-1, except that the structure of the CAR was changed from two B7H3 antigen-binding regions to a B7H3 antigen-binding region Hbrca84d-2.

The preparation procedure of F was similar to that of Example 4-5, except that the structure of the CAR was changed from two B7H3 antigen-binding regions to a B7H3 antigen-binding region Hbrca84d-2.

The sequence of Hbrca84d-2 is as follows:

Hbrca84d-2 sequence:

MALPVTALLLPLALLLHAARPEVQLVESGGGLVQPGGSLRLSCAASGFTF

SSFGMHWVRQAPGKGLEWVAYISSDSSAIYYADTVKGRFTISRDNAKNSL

YLQMNSLRDEDTAVYYCGRGRENIYYGSRLDYWGQGTTVTVSSGGGGS

GGGGSGGGGSDIQLTQSPSFLSASVGDRVTITCKASQNVDTNVAWYQQKP

GKAPKALIYSASYRYSGVPSRFSGSGSGTDFTLTISSLQPEDFATYYCQQY

NNYPFTFGQGTKLEIK

2. Killing of B7H3 CAR-T cells against target cells weakly expressing B7-H3:

1) Selection of target cells weekly expressing B7H3: K562 tumor cells were selected as target cells; the state of the target cells was adjusted to the log phase; and the cells were continuously passaged 2 times before experiments.

2) The cells in groups A-G were prepared.

3) Several groups of prepared cells were collected by centrifugation, each group of $6 \times 10^6$ cells.

4) The target cells were resuspended in 1640 + 10% FBS. For each target, three 24-well plates were taken, and the target cells were seeded at $2 \times 10^5$ cells/well (both target and effector cells were seeded at a density of $2 \times 10^6$ cells/mL). Effector cells were then added at an E/T ratio (effector-to-target ratio, effector cells:target cells). Each well was supplemented to a maximum volume (600 $\mu$L). The same amount of target cells were seeded in the control group, without effector cells (600 $\mu$L). The well plate was incubated in an incubator with 5% $CO_2$ at 37 °C for 24 h. The cells were plated at the following E/T ratios: 0.5:1, 1:1, 2:1, 5:1, and 10:1, and repeated three times.

5) After 24 h of culture, the well plate was taken out of the incubator, and 200 $\mu$L of supernatant was collected. The lysis capacity of the recombinant CAR-T cells on the target cells was then reflected by the assay for U251 activity.

6) The calculation formula for the lysis percentage of the target cells is as follows:

$$Lysis\% = (1 - \frac{Luc\text{Activity}_{\text{Mixed sample}}}{Luc\text{Activity}_{\text{Control sample}}}) \times 100\%$$

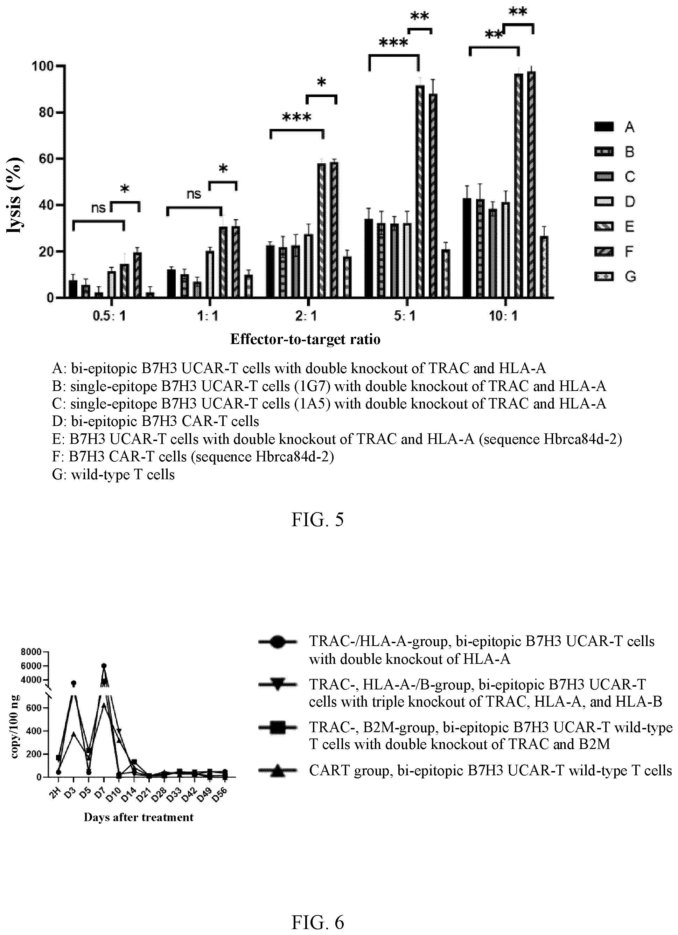

Analysis of results: the killing effects of single-epitope or bi-epitopic B7-H3-targeted UCAR-T cells (groups A, B, and C) and CAR-T cells (group D) on the target cells weakly expressing B7-H3 were weaker than those of groups E and F, and were not significantly different from the killing effect of the wild-type T cells on the target cells weakly expressing B7-H3 (as shown in FIG. 5), indicating that the CAR-T cells with the relatively low-affinity antibody have no/low toxic and side effects on normal tissues of the human body, and have better safety *in vivo* than that of the CAR-T cells with the high-affinity antibody.

**Example 8. *In Vivo* Half-Life Assay of B7H3-Targeted UCAR Cells**

Three humanized immune system mice (hHSC-NCG) were prepared, B7H3 CAR-T or T cells of groups A-D were prepared, and the cells were injected into the humanized immune mice at $1 \times 10^7$ cells/mouse; blood was collected at different time points: D0, 2 h, D3, D7, D10, D14, D21, D28, D33, D42, D49, and D56; genomes in the blood samples at different time points were extracted, and copy/ng genome DNA was calculated by QPCR absolute quantification method; and UCAR-T cells harvested on day 14 were used as a positive control, and DEPC water was used as a negative control.

Different types of T cells in groups A-D in Example 8 were prepared as follows:

A: bi-epitopic B7H3-targeted UCAR-T cells with double knockout of TRAC and HLA-A, as described in Example 4-1;

C: bi-epitopic B7H3-targeted UCAR-T cells with triple knockout of TRAC and HLA-A/B, as described in Example 4-3;

C: bi-epitopic B7H3-targeted UCAR-T wild-type T cells with double knockout of TRAC and B2M, which was similar to that in Example 4-5, except that TRAC and B2M were further knocked out; and

D: bi-epitopic B7H3-targeted UCAR-T wild-type T cells, as described in Example 4-5.

Analysis of results: UCAR-T in groups A and B exhibited the most *in vivo* expansion and could still be detected in mice on D56, and the *in vivo* persistence and number of these cells were better than those in the other two groups (see FIG. 6).

## Example 9. *In Vitro* Safety Validation of Universal T Cells

1) GVHD response: T cells with double knockout of TRAC and HLA-A, T cells with double knockout of TRAC and HLA-B, T cells with triple knockout of TRAC and HLA-A/B, and T cells without gene knockout were prepared; these groups of prepared cells were separately stimulated by using irradiated allogeneic PBMCs; and IFN-$\gamma$ levels were assayed. Analysis of results: the T cell group with double knockout of TRAC and HLA-A, the T cell group with double knockout of TRAC and HLA-B, and the T cell group with triple knockout of TRAC and HLA-A/B had very low IFN-$\gamma$ secretion levels, indicating that the knockout of TRAC reduces the GVHD response.

2) Allogeneic response: after the allogeneic PBMCs were stimulated and irradiated, 4 groups of cells were assayed for the IFN-$\gamma$ levels.

Analysis of results: the T cell group with double knockout of TRAC and HLA-A, the T cell group with double knockout of TRAC and HLA-B, and the T cell group with triple knockout of TRAC and HLA-A/B had very low IFN-$\gamma$ secretion levels, indicating that the knockout of HLA-A reduces the allogeneic response.

## Example 10. *In Vivo* Safety Validation of Universal T Cells

1) GVHD response: bi-epitopic B7H3 CAR-T cells with double knockout of TRAC and HLA-A (TRAC-/HLA-A-group), bi-epitopic B7H3 CAR-T cells with double knockout of TRAC and HLA-B (TRAC-/HLA-B-group), and bi-epitopic B7H3 CAR-T cells with triple knockout of TRAC and HLA-A/B (TRAC, HLA-A-/HLA-B-group) were prepared. The cells were injected into NSG mice aged 8-10 weeks at $1 \times 10^7$ cells/mouse, and according to clinical criteria, such as survival rate, coat texture, skin integrity, and the like, graft-versus-host response was observed. Assay for cytokines: peripheral blood serum was collected to assay the levels of cytokines such as IL-2, TNF-$\alpha$, and IFN-$\gamma$. The blood collection time points were as follows: before the reinfusion, D1, D3, D7, D14, D21, D28, D35, D42, D49, D56, and D63. Assay for visceral lesion: at the end of the observation period (about 2 months), the spleen, liver, skin, gastrointestinal tract, lung, and kidney of the mice were collected for HE section staining analysis.

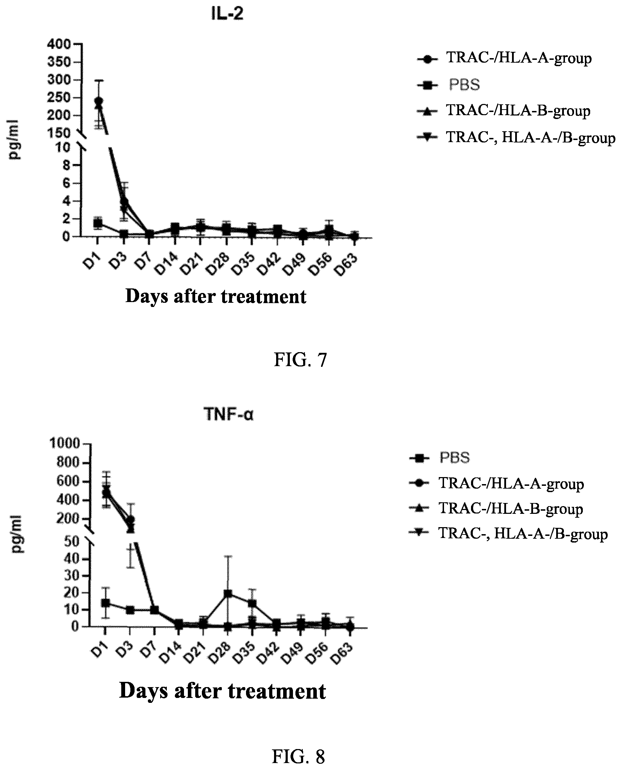

Analysis of results: different gene knockout strategies were effective in reducing the risk of GVHD, with extensive secretion of cytokines occurring in a short time after reinfusion, and then the cytokines in each group were maintained at low levels (see FIGs. 7-9), while there were no abnormalities during the mouse cage-side observation.

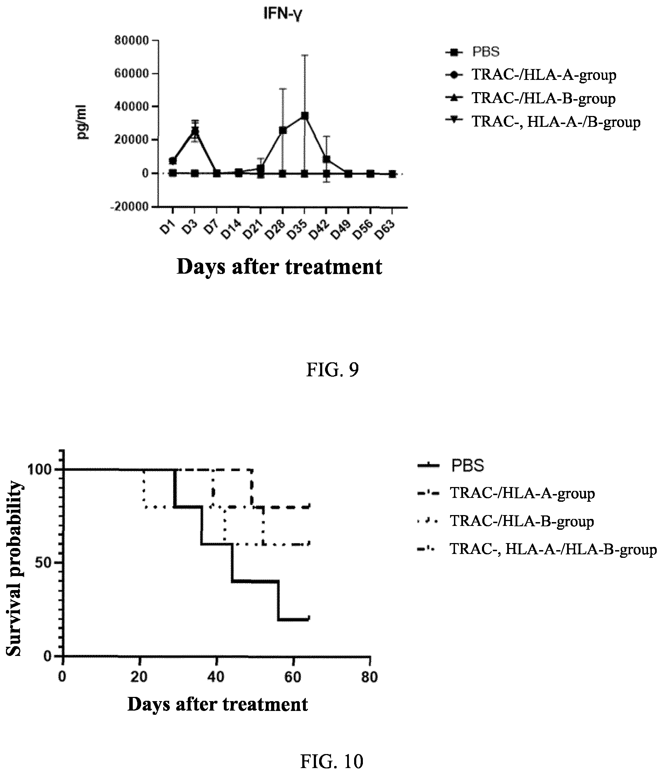

According to survival rate analysis (as shown in FIG. 10), the survival rates of the TRAC-/HLA-A-group, the TRAC-/HLA-B-group, and the TRAC-/HLA-A-/HLA-B-group were 80%, 60%, and 60%, respectively, 60 days after the reinfusion, indicating that the TRAC-/HLA-A-group can better improve the survival rate of the mice and further reduce the risk of GVHD.

2) Allogeneic response: four groups of cells, i.e., bi-epitopic B7H3 CAR-T cells with double knockout of TRAC and HLA-A, bi-epitopic B7H3 CAR-T cells with triple knockout of TRAC and HLA-A/B, bi-epitopic B7H3 CAR-T cells with double knockout of TRAC and B2M, and bi-epitopic B7H3 CAR-T cells with single knockout of TRAC, were prepared and separately injected into huHSC-NCG-hIL15 mice at $1 \times 10^7$ cells/mouse. The used huHSC-NCG-hIL5 mouse model achieves the aim of reconstructing a human immune system by reinfusing human hematopoietic stem cells, and this model can effectively reconstruct human T, B, and NK cells. Control group: PBS buffer was injected into NSG mice.

Blood was collected at different time points to determine the CAR copy number. The changes in the copy number were compared for CARs. Time points: 2 h before the reinfusion, D2, D4, D8, D15, D22, and D31. Assay for cytokines: peripheral blood serum was collected to assay the levels of cytokines such as IL6. Time points: 2 h before the reinfusion, D2, D4, D8, D13, D17, D22, D29, and D36.

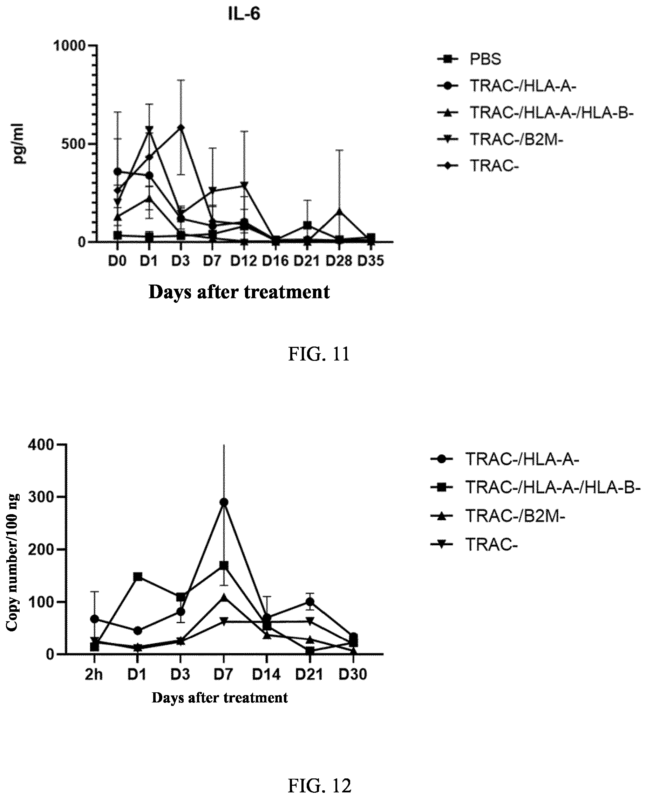

Conclusion: according to the assay results of IL-6 (see FIG. 11), the secretion level in the UCAR-T cells of the TRAC-/HLA-A-group was less than that of the other groups, and the risk of cytokine storm could be effectively reduced. According to the copy number assay, the UCAR-T cells of the TRAC-/HLA-A-group exhibited significant expansion in mice, and the number of surviving copies was higher than that of the other groups (see FIG. 12).

### Example 11. Safety Analysis of Gene Editing

T cells with double knockout of TRAC and HLA-A and T cells without gene knockout were prepared, and after the assay for knockout efficiency, the following analyses were performed:

1) Off-target: **off-target: on-target and off-target: (GUIDE-seq):** control group: transgenic CAS9+ ODN tag; experimental group: transgenic CAS9 + sgRNA (TRAC + HLA-A) + ODN tag. On D14, the DNA in the two groups of cells was extracted and sent to Suzhou Genewiz Biological Technology Co., Ltd. for assay.

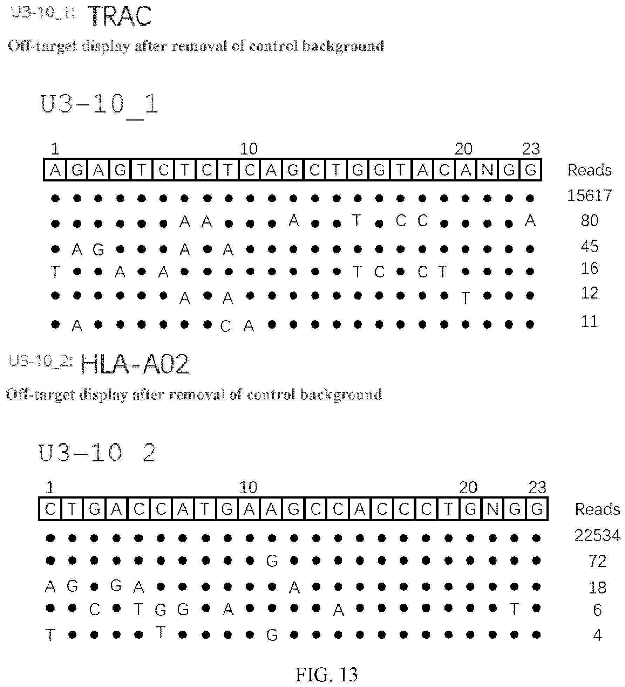

Analysis of results: the off-target rate of the experimental group was very low, and the off-target was mainly concentrated among genes and on introns, so that the effect on the gene function is not great (see FIG. 13).

2) Chromosome translocation: the qPCR method was used to quantify rearrangements that may occur when editing both TRAC and HLA loci simultaneously. The two translocations were labeled as TRAC:HLA and HLA:TRAC. Positive reference samples in the synthesized template plasmid were evaluated as assay controls. Amplified fragments on both sides of the target region of the HLA genome were used as internal controls. The genome DNA was extracted to perform real-time quantitative PCR, and the gene copy number of the genome DNA was calculated according to the standard curve and Cq value.

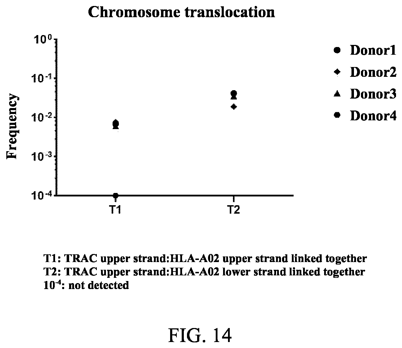

Analysis of results: no rearrangement of the loci occurred (see FIG. 14). The assay values for the two types of translocation were close to zero.

3) Karyotyping: $1 \times 10^6$ of T cells without gene knockout (control group) and T cells with double knockout of TRAC and HLA-A (experimental group), which had a confluence of 70%-80%, were each put into two T25 bottles. The bottles were filled with a culture medium, covered with a fully sealed lid, wrapped with a sealing film, and sent to Zhejiang Ruyao Biotech Co., Ltd. for assay.

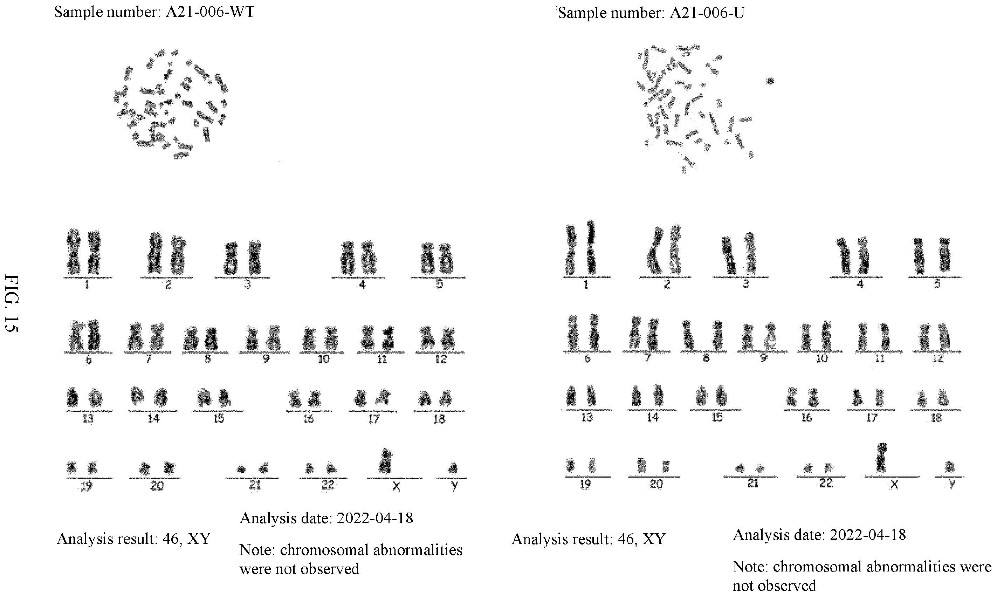

Analysis of results: compared with the control group, the experimental group was normal in the karyotype (see FIG. 15).

4) Cas9 protein residue: when the cells were prepared, $1 \times 10^6$ cells at three time points, i.e., before knockout, after knockout, and before harvest, were separately collected for lysis, then a protein quantification kit (NOVATEINBIO, Catalog No. NB-E1372PR) was used for quantification, and each group of samples were adjusted to be 2 $\mu$g of the same sample loading amount and were assayed by a CRISPR/Cas9 protein ELISA kit according to the instruction. The Cas9 protein in the sample was firmly and stably attached to a test paper hole. The bound Cas9 protein was then recognized using an assay antibody and then developed with a developing agent. The Cas9 ratio was directly proportional to the absorbance, and absolute amounts of Cas9 protein were quantified by comparison to Cas9 control samples.

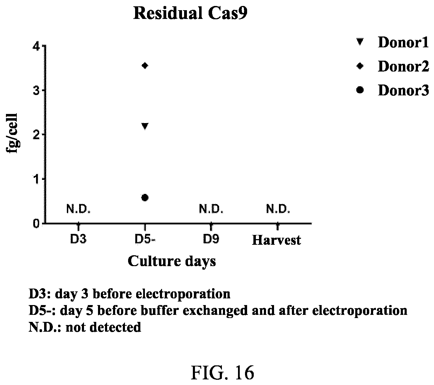

Analysis of results: the T cells with double knockout (TRAC + HLA-A) were assayed for the residue of spCas9 at four time points, i.e., before electroporation (D3), before buffer exchange and after electroporation (D5), D9, and D14 (harvest). The residues were not detected at all the other three time points except for the assay for trace residue before buffer exchange and after electroporation (D5) (see FIG. 16).

### Claims

1. An isolated B7H3-binding polypeptide, comprising a first heavy chain variable region of a heavy-chain antibody (VHH-1) and a second heavy chain variable region of a heavy-chain antibody (VHH-2), both targeting B7H3, wherein the VHH-1 comprises an HCDR1 set forth in SEQ ID NO: 1, an HCDR2 set forth in SEQ ID NO: 3, and an HCDR3 set forth in SEQ ID NO: 5; and/or the VHH-2 comprises an HCDR1 set forth in SEQ ID NO: 2, an HCDR2 set forth in SEQ ID NO: 4, and an HCDR3 set forth in SEQ ID NO: 6.

2. The B7H3-binding polypeptide according to claim 1, wherein the VHH-1 and/or VHH-2 comprises a human antibody, a humanized antibody, or a chimeric antibody.

3. The B7H3-binding polypeptide according to any one of claims 1-2, wherein the VHH-1 and VHH-2 are linked directly or via a linker.

4. The B7H3-binding polypeptide according to any one of claims 1-3, wherein the B7H3-binding polypeptide sequentially comprises, from the N-terminus to the C-terminus, i) VHH-1-linker-VHH-2; or ii) VHH-2-linker-VHH-1.

5. The B7H3-binding polypeptide according to any one of claims 1-4, wherein the linker comprises the amino acid sequence set forth in SEQ ID NO: 22.

6. The B7H3-binding polypeptide according to any one of claims 1-4, wherein the VHH-1 comprises:

   i) an HFR1 comprising the amino acid sequence set forth in SEQ ID NO: 7, an HFR2 comprising the amino acid sequence set forth in SEQ ID NO: 11, an HFR3 comprising the amino acid sequence set forth in SEQ ID NO: 13, and an HFR4 comprising the amino acid sequence set forth in SEQ ID NO: 16; or
   ii) an HFR1 comprising the amino acid sequence set forth in SEQ ID NO: 8, an HFR2 comprising the amino acid sequence set forth in SEQ ID NO: 11, an HFR3 comprising the amino acid sequence set forth in SEQ ID NO: 14, and an HFR4 comprising the amino acid sequence set forth in SEQ ID NO: 17.

7. The B7H3-binding polypeptide according to any one of claims 1-6, wherein the VHH-1 comprises the amino acid sequence set forth in SEQ ID NO: 18 or SEQ ID NO: 19.

8. The B7H3-binding polypeptide according to any one of claims 1-7, wherein the VHH-2 comprises:

   i) an HFR1 comprising the amino acid sequence set forth in SEQ ID NO: 9, an HFR2 comprising the amino acid sequence set forth in SEQ ID NO: 12, an HFR3 comprising the amino acid sequence set forth in SEQ ID NO: 15, and an HFR4 comprising the amino acid sequence set forth in SEQ ID NO: 16; or
   ii) an HFR1 comprising the amino acid sequence set forth in SEQ ID NO: 10, an HFR2 comprising the amino acid sequence set forth in SEQ ID NO: 12, an HFR3 comprising the amino acid sequence set forth in SEQ ID NO: 14, and an HFR4 comprising the amino acid sequence set forth in SEQ ID NO: 17.

9. The B7H3-binding polypeptide according to any one of claims 1-8, wherein the VHH-2 comprises the amino acid sequence set forth in SEQ ID NO: 20 or SEQ ID NO: 21.

10. The B7H3-binding polypeptide according to any one of claims 1-9, comprising the amino acid sequence set forth in any one of SEQ ID NO: 23, SEQ ID NO: 24, SEQ ID NO: 26, and SEQ ID NO: 25.

11. An immunoconjugate, comprising the B7H3-binding polypeptide according to any one of claims 1-10.

12. A chimeric antigen receptor, comprising an extracellular antigen-binding domain targeting B7H3, a transmembrane domain, an intracellular co-stimulatory signaling domain, and an intracellular signaling domain, wherein the extracellular antigen-binding domain comprises at least two VHHs that bind to distinct epitopes of B7H3.

13. The chimeric antigen receptor according to claim 12, wherein the extracellular antigen-binding domain comprises two VHHs that bind to distinct epitopes of B7H3.

14. A chimeric antigen receptor, comprising an extracellular antigen-binding domain targeting B7H3, a transmembrane domain, an intracellular co-stimulatory signaling domain, and an intracellular signaling domain, wherein the extracellular antigen-binding domain comprises the B7H3-binding polypeptide according to any one of claims 1-10.

15. The chimeric antigen receptor according to any one of claims 12-14, wherein the transmembrane domain comprises a transmembrane domain derived from one or more proteins selected from the group consisting of: CD8A, CD8B, CD28, CD3ε (CD3e), 4-1BB, CD4, CD27, CD7, PD-1, TRAC, TRBC, CD3ζ, CTLA-4, LAG-3, CD5, ICOS, OX40, NKG2D, 2B4 (CD244), FcεRIγ, BTLA, CD30, GITR, HVEM, DAP10, CD2, NKG2C, LIGHT, DAP12, CD40L (CD154), TIM1, CD226, DR3, CD45, CD80, CD86, CD9, CD16, CD22, CD33, CD37, CD64, and SLAM.

16. The chimeric antigen receptor according to claim 15, wherein the transmembrane domain comprises a transmembrane domain derived from CD8A.

17. The chimeric antigen receptor according to any one of claims 12-16, wherein the transmembrane domain comprises the amino acid sequence set forth in any one of SEQ ID NO: 62 to SEQ ID NO: 110.

18. The chimeric antigen receptor according to any one of claims 12-17, wherein the transmembrane domain comprises the amino acid sequence set forth in SEQ ID NO: 62.

19. The chimeric antigen receptor according to any one of claims 12-18, wherein the intracellular co-stimulatory signaling domain comprises an intracellular co-stimulatory signaling domain derived from one or more proteins selected from the group consisting of: CD28, CD137, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

20. The chimeric antigen receptor according to claim 19, wherein the intracellular co-stimulatory signaling domain is derived from a 4-1BB co-stimulatory signaling domain.

21. The chimeric antigen receptor according to any one of claims 12-20, wherein the intracellular co-stimulatory signaling domain comprises the amino acid sequence set forth in any one of SEQ ID NO: 111 to SEQ ID NO: 143.

22. The chimeric antigen receptor according to any one of claims 12-21, wherein the intracellular co-stimulatory signaling domain comprises the amino acid sequence set forth in SEQ ID NO: 112.

23. The chimeric antigen receptor according to any one of claims 12-22, wherein the intracellular signaling domain comprises an intracellular signaling domain derived from one or more proteins selected from the group consisting of: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, DAP10, DAP-12, and a domain comprising at least one ITAM.

24. The chimeric antigen receptor according to claims 12-23, wherein the intracellular signaling domain comprises a signaling domain derived from CD3ζ.

25. The chimeric antigen receptor according to any one of claims 12-23, wherein the intracellular signaling domain comprises the amino acid sequence set forth in any one of SEQ ID NO: 127, SEQ ID NO: 131, SEQ ID NO: 132, and SEQ ID NO: 144 to SEQ ID NO: 154.

26. The chimeric antigen receptor according to any one of claims 12-25, wherein the intracellular signaling domain comprises the amino acid sequence set forth in SEQ ID NO: 144.

27. The chimeric antigen receptor according to any one of claims 12-26, comprising a hinge region between the extracellular antigen-binding domain and the transmembrane domain, wherein the hinge region comprises a hinge region derived from one or more proteins selected from the group consisting of: CD28, IgG1, IgG4, IgD, 4-1BB, CD4, CD27, CD7, CD8A, PD-1, ICOS, OX40, NKG2D, NKG2C, FcεRIγ, BTLA, GITR, DAP10, TIM1, SLAM, CD30, and LIGHT.

28. The chimeric antigen receptor according to any one of claims 12-27, wherein the hinge region comprises a hinge region derived from CD8A.

29. The chimeric antigen receptor according to any one of claims 12-128, wherein the hinge region comprises the amino acid sequence set forth in any one of SEQ ID NO: 155 to SEQ ID NO: 176.

30. The chimeric antigen receptor according to any one of claims 12-29, wherein the hinge region comprises the amino acid sequence set forth in SEQ ID NO: 163.

31. The chimeric antigen receptor according to any one of claims 12-30, wherein a non-targeting portion of the chimeric antigen receptor comprises a CD8A hinge region, a CD8A transmembrane domain, a 4-1BB intracellular co-stimulatory signaling domain, and a CD3ζ intracellular signaling domain.

32. The chimeric antigen receptor according to any one of claims 12-31, wherein the non-targeting portion of the chimeric antigen receptor comprises the amino acid sequence set forth in SEQ ID NO: 27.

33. The chimeric antigen receptor according to any one of claims 12-32, further comprising a signal peptide fragment, wherein the C-terminus of the signal peptide fragment is linked to the N-terminus of the extracellular antigen-binding domain.

34. The chimeric antigen receptor according to any one of claims 12-33, wherein the chimeric antigen receptor sequentially comprises, from the N-terminus to the C-terminus, the signal peptide fragment, the extracellular antigen-binding domain, the transmembrane domain, the intracellular co-stimulatory signaling domain, and the intracellular signaling domain.

35. The chimeric antigen receptor according to any one of claims 33-34, wherein the signal peptide fragment comprises a CD8A signal peptide fragment.

36. The chimeric antigen receptor according to any one of claims 33-35, wherein the signal peptide fragment comprises the amino acid sequence set forth in SEQ ID NO: 28.

37. The chimeric antigen receptor according to any one of claims 12-36, comprising the amino acid sequence set forth in any one of SEQ ID NO: 33 to SEQ ID NO: 36.

38. One or more isolated polypeptides, comprising the chimeric antigen receptor according to any one of claims 12-37 and an enhancer capable of enhancing one or more activities of an immune effector cell.

39. The polypeptide according to claim 38, wherein the enhancer comprises a cytokine and/or a cytokine receptor.

40. The polypeptide according to any one of claims 38-39, wherein the enhancer is a membrane-bound cytokine and/or cytokine receptor.

41. The polypeptide according to any one of claims 38-40, wherein the enhancer is selected from the group consisting of: IL-2, IL-3, IL-4, IL-6, IL-7, IL-8, IL-10, IL-11, IL-12, IL-15, IL-17, IL-18, IL-21, IL-23, receptors thereof, functional variants thereof, and combinations thereof.

42. The polypeptide according to any one of claims 38-41, wherein the enhancer comprises IL-15 or a functional variant thereof, or a chimeric cytokine receptor comprising an IL-15 receptor (IL-15R).

43. The polypeptide according to any one of claims 38-42, wherein the enhancer comprises membrane-bound IL-15 (mIL-15).

44. The polypeptide according to any one of claims 38-43, wherein the enhancer comprises the amino acid sequence set forth in SEQ ID NO: 46 or SEQ ID NO: 54.

45. The polypeptide according to any one of claims 38-44, comprising i) the amino acid sequence set forth in any one of SEQ ID NO: 33 to SEQ ID NO: 36; and ii) the amino acid sequence set forth in SEQ ID NO: 46 or SEQ ID NO: 54.

46. The polypeptide according to any one of claims 38-45, wherein the chimeric antigen receptor and the enhancer are located in separate polypeptides.

47. The polypeptide according to any one of claims 38-45, wherein the chimeric antigen receptor and the enhancer are located in the same polypeptide.

48. The polypeptide according to claim 47, wherein the chimeric antigen receptor and the enhancer may be linked directly or indirectly.

49. The polypeptide according to any one of claims 47-48, wherein the chimeric antigen receptor and the enhancer are linked via a linker.

50. The polypeptide according to any one of claims 47-50, comprising the following structures, from the N-terminus to the C-terminus, i) the chimeric antigen receptor, the linker, and the enhancer; or ii) the enhancer, the linker, and the chimeric antigen receptor.

51. The polypeptide according to any one of claims 49-51, wherein the linker comprises a self-cleaving peptide.

52. The polypeptide according to claim 51, wherein the self-cleaving peptide comprises P2A, F2A, E2A, or T2A.

53. The polypeptide according to claim 51, wherein the T2A comprises the amino acid sequence set forth in SEQ ID NO: 44.

54. The polypeptide according to claims 38-53, comprising the amino acid sequence set forth in SEQ ID NO: 241 or SEQ ID NO: 243.

55. One or more isolated nucleic acid molecules encoding the B7H3-binding polypeptide according to any one of claims 1-10, the chimeric antigen receptor according to any one of claims 12-37, or the polypeptide according to any one of claims 38-54.

56. One or more isolated nucleic acid molecules, comprising a first nucleotide sequence encoding the chimeric antigen receptor according to any one of claims 12-37, and a second nucleotide sequence encoding an enhancer capable of enhancing one or more activities of an immune effector cell.

57. The nucleic acid molecule according to claim 56, wherein the enhancer comprises a cytokine and/or a cytokine receptor.

58. The nucleic acid molecule according to any one of claims 56-57, wherein the enhancer is a membrane-bound cytokine and/or cytokine receptor.

59. The nucleic acid molecule according to any one of claims 56-58, wherein the enhancer is selected from the group consisting of: IL-2, IL-3, IL-4, IL-6, IL-7, IL-8, IL-10, IL-11, IL-12, IL-15, IL-17, IL-18, IL-21, IL-23, receptors thereof, functional variants thereof, and combinations thereof.

60. The nucleic acid molecule according to any one of claims 56-59, wherein the enhancer comprises IL-15 or a functional variant thereof, or a chimeric cytokine receptor comprising an IL-15 receptor (IL-15R).

61. The nucleic acid molecule according to any one of claims 56-60, wherein the enhancer comprises membrane-bound IL-15 (mIL-15).

62. The nucleic acid molecule according to any one of claims 56-61, wherein the enhancer comprises the amino acid sequence set forth in SEQ ID NO: 46 or SEQ ID NO: 54.

63. The nucleic acid molecule according to any one of claims 56-62, comprising i) a nucleotide sequence encoding the amino acid sequence set forth in any one of SEQ ID NO: 33 to SEQ ID NO: 36; and ii) the amino acid sequence set forth in SEQ ID NO: 47 or SEQ ID NO: 55.

64. The nucleic acid molecule according to any one of claims 56-63, wherein the first nucleotide sequence and the second nucleotide sequence are located in separate nucleic acid molecules.

65. The nucleic acid molecule according to any one of claims 56-63, wherein the first nucleotide sequence and the second nucleotide sequence are located in the same nucleic acid molecule.

66. The nucleic acid molecule according to claim 65, wherein the first nucleotide sequence and the second nucleotide sequence are linked directly or indirectly.

67. The nucleic acid molecule according to any one of claims 65-66, wherein the first nucleotide sequence and the second nucleotide sequence are linked via a spacer sequence.

68. The nucleic acid molecule according to claim 67, wherein the spacer sequence comprises an internal ribosome entry site (IRES), or the spacer sequence encodes a self-cleaving polypeptide.

69. The nucleic acid molecule according to claim 68, wherein the spacer sequence encodes a viral self-cleaving polypeptide.

70. The nucleic acid molecule according to claim 69, wherein the spacer sequence encodes a viral self-cleaving 2A polypeptide (2A).

71. The nucleic acid molecule according to claim 70, wherein the viral self-cleaving 2A polypeptide comprises a foot-and-mouth disease virus (FMDV) (F2A) peptide, an equine rhinitis A virus (ERAV) (E2A) peptide, a Thoseaasigna virus (TaV) (T2A) peptide, a porcine teschovirus-1 (PTV-1) (P2A) peptide, a Theilovirus 2A peptide, or an encephalomyocarditis virus 2A peptide.

72. The nucleic acid molecule according to any one of claims 67-71, wherein the spacer sequence comprises a nucleotide sequence encoding T2A.

73. The nucleic acid molecule according to any one of claims 67-72, wherein the spacer sequence comprises the nucleotide sequence set forth in SEQ ID NO: 43.

74. The nucleic acid molecule according to any one of claims 68-73, wherein the IRES is selected from at least one of the group consisting of: poliomyelitis IRES, rhinovirus IRES, encephalomyocarditis virus IRES (EMCV-IRES), picornavirus IRES, foot-and-mouth disease virus IRES (FMDV-IRES), oropharyngeal virus IRES, Kaposi's sarcoma-associated herpesvirus IRES (KSHV-IRES), hepatitis A virus IRES, hepatitis C virus IRES, classical swine fever virus IRES, pestivirus IRES, bovine viral diarrhea virus IRES, Friend murine leukemia virus IRES, Moloney murine leukemia virus IRES (MMLV-IRES), Rous sarcoma virus IRES, human immunodeficiency virus IRES (HIV-IRES), Plautia stali intestine virus IRES, dicistrovirus IRES, cricket paralysis virus IRES, Triatoma virus IRES, Rhopalosiphum padi virus IRES, Marek's disease virus IRES, fibroblast growth factors (FGF-1 IRES and FGF-2 IRES), platelet-derived growth factor B (PDGF/c-sis IRES), vascular endothelial growth factor (VEGF IRES), and insulin-like growth factor 2 (IGF-II IRES).

75. The nucleic acid molecule according to any one of claims 56-74, wherein the nucleic acid molecule sequentially comprises, from the 5' end to the 3' end, i) the first nucleotide sequence, the spacer sequence, and the second nucleotide sequence; or ii) the second nucleotide sequence, the spacer sequence, and the first nucleotide sequence.

76. The nucleic acid molecule according to any one of claims 56-75, wherein the nucleic acid molecule sequentially comprises, from the 5' end to the 3' end, a nucleotide sequence encoding the CAR according to any one of claims 12-37, the spacer sequence, and a nucleotide sequence encoding the enhancer.

77. The isolated nucleic acid molecule according to claims 56-76, comprising the nucleotide sequence set forth in SEQ ID NO: 240 or SEQ ID NO: 242.

78. A vector, comprising the isolated nucleic acid molecule according to any one of claims 55-77.

79. The vector according to claim 78, wherein the vector is an expression vector.

80. The vector according to any one of claims 78-79, wherein the vector is selected from the group consisting of: a DNA vector, an RNA vector, a plasmid, a lentiviral vector, an adenoviral vector, an adeno-associated viral vector, and a retroviral vector.

81. A cell, comprising the isolated nucleic acid molecule according to any one of claims 55-77 or the vector according to any one of claims 78-80; and/or expressing the B7H3-binding polypeptide according to any one of claims 1-10, the chimeric antigen receptor according to any one of claims 12-37, or the polypeptide according to any one of claims 38-54.

82. An immune effector cell, comprising the isolated nucleic acid molecule according to any one of claims 55-77 or the vector according to any one of claims 78-80; and/or expressing the B7H3-binding polypeptide according to any one of claims 1-10, the chimeric antigen receptor according to any one of claims 12-37, or the polypeptide according to any one of claims 38-54.

83. An immune effector cell, comprising a chimeric antigen receptor targeting B7H3 and a membrane-bound IL15 molecule (mIL15).

84. The immune effector cell according to claim 83, wherein an extracellular antigen-binding domain of the chimeric antigen receptor comprises at least two VHHs that bind to distinct epitopes of B7H3.

85. The immune effector cell according to any one of claims 83-84, wherein the extracellular antigen-binding domain comprises two VHHs that bind to distinct epitopes of B7H3.

86. The immune effector cell according to any one of claims 83-85, wherein the mIL15 comprises the amino acid sequence set forth in any one of SEQ ID NO: 46 or SEQ ID NO: 54.

87. The immune effector cell according to any one of claims 83-86, comprising the chimeric antigen receptor according to any one of claims 12-37.

88. The immune effector cell according to any one of claims 83-87, wherein the chimeric antigen receptor comprises the amino acid sequence set forth in any one of SEQ ID NO: 29 to SEQ ID NO: 36.

89. The immune effector cell according to claims 82-88, wherein the immune effector cell comprises a human cell.

90. The immune effector cell according to any one of claims 82-89, wherein the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

91. The immune effector cell according to any one of claims 82-90, wherein the immune effector cell comprises an autologous or non-autologous immune effector cell.

92. The immune effector cell according to any one of claims 82-91, wherein the immune effector cell comprises a modified immune effector cell.

93. The immune effector cell according to claim 92, wherein the modified immune effector cell comprises a cell that mitigates immune rejection caused by allogeneic cell therapy.

94. The immune effector cell according to any one of claims 92-93, wherein the functions of T cell antigen receptor (TCR) and major histocompatibility complex (MHCI, MHCII) in the modified immune effector cell are suppressed in a T cell.

95. The immune effector cell according to any one of claims 92-94, wherein the modification comprises down-regulating the expression and/or activity of one or more of immune rejection-associated genes.

96. The immune effector cell according to claim 95, wherein the immune rejection-associated gene is selected from one or more of the following genes: TRAC, TRBC, HLA-A, HLA-B, B2M, and CIITA.

97. The immune effector cell according to any one of claims 92-96, wherein the expression and/or activity of the TRAC gene and the HLA-A gene in the modified immune effector cell is down-regulated as compared to a corresponding unmodified cell.

98. The immune effector cell according to any one of claims 92-97, wherein the expression and/or activity of the TRAC gene, the HLA-A gene, and the HLA-B gene in the modified immune effector cell is down-regulated as compared to the corresponding unmodified cell.

99. The immune effector cell according to any one of claims 92-98, wherein the expression and/or activity of the CIITA gene in the modified immune effector cell is not down-regulated as compared to the corresponding unmodified cell.

100.

The immune effector cell according to any one of claims 92-99, wherein the expression and/or activity of the B2M gene in the modified immune effector cell is not down-regulated as compared to the corresponding unmodified cell.

**101.**
The immune effector cell according to any one of claims 92-100, wherein the expression and/or activity of the TRAC gene and the HLA-A gene in the modified immune effector cell is down-regulated as compared to a corresponding wild-type cell.

**102.**
The immune effector cell according to any one of claims 92-101, wherein the expression and/or activity of the B2M gene in the modified immune effector cell is not down-regulated as compared to the corresponding wild-type cell.

**103.**
The immune effector cell according to any one of claims 92-102, wherein the expression and/or activity of the CIITA gene in the modified immune effector cell is not down-regulated as compared to the corresponding wild-type cell.

**104.**
The immune effector cell according to any one of claims 92-103, wherein down-regulating the expression level and/or activity of the gene comprises down-regulating the expression and/or activity of a nucleic acid molecule encoding the gene; and/or down-regulating the expression and/or activity of a protein product encoded by the gene.

**105.**
The immune effector cell according to any one of claims 92-104, wherein the modification comprises: gene knockout, gene mutation, and/or gene silencing.

**106.**
The immune effector cell according to any one of claims 92-105, wherein the modification comprises knocking out an exon of the TRAC gene and knocking out an exon of the HLA-A gene in the immune cell.

**107.**
The immune effector cell according to any one of claims 92-106, wherein the modification comprises knocking out an exon of the TRAC gene, knocking out an exon of the HLA-A gene, and knocking out an exon of the HLA-B gene in the immune cell.

**108.**
The immune effector cell according to any one of claims 92-107, wherein the modification comprises administering to the immune effector cell one or more substances selected from the group consisting of: antisense RNA, siRNA, shRNA, and a CRISPR/Cas9 system.

**109.**
The immune effector cell according to any one of claims 92-108, wherein the modification comprises administering to the immune effector cell the CRISPR/Cas9 system.

**110.**
The immune effector cell according to claims 92-109, wherein the modification further comprises administering to the immune effector cell sgRNA targeting an exonic region of the TRAC gene.

**111.** The immune effector cell according to claim 110, wherein the sgRNA targeting the exonic region of the TRAC gene comprises the nucleotide sequence set forth in any one of SEQ ID NO: 177 to SEQ ID NO: 191.

**112.**
The immune effector cell according to any one of claims 92-111, wherein the modification comprises administering to the immune effector cell sgRNA targeting an exonic region of the HLA-A gene.

**113.**
The immune effector cell according to claim 112, wherein the sgRNA targeting the exonic region of the HLA-A gene comprises the nucleotide sequence set forth in any one of SEQ ID NO: 192 to SEQ ID NO: 232.

**114.**

The immune effector cell according to any one of claims 92-113, wherein the modification comprises administering to the immune effector cell sgRNA targeting an exonic region of the HLA-B gene.

**115.**

The immune effector cell according to claim 112, wherein the sgRNA targeting the exonic region of the HLA-B gene comprises the nucleotide sequence set forth in any one of SEQ ID NO: 237 to SEQ ID NO: 239.

**116.**

The immune effector cell according to any one of claims 92-115, wherein the modification further comprises administering to the cell a Cas enzyme.

**117.**

The immune effector cell according to claim 116, wherein the Cas enzyme comprises a Cas9 protein.

**118.**

The immune effector cell according to claim 108, wherein the antisense RNA comprises the nucleotide sequence set forth in any one of SEQ ID NO: 233 to SEQ ID NO: 236.

**119.**

The immune effector cell according to any one of claims 82-118, wherein the immune effector cell is an HLA-B homozygous cell or heterozygous cell.

**120.**

The immune effector cell according to claim 119, wherein the HLA-B homozygote comprises HLA-B*40 homozygote, HLA-B* 15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

**121.**

The immune effector cell according to any one of claims 82-120, wherein the immune effector cell is an HLA-A homozygous or heterozygous cell.

**122.**

The immune effector cell according to claims 82-121, wherein the HLA-A homozygote or heterozygote comprises HLA-A*02 homozygote, HLA-A*11 homozygote, HLA-A*02/A*11 heterozygote, or HLA-A*24 homozygote.

**123.**

A method for preparing an immune effector cell, comprising introducing the nucleic acid molecule according to any one of claims 55-77 or the vector according to any one of claims 78-80 into an immune effector cell.

**124.**

The method according to claim 123, further comprising: modifying an immune effector cell before/after introducing the nucleic acid molecule according to any one of claims 55-77 or the vector according to any one of claims 78-80 into the immune effector cell, wherein the modification comprises down-regulating the expression and/or activity of one or more of immune rejection-associated genes.

**125.**

The method according to claim 124, wherein the immune rejection-associated gene is selected from one or more of the following genes: TRAC, TRBC, HLA-A, HLA-B, B2M, and CIITA.

**126.**

The method according to any one of claims 124-125, wherein the expression and/or activity of the TRAC gene and the HLA-A gene in the immune effector cell is down-regulated as compared to the expression and/or activity of the corresponding gene in a corresponding unmodified cell.

**127.**

The method according to any one of claims 124-126, wherein the expression and/or activity of the TRAC gene, the HLA-A gene, and the HLA-B gene in the immune effector cell is down-regulated as compared to the expression and/or activity of

### EP 4 620 978 A1

the corresponding gene in the corresponding unmodified cell.

**128.**

The method according to any one of claims 124-127, wherein the expression and/or activity of the CIITA gene is not down-regulated as compared to the expression and/or activity of the corresponding gene in the corresponding unmodified cell.

**129.**

The method according to any one of claims 124-128, wherein the expression and/or activity of the B2M gene is not down-regulated as compared to the expression and/or activity of the corresponding gene in the corresponding unmodified cell.

**130.**

The method according to any one of claims 124-129, wherein the expression and/or activity of the TRAC gene and the HLA-A gene in the immune effector cell is down-regulated as compared to a corresponding wild-type cell.

**131.**

The method according to any one of claims 124-130, wherein the expression and/or activity of the TRAC gene, the HLA-A gene, and the HLA-B gene in the immune effector cell is down-regulated as compared to the corresponding wild-type cell.

**132.**

The method according to any one of claims 124-131, wherein the expression and/or activity of the CIITA gene is not down-regulated as compared to the corresponding wild-type cell.

**133.**

The method according to any one of claims 124-132, wherein the expression and/or activity of the B2M gene is not down-regulated as compared to the corresponding wild-type cell.

**134.**

The method according to any one of claims 124-133, wherein down-regulating the expression level and/or activity of the gene comprises down-regulating the expression and/or activity of a nucleic acid molecule encoding the gene; and/or down-regulating the expression and/or activity of a protein product encoded by the gene.

**135.**

The method according to any one of claims 124-134, wherein the modification comprises: gene knockout, gene mutation, and/or gene silencing.

**136.**

The method according to any one of claims 124-135, wherein the modification comprises knocking out an exon of the TRAC gene and knocking out an exon of the HLA-A gene in the immune cell.

**137.**

The method according to any one of claims 124-136, wherein the modification comprises knocking out an exon of the TRAC gene, knocking out an exon of the HLA-A gene, and knocking out an exon of the HLA-B gene in the immune cell.

**138.**

The method according to any one of claims 124-137, wherein the modification comprises administering to the immune effector cell one or more substances selected from the group consisting of: antisense RNA, siRNA, shRNA, and a CRISPR/Cas9 system.

**139.**

The method according to any one of claims 124-138, wherein the modification comprises administering to the immune effector cell the CRISPR/Cas9 system.

**140.**

The method according to claims 127-139, wherein the modification comprises administering to the immune effector cell sgRNA targeting an exonic region of the TRAC gene.

**141.**

The method according to claim 140, wherein the sgRNA targeting the exonic region of the TRAC gene comprises the nucleotide sequence set forth in any one of SEQ ID NO: 177 to SEQ ID NO: 191.

**142.**

The method according to any one of claims 124-141, wherein the modification comprises administering to the immune effector cell sgRNA targeting an exonic region of the HLA-A gene.

**143.**

The method according to claim 142, wherein the sgRNA targeting the exonic region of the HLA-A gene comprises the nucleotide sequence set forth in any one of SEQ ID NO: 192 to SEQ ID NO: 232.

**144.**

The method according to any one of claims 124-143, wherein the modification comprises administering to the immune effector cell sgRNA targeting an exonic region of the HLA-B gene.

**145.**

The method according to claim 144, wherein the sgRNA targeting the exonic region of the HLA-B gene comprises the nucleotide sequence set forth in any one of SEQ ID NO: 237 to SEQ ID NO: 239.

**146.**

The method according to any one of claims 124-145, wherein the modification further comprises administering to the cell a Cas enzyme.

**147.**

The method according to claim 146, wherein the Cas enzyme comprises a Cas9 protein.

**148.**

The method according to claim 138, wherein the antisense RNA comprises the nucleotide sequence set forth in any one of SEQ ID NO: 233 to SEQ ID NO: 236.

**149.**

The method according to any one of claims 123-148, wherein the immune effector cell comprises a human cell.

**150.**

The method according to any one of claims 123-149, wherein the immune effector cell comprises a T cell, a B cell, a natural killer cell (NK cell), a macrophage, an NKT cell, a monocyte, a dendritic cell, a granulocyte, a lymphocyte, a leukocyte, and/or a peripheral blood mononuclear cell.

**151.**

The method according to any one of claims 123-150, wherein the immune effector cell comprises an autologous or non-autologous immune effector cell.

**152.**

The method according to any one of claims 123-151, wherein the cell is an HLA-B homozygous or heterozygous cell.

**153.**

The method according to claim 152, wherein the HLA-B homozygote comprises HLA-B*40 homozygote, HLA-B*15 homozygote, HLA-B*46 homozygote, HLA-B*13 homozygote, HLA-B*51 homozygote, HLA-B*58 homozygote, HLA-B*07 homozygote, HLA-B*35 homozygote, HLA-B*44 homozygote, HLA-B*52 homozygote, HLA-B*57 homozygote, HLA-B*54 homozygote, and HLA-B*55 homozygote.

**154.**

The method according to any one of claims 123-153, wherein the cell is an HLA-A homozygous or heterozygous cell.

**155.**

The method according to claim 154, wherein the HLA-A homozygote or heterozygote comprises HLA-A*02 homozygote, HLA-A* 11 homozygote, HLA-A* 02/A* 11 heterozygote, or HLA-A 24 homozygote.

**156.**

Use of the isolated nucleic acid molecule according to any one of claims 55-77, the vector according to any one of claims 78-80, the cell according to claim 81, or the immune effector cell according to any one of claims 82-122 in the preparation of a CAR-T cell.

157.

A pharmaceutical composition, comprising the B7H3-binding polypeptide according to any one of claims 1-10, the isolated nucleic acid molecule according to any one of claims 55-77, the vector according to any one of claims 78-80, the cell according to claim 81, and/or the immune effector cell according to any one of claims 82-122, and optionally a pharmaceutically acceptable carrier.

158.

Use of the B7H3-binding polypeptide according to any one of claims 1-10, the isolated nucleic acid molecule according to any one of claims 55-77, the vector according to any one of claims 78-80, the cell according to claim 81, the immune effector cell according to any one of claims 82-122, and/or the pharmaceutical composition according to claim 157 in the treatment of a disease or disorder associated with the expression of B7H3.

159.

The use according to claim 158, wherein the disease or disorder associated with the expression of B7H3 comprises a disease or disorder associated with up-regulation of the expression of B7H3.

160.

The use according to claims 158-159, wherein the disease or disorder associated with the expression of B7H3 comprises cancer.

161.

The use according to claim 160, wherein the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

162.

A method for preventing and/or treating a disease or disorder associated with the expression of B7H3, comprising administering to a subject in need thereof an effective amount of the B7H3-binding polypeptide according to any one of claims 1-10, the isolated nucleic acid molecule according to any one of claims 55-77, the vector according to any one of claims 78-80, the cell according to claim 81, the immune effector cell according to any one of claims 82-122, and/or the pharmaceutical composition according to claim 157.

163.

The method according to claim 162, wherein the disease or disorder associated with the expression of B7H3 comprises a disease or disorder associated with up-regulation of the expression of B7H3.

164.

The method according to any one of claims 162-163, wherein the disease or disorder associated with the expression of B7H3 comprises cancer.

165.

The method according to claim 164, wherein the cancer comprises adrenocortical carcinoma, bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, or endometrial cancer.

FIG. 1

A: bi-epitopic B7H3 UCAR-T cells with double knockout of TRAC and HLA-A
B: bi-epitopic B7H3 UCAR-T cells with double knockout of TRAC and HLA-B
C: bi-epitopic B7H3 UCAR-T cells with triple knockout of TRAC and HLA-A/B
D: single-epitope B7H3 UCAR-T cells (1G7) with double knockout of TRAC and HLA-A
E: single-epitope B7H3 UCAR-T cells (1A5) with double knockout of TRAC and HLA-A
G: bi-epitopic B7H3 CAR-T cells
H: wild-type T cells

FIG. 2

FIG. 3

A: bi-epitopic B7H3 UCAR-T cells with double knockout of TRAC and HLA-A
B: bi-epitopic B7H3 UCAR-T cells with double knockout of TRAC and HLA-B
C: bi-epitopic B7H3 UCAR-T cells with triple knockout of TRAC and HLA-A/B
D: single-epitope B7H3 UCAR-T cells (1G7) with double knockout of TRAC and HLA-A
E: single-epitope B7H3 UCAR-T cells (1A5) with double knockout of TRAC and HLA-A
G: bi-epitopic B7H3 CAR-T cells
H: wild-type T cells

A: bi-epitopic B7H3 UCAR-T cells with double knockout of TRAC and HLA-A
B: bi-epitopic B7H3 UCAR-T cells with double knockout of TRAC and HLA-B
C: bi-epitopic B7H3 UCAR-T cells with triple knockout of TRAC and HLA-A/B
D: single-epitope B7H3 UCAR-T cells (1G7) with double knockout of TRAC and HLA-A
E: single-epitope B7H3 UCAR-T cells (1A5) with double knockout of TRAC and HLA-A
G: bi-epitopic B7H3 CAR-T cells
H: wild-type T cells

FIG. 4

A: bi-epitopic B7H3 UCAR-T cells with double knockout of TRAC and HLA-A
B: single-epitope B7H3 UCAR-T cells (1G7) with double knockout of TRAC and HLA-A
C: single-epitope B7H3 UCAR-T cells (1A5) with double knockout of TRAC and HLA-A
D: bi-epitopic B7H3 CAR-T cells
E: B7H3 UCAR-T cells with double knockout of TRAC and HLA-A (sequence Hbrca84d-2)
F: B7H3 CAR-T cells (sequence Hbrca84d-2)
G: wild-type T cells

FIG. 5

TRAC-/HLA-A-group, bi-epitopic B7H3 UCAR-T cells with double knockout of HLA-A

TRAC-, HLA-A-/B-group, bi-epitopic B7H3 UCAR-T cells with triple knockout of TRAC, HLA-A, and HLA-B

TRAC-, B2M-group, bi-epitopic B7H3 UCAR-T wild-type T cells with double knockout of TRAC and B2M

CART group, bi-epitopic B7H3 UCAR-T wild-type T cells

FIG. 6

## IL-2

- TRAC-/HLA-A-group
- PBS
- TRAC-/HLA-B-group
- TRAC-, HLA-A-/B-group

**Days after treatment**

FIG. 7

## TNF-α

- PBS
- TRAC-/HLA-A-group
- TRAC-/HLA-B-group
- TRAC-, HLA-A-/B-group

**Days after treatment**

FIG. 8

FIG. 9

FIG. 10

FIG. 11

FIG. 12

U3-10_1: TRAC

**Off-target display after removal of control background**

## U3-10_1

| | 1 | | | | | | | | | | 10 | | | | | | | | | 20 | | | 23 | |
|---|---|---|---|---|---|---|---|---|---|---|---|---|---|---|---|---|---|---|---|---|---|---|---|---|
| | A | G | A | G | T | C | T | C | T | C | A | G | C | T | G | G | T | A | C | A | N | G | G | Reads |
| | ● | ● | ● | ● | ● | ● | ● | ● | ● | ● | ● | ● | ● | ● | ● | ● | ● | ● | ● | ● | ● | ● | ● | 15617 |
| | ● | ● | ● | ● | ● | ● | A | A | ● | ● | ● | A | ● | ● | T | ● | C | C | ● | ● | ● | ● | A | 80 |
| | ● | A | G | ● | ● | ● | A | ● | A | ● | ● | ● | ● | ● | ● | ● | ● | ● | ● | ● | ● | ● | ● | 45 |
| | T | ● | ● | A | ● | A | ● | ● | ● | ● | ● | ● | ● | ● | T | C | ● | C | T | ● | ● | ● | ● | 16 |
| | ● | ● | ● | ● | ● | ● | A | ● | A | ● | ● | ● | ● | ● | ● | ● | ● | ● | ● | T | ● | ● | ● | 12 |
| | ● | A | ● | ● | ● | ● | ● | ● | C | A | ● | ● | ● | ● | ● | ● | ● | ● | ● | ● | ● | ● | ● | 11 |

U3-10_2: HLA-A02

**Off-target display after removal of control background**

## U3-10_2

| | 1 | | | | | | | | | | 10 | | | | | | | | | 20 | | | 23 | |
|---|---|---|---|---|---|---|---|---|---|---|---|---|---|---|---|---|---|---|---|---|---|---|---|---|
| | C | T | G | A | C | C | A | T | G | A | A | G | C | C | A | C | C | C | T | G | N | G | G | Reads |
| | ● | ● | ● | ● | ● | ● | ● | ● | ● | ● | ● | ● | ● | ● | ● | ● | ● | ● | ● | ● | ● | ● | ● | 22534 |
| | ● | ● | ● | ● | ● | ● | ● | ● | ● | G | ● | ● | ● | ● | ● | ● | ● | ● | ● | ● | ● | ● | ● | 72 |
| | A | G | ● | G | A | ● | ● | ● | ● | ● | ● | A | ● | ● | ● | ● | ● | ● | ● | ● | ● | ● | ● | 18 |
| | ● | ● | C | ● | T | G | G | ● | A | ● | ● | ● | ● | A | ● | ● | ● | ● | ● | ● | ● | T | ● | 6 |
| | T | ● | ● | ● | ● | T | ● | ● | ● | ● | G | ● | ● | ● | ● | ● | ● | ● | ● | ● | ● | ● | ● | 4 |

FIG. 13

**Chromosome translocation**

T1: TRAC upper strand:HLA-A02 upper strand linked together
T2: TRAC upper strand:HLA-A02 lower strand linked together
$10^{-4}$: not detected

FIG. 14

FIG. 15

## Residual Cas9

**D3: day 3 before electroporation**
**D5-: day 5 before buffer exchanged and after electroporation**
**N.D.: not detected**

FIG. 16

## INTERNATIONAL SEARCH REPORT

| International application No. |
| --- |
| **PCT/CN2023/129566** |

**A. CLASSIFICATION OF SUBJECT MATTER**

C07K16/30(2006.01)i; C12N5/10(2006.01)i

According to International Patent Classification (IPC) or to both national classification and IPC

**B. FIELDS SEARCHED**

Minimum documentation searched (classification system followed by classification symbols)

IPC: C07K, C12N

Documentation searched other than minimum documentation to the extent that such documents are included in the fields searched

Electronic data base consulted during the international search (name of data base and, where practicable, search terms used)

CNTXT, CNABS, VEN, ENTXT, 万方, WANFANG, CNKI, PubMed, GenBank, ISI web of knowledge: B7H3, CD276, 抗体, VHH, 纳米抗体, CAR, 嵌合抗原受体, 茂行制药(苏州)有限公司, 尚小云, 蒋海娟, 马少文, SEQ ID NOs: 1-244

**C. DOCUMENTS CONSIDERED TO BE RELEVANT**

| Category* | Citation of document, with indication, where appropriate, of the relevant passages | Relevant to claim No. |
| --- | --- | --- |
| PX | WO 2023274384 A1 (NINGBO T-MAXIMUM BIOPHARMACEUTICALS CO., LTD.) 05 January 2023 (2023-01-05)<br>claims 1-126 | 1-165 |
| X | CN 114729041 A (THE UNITED STATES OF AMERICA, AS REPRESENTED BY THE SECRETARY, DEPARTMENT OF HEALTH AND HUMAN SERVICES) 08 July 2022 (2022-07-08)<br>claims 1 and 19-20, and description, paragraph [113] | 12-13, 15-36, 38-82 |
| X | CN 113501884 A (XUZHOU MEDICAL UNIVERSITY) 15 October 2021 (2021-10-15)<br>abstract | 83 |
| Y | CN 113501884 A (XUZHOU MEDICAL UNIVERSITY) 15 October 2021 (2021-10-15)<br>abstract | 84-87, 89-165 |
| Y | CN 114729041 A (THE UNITED STATES OF AMERICA, AS REPRESENTED BY THE SECRETARY, DEPARTMENT OF HEALTH AND HUMAN SERVICES) 08 July 2022 (2022-07-08)<br>description, paragraph [113] | 84-87, 89-165 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 December 2023** | **14 December 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/129566** |

### C.    DOCUMENTS CONSIDERED TO BE RELEVANT

| Category* | Citation of document, with indication, where appropriate, of the relevant passages | Relevant to claim No. |
| --- | --- | --- |
| Y | US 2011028695 A1 (REVETS, H.A.P. et al.) 03 February 2011 (2011-02-03)<br>    claims 1-22 | 12-13, 15-36, 38-82, 84-87, 89-165 |
| Y | CN 113061580 A (JIANGSU T- MAXIMUM BIOTECH CO., LTD.) 02 July 2021 (2021-07-02)<br>    claims 1-10, and description, paragraph [244] | 94-165 |
| A | CN 113039206 A (SEATTLE CHILDREN'S HOSPITAL (DBA SEATTLE CHILDREN'S RESEARCH INSTITUTE)) 25 June 2021 (2021-06-25)<br>    entire document | 1-165 |
| A | CN 113402619 A (XUZHOU MEDICAL UNIVERSITY) 17 September 2021 (2021-09-17)<br>    entire document | 1-165 |
| A | WO 2022126689 A1 (GUANGZHOU BIO-GENE TECHNOLOGY CO., LTD.) 23 June 2022 (2022-06-23)<br>    entire document | 1-165 |

Form PCT/ISA/210 (second sheet) (July 2022)

## INTERNATIONAL SEARCH REPORT

| International application No. |
| --- |
| **PCT/CN2023/129566** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**

International application No.

**PCT/CN2023/129566**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **158-165**
because they relate to subject matter not required to be searched by this Authority, namely:

Claims 158-165 relate to a method for treating a living human body or animal body, which falls within the cases set out in PCT Rule 39.1(iv) for which an international search is not required. In the present report, a search is conducted on the basis of "the uses in preparation of a pharmaceutical composition".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
| --- |
| **PCT/CN2023/129566** |

| Patent document cited in search report | | | Publication date (day/month/year) | Patent family member(s) | | | Publication date (day/month/year) |
| --- | --- | --- | --- | --- | --- | --- | --- |
| WO | 2023274384 | A1 | 05 January 2023 | None | | | |
| CN | 114729041 | A | 08 July 2022 | CA | 3156761 | A1 | 29 April 2021 |
| | | | | JP | 2022552875 | A | 20 December 2022 |
| | | | | US | 2022380471 | A1 | 01 December 2022 |
| | | | | AU | 2020370125 | A1 | 19 May 2022 |
| | | | | EP | 4031250 | A1 | 27 July 2022 |
| | | | | WO | 2021081052 | A1 | 29 April 2021 |
| CN | 113501884 | A | 15 October 2021 | None | | | |
| US | 2011028695 | A1 | 03 February 2011 | EP | 2215123 | A1 | 11 August 2010 |
| | | | | CA | 2706200 | A1 | 04 June 2009 |
| | | | | KR | 20100097716 | A | 03 September 2010 |
| | | | | EP | 2215125 | A1 | 11 August 2010 |
| | | | | JP | 2011504740 | A | 17 February 2011 |
| | | | | EP | 2650311 | A2 | 16 October 2013 |
| | | | | EP | 2650311 | A3 | 04 June 2014 |
| | | | | US | 2015232562 | A1 | 20 August 2015 |
| | | | | US | 9969805 | B2 | 15 May 2018 |
| | | | | WO | 2009068631 | A1 | 04 June 2009 |
| | | | | US | 2011059090 | A1 | 10 March 2011 |
| | | | | US | 8975382 | B2 | 10 March 2015 |
| | | | | WO | 2009068630 | A1 | 04 June 2009 |
| | | | | AU | 2008328784 | A1 | 04 June 2009 |
| | | | | AU | 2008328784 | B2 | 27 March 2014 |
| | | | | US | 2011053865 | A1 | 03 March 2011 |
| | | | | ZA | 201004057 | B | 28 April 2011 |
| | | | | WO | 2009068628 | A1 | 04 June 2009 |
| | | | | BRPI | 0819656 | A2 | 23 June 2015 |
| | | | | WO | 2009068627 | A2 | 04 June 2009 |
| | | | | WO | 2009068627 | A3 | 01 October 2009 |
| | | | | US | 2011189203 | A1 | 04 August 2011 |
| | | | | WO | 2009068625 | A2 | 04 June 2009 |
| | | | | WO | 2009068625 | A3 | 06 August 2009 |
| | | | | EP | 2220120 | A2 | 25 August 2010 |
| | | | | CA | 2705890 | A1 | 04 June 2009 |
| | | | | MX | 2010005783 | A | 10 August 2010 |
| | | | | EP | 2225278 | A2 | 08 September 2010 |
| | | | | AU | 2008328785 | A1 | 04 June 2009 |
| | | | | AU | 2008328781 | A1 | 04 June 2009 |
| | | | | CA | 2706675 | A1 | 04 June 2009 |
| | | | | IL | 205734 | A0 | 30 November 2010 |
| | | | | AU | 2008328779 | A1 | 04 June 2009 |
| | | | | AU | 2008328779 | B2 | 05 June 2014 |
| | | | | CA | 2706425 | A1 | 04 June 2009 |
| CN | 113061580 | A | 02 July 2021 | WO | 2021136263 | A1 | 08 July 2021 |
| | | | | CA | 3163304 | A1 | 08 July 2021 |
| | | | | EP | 4086342 | A1 | 09 November 2022 |
| | | | | AU | 2020418199 | A1 | 21 July 2022 |
| | | | | KR | 20220124197 | A | 13 September 2022 |
| | | | | JP | 2023508740 | A | 03 March 2023 |
| | | | | US | 2022193135 | A1 | 23 June 2022 |

Form PCT/ISA/210 (patent family annex) (July 2022)

## INTERNATIONAL SEARCH REPORT
### Information on patent family members

International application No.

**PCT/CN2023/129566**

| Patent document cited in search report | | | Publication date (day/month/year) | Patent family member(s) | | | Publication date (day/month/year) |
|---|---|---|---|---|---|---|---|
| CN | 113039206 | A | 25 June 2021 | AU | 2019333149 | A1 | 25 March 2021 |
| | | | | IL | 281129 | A | 29 April 2021 |
| | | | | WO | 2020047257 | A1 | 05 March 2020 |
| | | | | JP | 2021534784 | A | 16 December 2021 |
| | | | | EP | 3844191 | A1 | 07 July 2021 |
| | | | | EP | 3844191 | A4 | 15 June 2022 |
| | | | | US | 2021324083 | A1 | 21 October 2021 |
| | | | | CA | 3110858 | A1 | 05 March 2020 |
| CN | 113402619 | A | 17 September 2021 | None | | | |
| WO | 2022126689 | A1 | 23 June 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4751180 A
- US 4935233 A
- WO 8809344 A
- WO 9954440 A
- CN 106279416 B

### Non-patent literature cited in the description

- **MILLER et al.** *Jour. of Immunology*, 2003, vol. 170, 4854-4861
- **WARD et al.** Binding Activities of a Repertoire of Single Immunoglobulin Variable Domains Secreted From Escherichia coli. *Nature*, 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc. Natl. Acad. Sci. USA*, 1988, vol. 85, 5879-5883
- **NGUYEN V.K. et al.** *The EMBO Journal*, 2000, vol. 19, 921-930
- **MUYLDERMANS S.** *J Biotechnol.*, 2001, vol. 74, 277-302
- **VANLANDSCHOOT P. et al.** *Antiviral Research*, 2011, vol. 92, 389-407
- **KANG XIAOZHEN et al.** *Chinese Journal of Biotechnology*, 2018, vol. 34 (12), 1974-1984
- **KABAT et al.** Sequences of Proteins of Immunological Interest. National Institutes of Health, 1987
- **CHOTHIA ; LESK**. *J. Mol. Biol.*, 1987, vol. 196, 901-917
- **CHOTHIA et al.** *Nature*, 1989, vol. 342, 877-883
- **PADLAN**. *FASEB J.*, 1995, vol. 9, 133-139
- **MACCALLUM**. *J Mol Biol*, 1996, vol. 262 (5), 732-45
- **JACKSON et al.** *Trends Biochem Sci*, 1990, vol. 15 (12), 477-83
- **JACKSON ; KAMINSKI**. *RNA*, 1995, vol. 1 (10), 985-1000
- **DALL'ACQUA et al.** *Biochem.*, 1998, vol. 37, 9266-9273
- **CHEADLE et al.** *MolImmunol*, 1992, vol. 29, 21-30
- **RAAG ; WHITLOW**. *FASEB*, 1995, vol. 9 (1), 73-80
- **SAMBROOK et al.** Molecular Cloning: A Laboratory Manual. Cold Spring Harbor Laboratory Press, 2001
- **KIM et al.** *PLOS One*, 2011, vol. 6, e18556